(19) Europäisches Patentamt
European Patent Office
Office européen des brevets

(11) Veröffentlichungsnummer: **0 602 524 A1**

(12) # EUROPÄISCHE PATENTANMELDUNG

(21) Anmeldenummer: **93119783.4**

(22) Anmeldetag: **08.12.93**

(51) Int. Cl.5: **C07H 21/00**, C07D 219/16, C07D 405/14, C07D 473/22, G01N 33/533, C12Q 1/68

(30) Priorität: **15.12.92 DE 4242202**

(43) Veröffentlichungstag der Anmeldung: **22.06.94 Patentblatt 94/25**

(84) Benannte Vertragsstaaten: **AT BE CH DE DK ES FR GB IT LI NL SE**

(71) Anmelder: **HOECHST AKTIENGESELLSCHAFT**
**Brüningstrasse 50**
**D-65929 Frankfurt am Main(DE)**

(72) Erfinder: **Skrzipczyk, Heinz Jürgen, Dr.**
**Forsthausweg 11**
**D-63263 Neu-Isenburg (Zeppelinheim)(DE)**
Erfinder: **Uhlmann, Eugen, Dr.**
**Zum Talblick 31**
**D-61479 Glashütten(DE)**
Erfinder: **Mayer, Andreas, Dr.**
**Rosenpark 32**
**D-65795 Hattersheim am Main(DE)**

(54) **Chemilumineszenzmarkierte Gensonden und ihre Verwendung in Gensondentesten.**

(57) Die Erfindung betrifft ein Verfahren zu Herstellung und Reinigung von mit Acridiniumderivaten markierten Gensonden sowie deren Verwendung in Gensondentesten.

Fig. 1

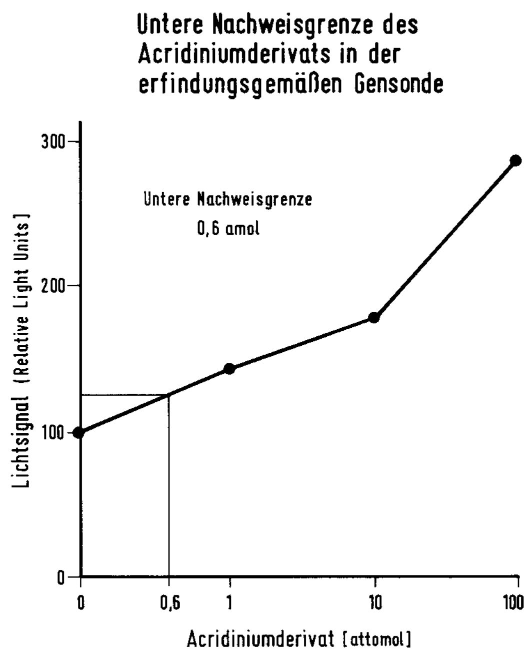


EP 0 602 524 A1

Der Gegenstand der vorliegenden Erfindung betrifft ein Verfahren zu Herstellung und Reinigung von mit Acridiniumderivaten markierten Gensonden sowie deren Verwendung in Gensondentesten.

Lumineszierende Verbindungen finden bereits vielfältige Anwendung. Sie werden als Indikatoren in Bioassays, Enzymimmunoassays und Lumineszenzimmunoassays (vgl. W. P. Collins "Alternative Immunoassays", Verlag John Wiley & Sons Ltd., Chichester, 1985), aber auch in Nukleinsäure - Hybridisierungsassays eingesetzt (vgl. J. A. Matthews et al. "Analytical ßiochemistry", 151, 205 - 289, 1985). Außerdem werden chemilumineszierende Verbindungen bei der "flow injection analysis", in post-column-Detektoren in der Flüssigkeitschromatographie, in der Strömungsforschung und in künstlichen Lichtquellen verwendet. Acridinderivate eignen sich außerdem in Testverfahren zur Lebensmittel- und Umweltanalyse.

Die Verwendung von Acridiniumlabeln in Nukleinsäure-Hybridisierungsassays wird in der EP-A-0 273 115 erwähnt sowie in der EP-A-0 212 951, EP-A-0 281 390, EP-A-0 310 312, EP-A-0 313 219 und der WO 89/02896 beschrieben. In allen Fällen, mit Ausnahme der erstgenannten Patentanmeldung werden Acridiniumesterverbindungen angegeben, die den Nachteil einer relativ geringen Stabilität besitzen. Dies führt unmittelbar zu Problemen bei der Synthese, Reinigung und Lagerstabilität der Gensonden, die mit den genannten Acridiniumverbindungen markiert werden und dadurch auch zu Problemen hinsichtlich der Eignung solcher Gensonden für den Einsatz in Assaysystemen.

In der EP-A-0 407 816 werden Nucleotid-Derivate mit der Base Uracil beschrieben, die ihrerseits über einen Spacer mit einer chemilumineszierenden Verbindung markiert ist.

Der vorliegenden Erfindung liegt daher die Aufgabe zugrunde, lumineszenzmarkierte Gensonden mit gegenüber dem Stand der Technik vorteilhaften Eigenschaften bereitzustellen sowie einen Gensondenassay, der auf den vorteilhaften Eigenschaften neuer Gensonden aufbauend, ebenfalls deutliche Vorteile aufweist.

Die Aufgabe wird überraschenderweise gelöst durch die Bereitstellung einer mit einem oder mehreren Acridiniumderivat(en) markierten Gensonde, sowie eines die Vorteile dieser neuen Gesonde ausnutzenden Gensondenassays.

Die vorliegende Erfindung betrifft daher eine Gensonde gemäß Formel (XI)

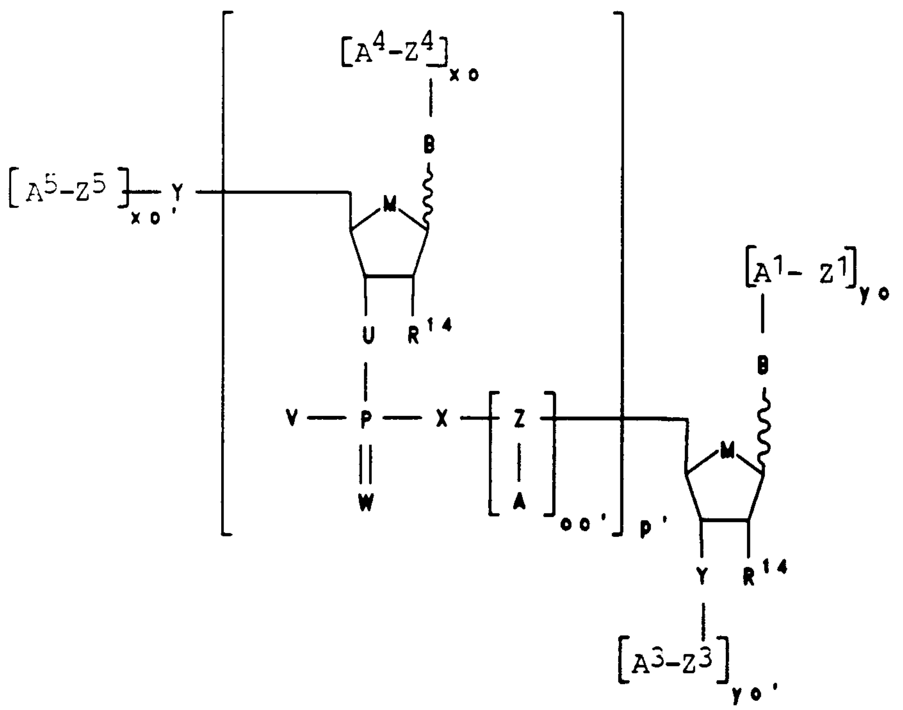

(XI)

worin

| | |
|---|---|
| $R^{14}$ | Wasserstoff, Hydroxy, Amino, $NHR^{15}$, Azid, Halogen oder eine geschützte Hydroxyl- oder Methoxygruppe in $\alpha$-oder $\beta$-Stellung bedeutet, wobei |
| $R^{15}$ | für $C_1$- bis $C_{18}$-Alkyl, vorzugsweise $C_1$-bis $C_8$-Alkyl, $C_6$- bis $C_{20}$-Aryl, ($C_6$- bis $C_{14}$)-Aryl-($C_1$- bis $C_8$)-alkyl, $-(CH_2)_c-[NH(CH_2)_c]_d-NR^{16}R^{16'}$ steht, worin |
| c | eine ganze Zahl von 2 bis 6 und |

| | |
|---|---|
| d | eine ganze Zahl von 0 bis 6 ist und |
| $R^{16}$, $R^{16'}$ | unabhängig voneinander Wasserstoff, $C_1$-bis $C_6$-Alkyl oder ($C_1$- bis $C_4$)-Alkoxy-($C_1$- bis $C_6$)-alkyl, bevorzugt Methoxyethyl bedeutet und Halogen für Fluor, Chlor, Brom oder Iod steht, oder einen Rest -$[Z^6-A^6]_{xo''}$ bedeutet, worin $Z^6$, $A^6$ und xo" die weiter unten angegebenen Bedeutungen haben, |
| $A^1$, $A^2$, $A^3$, $A^4$, $A^5$, $A^6$ | Acridiniumderivate der Formel I sind, welche über $R^4$ in Formel (I) an $Z^1$, $Z^2$, $Z^3$, $Z^4$, $Z^5$, $Z^6$ der Formel (XI) gebunden sind, |
| B | für eine in der Nukleotidchemie übliche Base außer Uracil, beispielsweise für natürliche Basen wie Adenin, Cytosin, Guanin und Thymin oder unnatürliche Basen wie Purin, 2,6-Diaminopurin, 7-Deazaadenin, 7-Deazaguanin, $N^4N^4$-Ethanocystin, $N^6N^6$-Ethano-2,6-diaminopurin, 5-Methylcytosin, Pseudoisocytosin, steht, |
| M | Oxy, Methylen, Fluormethylen oder Difluormethylen ist, |
| U | Oxy, Sulfandiyl, Imino oder Methylen bedeutet, |
| V | für Hydroxy, Mercapto, SeH, $C_1$- bis $C_{18}$-Alkoxy, vorzugsweise $C_1$- bis $C_6$-Alkoxy, $C_1$- bis $C_{18}$-Alkyl, vorzugsweise $C_1$-bis $C_6$-Alkyl, $C_6$- bis $C_{20}$-Aryl, ($C_6$- bis $C_{14}$)-Aryl-($C_1$- bis $C_8$)-alkyl, $NHR^{15}$, $NR^{16}R^{17}$ oder einen Rest der Formel $(OCH_2CH_2)_{m'}O(CH_2]_{n'}CH_2R^{18}$ bedeutet, worin |
| $R^{15}$ und $R^{16}$ | die oben angegebenen Bedeutungen haben, |
| $R^{17}$ | $C_1$- bis $C_{18}$-Alkyl, vorzugsweise $C_1$- bis $C_8$-Alkyl und besonders $C_1$- bis $C_4$-Alkyl, $C_6$- bis $C_{20}$-Aryl oder ($C_6$-bis $C_{10}$)-Aryl-($C_1$- bis $C_8$)-alkyl bedeutet oder im Falle von $NR^{16}R^{17}$ zusammen mit $R^{16}$ und dem sie tragenden Stickstoffatom einen 5-bis 6-gliedrigen heterozyklischen Ring bedeutet, der zusätzlich ein weiteres Heteroatom aus der Reihe O. S. N enthalten kann, |
| $R^{18}$ | Wasserstoff oder eine funktionelle Gruppe wie Hydroxy, Amino, $NHR^{16}$, COOH, $CONH_2$, $COOR^{19}$ oder Halogen bedeutet, worin $R^{19}$ $C_1$ - bis $C_4$-Alkyl, vorzugsweise Methyl, bedeutet und $R^{16}$ und Halogen die oben angegebenen Bedeutungen besitzen, |
| m' | eine ganze Zahl von 1 bis 100, bevorzugt 3 bis 20 und besonders bevorzugt 3 bis 8 ist, |
| n' | eine ganze Zahl von 0 bis 18, beorzugt 0 bis 15, ist, |
| W | Oxo, Thioxo oder Selenoxo ist, |
| X | Oxy, Thio, NH oder Methylen bedeutet, |
| Y | für Oxy, Thio, NH, Methylen oder eine Schutzgruppe steht, |
| $Z^1$, $Z^2$, $Z^3$, $Z^4$, $Z^5$ | unabhängig voneinander $C_1$- bis $C_{30}$-Alkyl, $C_6$- bis $C_{30}$-Aryl, ($C_6$- bis $C_{12}$)-Aryl-($C_1$- bis $C_{20}$)-alkyl, $C_1$- bis $C_{30}$-Alkoxy bedeutet, welche Substituenten gegebenenfalls einfach oder mehrfach mit $C_1$- bis $C_8$-Alkyl, $C_6$- bis $C_{20}$-Aryl, ($C_6$-bis $C_{14}$)-Aryl-($C_1$- bis $C_8$)-alkyl, $C_1$- bis $C_8$-Alkoxy, Imino, Hydroxy, $NHR^{15}$, Thio, Phosphat, Pyrophosphat oder Triphosphat substituiert sind, wobei $R^{15}$ die oben angegebene Bedeutung hat, |
| oo' | eine ganze Zahl von 0 bis 10 ist, |
| xo, yo, xo', yo', xo" | unabhängig voneinander 0 oder 1 bedeuten, mit der Maßgabe, daß mindestens eine der Variablen oo', xo, yo, xo', yo' oder xo" ungleich Null ist, |
| p' | für eine ganze Zahl von 1 bis 100 steht |

und

die geschweifte Klammer in 2'- und 3'-Stellung bedeutet, daß sich die an die 2'-bzw. 3'-Position gebundenen Reste U bzw. $R^1$ und Y bzw $R^1$ auch umgekehrt in 3'- bzw. 2'-Position befinden können,

wobei jedes Nukleotid in seiner D- bzw. L-Konfiguration vorliegen kann.

Die im Oligonukleotidstrang von Nukleotid zu Nukleotid wiederholt auftretenden Reste A, B, M, $R^{14}$, $R^{15}$, $R^{16}$, $R^{17}$, $R^{18}$, $R^{19}$, U, V, W, X, Y, Z und die Variablen oo', xo und xo" können in verschiedenen Nukleotiden voneinander unterschiedliche Bedeutungen haben.

Unter "Phosphat" wird im Rahmen vorliegender Erfindung eine Gruppe der allgemeinen Formel

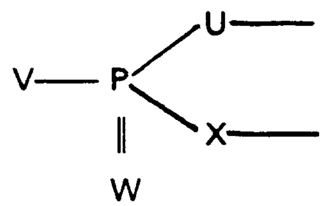

verstanden, worin U, V, W und X die oben angegebenen Bedeutungen haben. Bevorzugt ist eine Gensonde gemäß Formel (XI), worin

B für eine in der Nucleotidchemie übliche Base steht, einschließlich Uracil, und
xo, yo jeweils Null bedeutet.

Die vorliegende Erfindung betrifft bevorzugt eine Gensonde gemäß Formel (XI), worin

B für eine der oben angegebenen Basen in $\beta$-Stellung
und
M für 0
und
$R^{14}$ für H, OH
und
Y für Oxy oder NH steht
und
M, U, V, W für 0 stehen
und
$Z^1$, $Z^2$, $Z^3$, $Z^4$, $Z^5$ unabhängig voneinander $C_1$- bis $C_{30}$-Alkyl, $C_6$-bis $C_{30}$-Aryl, ($C_6$- bis $C_{12}$)-Aryl-($C_1$- bis $C_{20}$)-alkyl oder $C_1$- bis $C_{30}$-Alkoxy bedeuten, gegebenenfalls mit Imino und/oder Hydroxy substituiert
und
p' eine ganze Zahl von 5 bis 50
und
oo' eine ganze Zahl von 0 bis 3 bedeutet
und
zwei der Variablen xo', yo', xo'', oo' ungleich Null sind
und
alle übrigen Substituenten die oben angegebenen Bedeutungen haben.

Die vorliegende Erfindung betrifft besonders bevorzugt eine Gensonde gemäß Formel (XI), worin die Substituenten die für die bevorzugte Ausgestaltung der Formel (XI) zuletzt genannten Bedeutungen haben.
und

p' für eine anze Zahl von 10 bis 30 steht
und
oo' Null oder 1 bedeutet
und
nur jeweils eine Variable xo', yo', xo'', oo' 1 sein kann und die jeweils übrigen Variablen Null bedeuten.

Die vorliegende Erfindung betrifft weiterhin besonders bevorzugt eine Gensonde gemäß Formel (XI) mit den für die Substituenten und die Variable p' zuletzt genannten Bedeutungen,
worin

oo' für 1 steht
und
xo, yo, xo', yo', xo'' Null bedeuten.

Die vorliegende Erfindung betrifft weiterhin ganz besonders bevorzugt eine Gensonde gemäß Formel (XI) mit den für die Substituenten und die Variable p' zuletzt genannten Bedeutungen,
worin

xo' für 1 steht
und
xo, yo, yo', xo'', oo' Null bedeuten.

Die vorliegende Erfindung betrifft weiterhin ganz besonders bevorzugt eine Gensonde gemäß Formel (XI) mit den für die Substituenten und die Variable p' zuletzt genannten Bedeutungen,
worin

| | |
|---|---|
| yo' | für 1 steht |
| | und |
| xo, yo, xo', xo'', oo' | Null bedeuten. |

Bei den genannten Acridiniumderivaten (A1-A6), deren Struktur und Herstellung bereits in der EP-A-0 257 541 und der EP-A-0 330 050 beschrieben wurde, handelt es sich um Verbindungen der allgemeinen Formel (I)

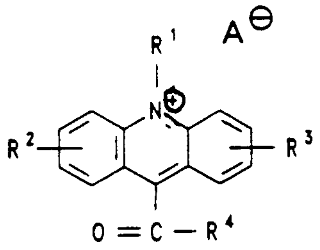

(I)

in der $R^1$ Wasserstoff, ein Alkyl-, Alkenyl- oder Alkinylrest mit 1 bis 10 Kohlenstoffatomen, eine Benzyl- oder Arylgruppe ist,
$R^2$ und $R^3$ Wasserstoff, eine Alkylgruppe mit 1 bis 10 Kohlenstoffatomen, eine substituierte oder unsubstituierte Aminogruppe, eine Carboxy-, Alkoxy-, Cyano-, Nitrogruppe oder Halogen sind,
$R^4$ einen Rest der Formel (II) oder (III) darstellt

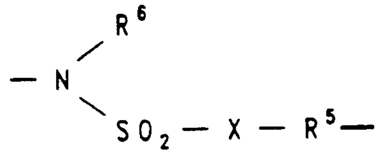

(II)

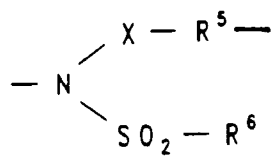

(III)

worin $R^5$ eine reaktive Gruppe ist, die zu einer Bindung mit einem Oligonukleotid, enthaltend 1 - 100 Nukleotide wie insbesondere Oligo- und Polynukleotidsequenzen (Targets) in DNA und RNA, übergeführt ist,
$R^6$ Wasserstoff, ein Alkyl-, Alkenyl- oder Alkoxyrest mit 1 bis 10 C-Atomen, eine substituierte Aminogruppe, eine Benzylgruppe, eine Arylgruppe, eine Heteroalkylgruppe oder ein Heterocyclus ist, der/die auch mit Hydroxy-, Amino-, Alkylamino-, Alkyl-, Alkenyl- oder Alkoxy mit 1 bis 10 C-Atomen, Polyalkoxy-oder Aryloxy-gruppen oder einer heterocyclischen Gruppe substituiert sein können, wobei die zuletzt genannten Substituenten ihrerseits mit einer heterocyclischen Verbindung oder einem Amin substituiert sein können oder zusammen einen O- und/oder S- und/oder NH- oder N-Alkyl-aufweisenden Heterocyclus bilden können, und X eine Arylgruppe, die direkt oder über eine Alkyl- oder Oxyalkylgruppe an das Stickstoff- oder Schwefelatom gebunden ist und über eine Alkyl- oder Oxyalkylgruppe an den Rest $R^5$ gebunden ist und die auch mit Alkyl-, Alkenyl-, Hydroxy-, Amino-, Alkoxy-, Polyalkoxy- oder Aryloxy-Gruppen und/oder Heteroatomen einfach oder mehrfach substituiert sein kann oder den Rest einer aliphatischen, araliphatischen oder aromatischen, nicht notwendigerweise natürlichen, Aminocarbonsäure, bedeutet oder eine Phenylgruppe ist, wenn $R^6$ eine Phenylgruppe ist, die einfach oder mehrfach mit $C_1$-$C_6$-Alkyl substituiert ist.

Das die Chemilumineszenz nicht beeinträchtigende Anion kann z. B. ein Tetrafluorborat-, Perchlorat-, Halogenid-, Alkylsulfat-, Halosulfonat-, Alkylsulfonat- oder Arylsulfonat-Anion sein. Auch jedes andere Anion kann eingesetzt werden, sofern es die Chemilumineszenz nicht löscht oder abschwächt.
Die Heteroalkylgruppen oder heterocyclischen Gruppen enthalten vorzugsweise Heteroatome, die zu einer Steigerung der Wasserlöslichkeit der erfindungsgemäßen Verbindungen beitragen können, wie z. B. Stickstoff, Sauerstoff, Schwefel, Phosphor, bzw. deren Kombinationen. Besonders geeignete Heterocyclen

sind z. B. Morpholin, Piperidin, Tetrahydrofuran, Dioxane.

Besondere Bedeutung haben Acridiniumderivate gemäß den Formeln (I) bis (III) die dadurch gekennzeichnet sind, daß X eine Gruppe der Formel (IV) ist,

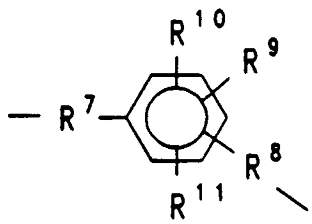 (IV)

in der $R^7$ ein Substituent der Formel $-(CH_2)_n-$ oder $((CH_2)_m-O)_n-$ ist mit n = 0 bis 4 und m = 1 bis 6, $R^8$ ein Substituent in ortho-, meta- oder para-Stellung zu $R^7$ der Formel $-(CH_2)_p-$, eine Polyalkylenoxid-Gruppe der Formel $-(O-(CH_2)_m-)_p$ oder $-((CH_2)_m-O-)_p$ bevorzugt mit p = 1 bis 6, oder ein verzweigter oder unverzweigter Kohlenwasserstoffrest mit 1 bis 4 C-Atomen ist und die Substituenten $R^9$ bis $R^{11}$ Wasserstoff oder geradkettige oder verzweigte Kohlenwasserstoffreste mit bis zu 30 C-Atomen sind, wobei eine oder mehrere $-CH_2$-Einheiten auch durch O, S, SO,$SO_2$, NH oder N-Alkyl ersetzt sein können und zwei dieser Substituenten ringförmig verknüpft sein können.

Durch die geeignete Auswahl der Gruppe $R^5$ erhält das Acridiniumderivat eine so hohe Reaktivität, daß es schon unter milden Bedingungen selektiv mit einer funktionellen Gruppe der biologischen Substanz von Interesse eine Bindung eingehen kann. Geeignete reaktive Gruppen sind aus der folgenden Zusammenstellung zu ersehen:

a) 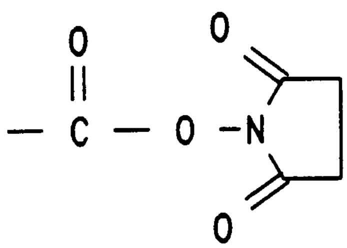

b) 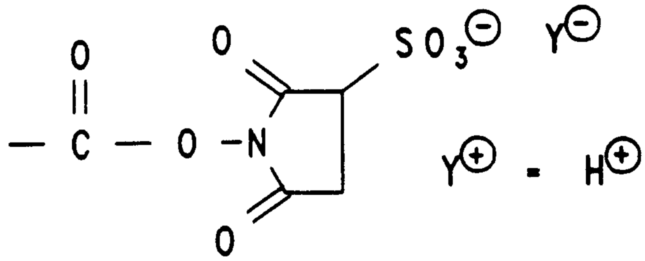

$Y^{\oplus}$ = $H^{\oplus}$, Alkali$^{\oplus}$

c) 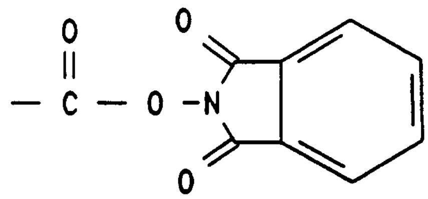

d) $-SO_2-CH=CH_2$

e) 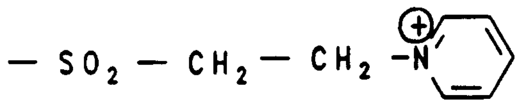 Halogenid$^{\ominus}$

f) $-N=C=S$

g) 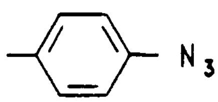

h) 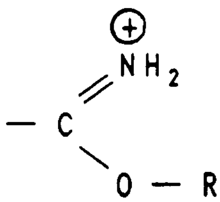 Halogenid$^{\ominus}$

i) 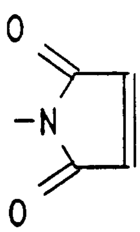

k) 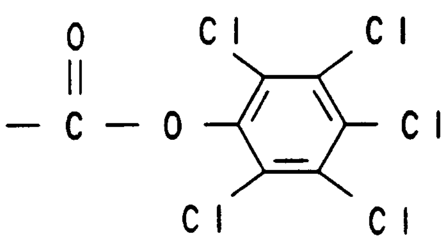

l) 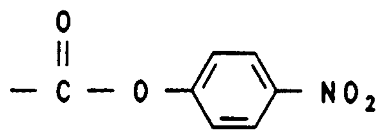

m) 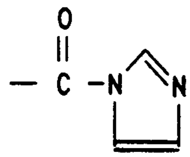

n) 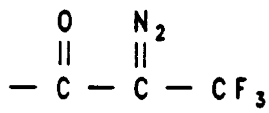

o) 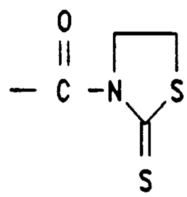

In vielen Fällen haben sich erfindungsgemäße Acridiniumderivate als geeignet erwiesen, bei denen $R^5$ eine Gruppe der Formel (V)

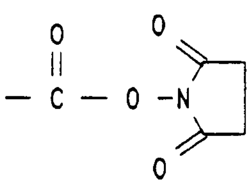

(V)

ist.

Weiterhin haben sich Acridiniumverbindungen der Formel (VI) als besonders geeignet erwiesen. In Formel (VI)

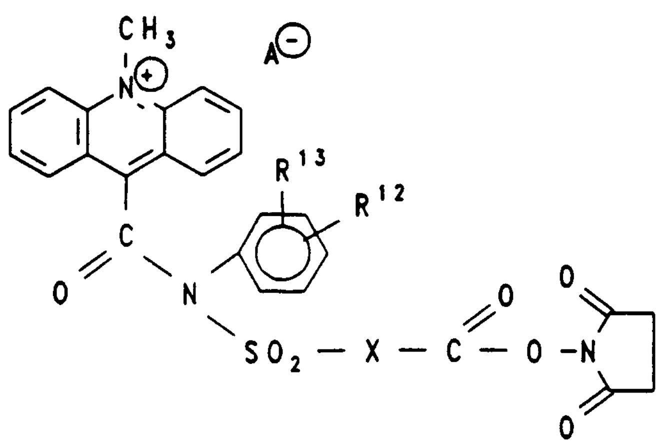

(VI)

ist X eine Gruppe der Formel (VII)

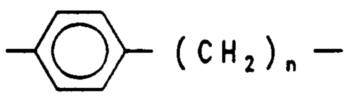

(VII)

mit n = 0 bis 4, vorzugsweise 2 bis 4
und $R^{12}$ und $R^{13}$ sind unabhängig voneinander Wasserstoff, eine Alkylgruppe, eine Alkoxygruppe mit 1 bis 4 C-Atomen, eine $(-O-CH_2-CH_2)_n-OR$-Gruppe, wobei n die Bedeutung 1 bis 8 hat und R eine Morpholinoethyl- oder eine Alkylgruppe mit 1 bis 4 C-Atomen oder eine N,N-Dimethylaminoethyl-Gruppe ist, oder zusammen eine Ethylendioxy-Gruppe sind und $A^{\ominus}$ die in Anspruch 1 genannten Bedeutungen hat.
Unter den zuletzt genannten Verbindungen der Formel (VII) sind wiederum diejenigen ganz besonders bevorzugt, in denen X eine p-Ethylenphenyl-Gruppe und

1) $R^{12}$ = H und $R^{13}$ eine p-Methoxy-Gruppe
oder
2) $R^{12}$ = H und $R^{13}$ ein p-Alkoxy-Rest der Formel (VIII)

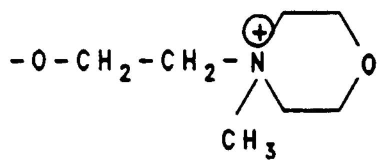

(VIII)

oder
3) $R^{12}$ eine o-Methoxy- und $R^{13}$ eine p-Methoxy-Gruppe ist
oder
4) $R^{12}$ und $R^{13}$ zusammen eine 3,4-Ethylendioxy-Gruppe sind,
wie z.B. die Verbindungen der Formel (IX)

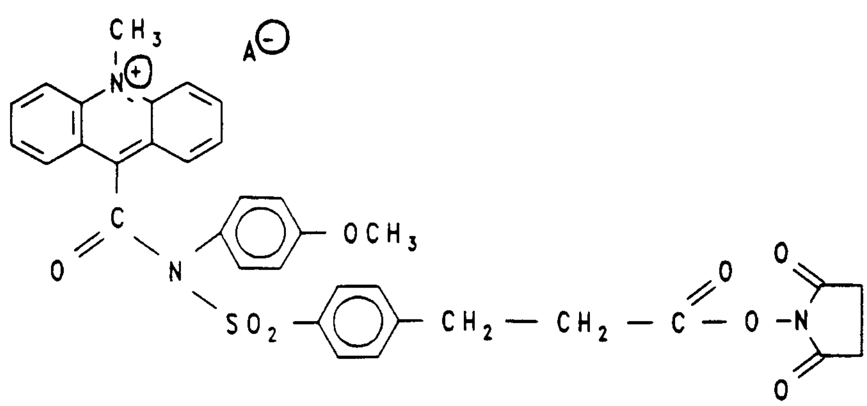

(IX)

Weitere besonders geeignete Acridiniumderivate haben die Formel (X)

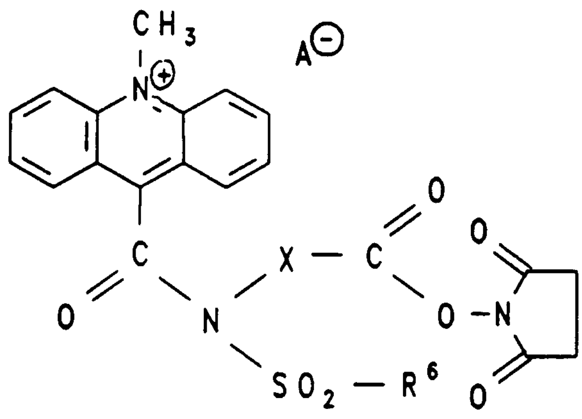

(X)

in der $R^6$ eine Alkylgruppe mit 1 bis 4 C-Atomen oder eine Phenylgruppe ist, die mit bis zu drei Alkyl- oder Alkoxygruppen mit jeweils 1 bis 4 C-Atomen, mit einer $-(-O-CH_2-CH_2)_n-OR$-Gruppe, wobei n die Bedeutung 1 bis 8 hat und R eine Morpholinoethyl- oder eine N,N-Dimethylaminoethyl-Gruppe oder Alkylgruppe mit 1 bis 4 C-Atomen ist oder mit einer Ethylendioxy-Gruppe substituiert sein kann und X die in Anspruch 1 oder 2 genannten Bedeutungen hat oder in der $R^6$ eine Phenylgruppe ist, die mit bis zu drei Alkylgruppen mit jeweils 1 bis 4 C-Atomen substituiert sein kann und X eine ortho-, meta- oder para-Phenylengruppe ist.

Die vorangehend beschriebenen, erfindungsgemäß angewandten Acridiniumverbindungen lassen sich dann mit Oligo- bzw. Polynukleotiden direkt oder über einen Spacer umsetzen, wobei ein (mehrere) Nukleotid(e) bzw. der Spacer eine funktionelle Gruppe, z. B. eine Amino- oder Sulphydryl-Gruppe trägt (tragen), die mit einer der oben aufgeführten Reaktivgruppen des Labels reagieren. Derart modifizierte Nukleotide sind auch kommerziell verfügbar.

Die vorliegende Erfindung betrifft weiterhin ganz besonders bevorzugt die in den Beispielen angegebenen Gensonden.

Der Gensondenassay:

Für den erfindungsgemäßen Gensondenassay zur Bestimmung einer Targetsequenz in einer Probe ist die Hybridisierung mit einer, wie oben beschriebenen, Gensonde erforderlich. Es gibt unterschiedliche Gensondenassay-Formate, die sich grundsätzlich in zwei Kategorien einteilen lassen:

1. Homogene Gensondenassays

Die homogenen Gensondenassays zeichnen sich durch einfache Handhabung und leichte Automatisierbarkeit aus, nachteilig ist die häufig geringe Nachweisempfindlichkeit.

Bevorzugte Ausführungsformen sind der "Hybridization Protection Assay" (Clin. Chem. 35/8, 1989, 1588-1594), die Technik der "Kissing Probes" (Nachr. Chem. Tech. Lab. 37/7, 1989, 698) und das "Energy transfer"-Prinzip (Analytical Biochemistry 169, 1988, 18). Die erfindungsgemäßen Gensonden lassen sich in homogenen Gensondentesten einsetzen. Aufgrund ihrer von der Mikroumgebung abhängigen (selektiven) Stabilität eignen sich diese Gensonden besonders gut für den "Hybridization Protection Assay" (Beispiel 16).

2. Heterogene Gensondenassay

Die heterogenen Gensondenassays sind im Vergleich zu den homogenen Verfahren handhabungstechnisch aufwendiger, führen aber in der Regel zu einer besseren Nachweisempfindlichkeit. Die erfindungsgemäßen Gensonden lassen sich in heterogenen Gensondenassays einsetzen. Bevorzugte Ausführungsformen sind nachfolgend aufgeführt: Blot-Techniken (Analytical Biochemistry 169, 1988, 1-2), "Strand Displacement Assay" (Clin. Chem. 32/9, 1986, 1631-1636), Separation über (magnetisierbare) Mikropartikel (EP-A-0 281 390) bzw. andere Festphasen. An diese Festphasen können das nachzuweisende Target bzw. das Hybrid direkt oder indirekt, beispielsweise über "Capture-Probes" (Clin. chem. 35/9, 1989, 1826-1831) oder andere hochaktive und spezifische Systeme wie Biotin/(Strept)-Avidin (Clin. Chem. 37/5, 1991, 632-633) oder Antigen/Antikörper-Wechselwirkungen (Analytical Biochemistry 169, 1988, 6-9) gebunden sein.

Die Abtrennung des Hybrids von überschüssiger Gensonde kann im Falle von Magnetpartikeln als Festphase auch durch Unterschiede im Adsorptionsverhalten bzw. in der ionischen Wechselwirkung (EP-A-0 281 390) erreicht werden.

Besonders gut eignen sich die erfindungsgemäßen Gensonden in einem heterogenen Gensondentest mit Magnetpartikeln als Festphase (Beispiel 17).

Ein wesentlicher Vorteil der erfindungsgemäßen Gensonden stellt ihre Stabilität in Lösung dar. In Tabelle 1 ist ein Stabilitätsvergleich mit einer kommerziellen Gensonde (Acridiniumesterlabel] der Fa. Gen Probe wiedergegeben. Die erfindungsgemäßen Gensonden weisen in Belastungstesten bei 37 °C und 50 °C selbst über einen Zeitraum von 10 Tagen keinen Signalabfall auf. Die kommerzielle Sonde besitzt dagegen zu diesem Zeitpunkt nur noch 70 % bzw. 23 % der ursprünglichen Signalaktivität.

Die Stabilität der erfindungsgemäßen Gensonden ist im Vergleich zu herkömmlichen Gensonden überraschenderweise sogar noch höher, als aus der im Vergleich zu herkömmlichen Acridiniumderivaten höheren Stabilität der in den erfindungsgemäßen Gensonden eingesetzten Acridiniumderivate hätte geschlossen werden können. Diese überraschend hohe Stabilität der erfindungsgemäßen Gensonden wird aus den in Tabelle 2 gezeigten Daten deutlich: während bei einer Belastung von 37 °C oder 50 °C über einen Zeitraum von 13 Tagen die Signalaktivität der freien Acridiniumverbindung ("eigenes Label") auf ca. 80 bis 85 % des Ausgangswertes abfällt (deutlicher Signalabfall bereits nach 1 bis 2 Tagen beobachtbar), bleibt die Signalaktivität derselben Acridiniumverbindung, die innerhalb der erfindungsgemäßen Gensonde gebunden vorliegt ("Gensonde mit eigenem Label") über einen Beobachtungszeitraum von insgesamt 10 Tagen stabil.

Die Daten in Tabelle 3 zeigen, daß die hervorragende Stabilität des Acridiniumderivats innerhalb der erfindungsgemäßen Gensonde sogar bei extrem hohen Temperaturen von bis zu 95 °C über einen Zeitraum von 4 bzw. 8 Stunden erhalten bleibt.

Ein weiterer Vorteil ergibt sich dadurch, daß die erfindungsgemäßen Gensonden aufgrund ihrer hohen Stabilität in gebrauchsfertiger Lösung statt in lyophilisierter Form eingesetzt werden können.

Die durch eine höhere Quantenausbeute der in den erfindungsgemäßen Gensonden eingesetzten Acridiniumderivate bedingte höhere Sensitivität wird aus Fig. 1 und 2 ersichtlich: gemäß Fig. 1 liegt die unter Nachweisgrenze des Acridiniumderivats, welches in der erfindungsgemäßen Gensonde Anwendung findet, bei ca. 0,6 attomol und ist somit um einen Faktor von ca. 10 tiefer (besser) als die bei ca. 5 attomol liegende untere Nachweisgrenze des Acridiniumesters der Firma Gen Probe (Fig. 2).

Zum Nachweis extrem niedriger Target-Konzentrationen ist es oftmals erforderlich, zusätzlich eine Target- und/oder Signalamplifikation durchzuführen.

Eine Targetamplifikation wird in derRegel dann durchgeführt, wenn das nachzuweisende Target (DNA- bzw. RNA-Sequenz) in sehr niedriger Konzentration vorliegt und es sich nicht bzw. nur schlecht in einer Hybridisierungsreaktion mit einer Gensonde nachweisen läßt.

Mit den derzeitigen in den Gensonden verwendeten Labeln können im besten Falle ca. $10^3$ - $10^4$ Targetmoleküle in einer Probe nachgewiesen werden. Für die Bestimmung von Viren z.B. ist jedoch im Extremfall der Nachweis einer einzigen Targetsequenz erforderlich. Zur Erreichung dieser Sensitivität muß der Hybridisierung eine Targetamplifikation vorgeschaltet sein. Bei der "Polymerase Chain Reaction" (PCR), dem wichtigsten Targetamplifikations-System, beginnt die spezifische Synthese mit einer Hitze-Denaturierung der zu vermehrenden doppelsträngigen DNA (ca. 95 °C). Anschließend erfolgt eine Anlagerung zweier Oligonucleotid-Start-DNA-Sequenzen (Primer) bei ca. 55 °C an ihre komplementären Sequenzen und Verlängerung der angelagerten Primer durch eine DNA-Polymerase (bei ca. 72 °C). Die Primer hybridisieren an gegenüberliegende Stränge der DNA-Sequenz und sind so orientiert, daß die Polymerase die Ziel-DNA-Sequenz zwischen den beiden Primern abschreibt und mit den im Reaktionsgemisch zur Verfügung stehenden Nukleotiden synthetisiert.

Da die Polymerase-Produkte selbst wieder komplementär zu den Primern sind und diese ebenfalls binden, verdoppelt der jeweilige Zyklus einer DNA-Amplifikation die in dem vorangegangenen Zyklus synthetisierte Menge an Ziel-DNA. Das Ergebnis ist eine exponentielle Akkumulation von Kopien der spezifischen Ziel-DNA um den Faktor $2^n$, wobei n für die Anzahl der durchgeführten Amplifikationszyklen steht. Nach 30 Zyklen wird eine Verstärkung um ca. Faktor $10^6$ erreicht. Durch Verwendung einer hitzestabilen Polymerase läßt sich der gesamte Prozeß automatisieren.

Eine weitere an Bedeutung gewinnende Targetamplifikations-Methode stellt die Vervielfältigung mit "Q$\beta$-Replicase", einer RNA-Polymerase, dar. Im Gegensatz zur PCR wird zuerst mit einer DNA-Sonde hybridisiert und dann das Produkt der Hybridisierungsreaktion verstärkt.

Bei der "Ligase Chain Reaction" (LCR) werden zur Amplifikation des Targets (Zielsequenz von z.B. 50 Basen) pro DNA-Strang zwei synthetisch hergestellte Oligonucleotide (je 25 Basen) eingesetzt, wobei diese beiden "Probes" direkt nebeneinander auf der Zielsequenz hybridisieren. Für das Target auf dem komplementären Strang werden ebenfalls "Probes" synthetisiert. Die nebeneinanderliegenden "Probes" sind an ihren nicht benachbarten Enden mit unterschiedlichen Labeln (z.B. Biotin, Fluorescein) markiert. Die vier Oligonukleotide werden im Überschuß zum Ansatz mit der Patientenprobe pipettiert.

Die Denaturierung der doppelstrangigen DNA der Probe erfolgt bei hoher Temperatur (ca. 75 °C). Nach Absenkung der Temperatur auf ca. 50 °C hybridisieren die "Probes" mit der komplementären Sequenz des Targets. Bei dieser Temperatur verbindet eine hitzestabile DNA-Ligase aus Thermus thermophilus die beiden benachbarten "Probes". Erneute Temperaturerhöhung auf 85 °C trennt die Targetsequenzen von den legierten Probe-Paaren, die selbst als neue und zusätzliche Targets dienen und somit eine exponentielle Vermehrung der ursprünglich vorhandenen Zielsequenzen ermöglichen.

Beim "Transcription amplification system" (TAS) wird eine Sequenz, die von einer DNA-abhängigen RNA-Polymerase erkannt wird, in die cDNA-Kopie der zu amplifizierenden Targetsequenz eingebaut. Die Amplifikation der Targetsequenz erfolgt durch Transkription der cDNA-Vorlage in viele RNA-Kopien.

"Nucleic acid sequence based amplification" (NASBA) stellt eine kontinuierliche, homogene und isotherme Verstärkungsmethode für RNA oder DNA dar. Sie ist einfach (kein spezielles Equipment erforderlich) und schnell.

Die erfindungsgemäßen Gensonden lassen sich mit targetamplifizierten Produkten hybridisieren. Bevorzugte Targetamplifikationssysteme stellen PCR (Labor Praxis, Dezember 1988, 1336-1341; Clin. Chem. 37/11, 1991, 1893-1894), Q$\beta$-Replicase (Nature 339, 401-402; Clin. chem. 35/9, 1989, 1826-1831; Clin. chem. 37/9, 1991, 1682-1685), LCR (Proc. Natl. Acad. Sci. USA 88, 1991, 189-193), TAS (Proc. Natl. Acad. Sci. USA 86, 1989, 1173-1177) und NASBA (Nature 350, 1991, 91-92) dar.

Es ist darüber hinaus aufgrund der hohen Stabilität der eingesetzten Acridiniumderivate möglich, eine "signalintegrierte" Targetamplifikation vorzunehmen (Clin. Chem. 37/9, 1991 1626-1632).

Signalamplifikationssysteme werden vorzugsweise dann eingesetzt, wenn eine direkte Markierung der Gensonde entweder zu keiner befriedigenden Sensitivität führt oder die Hybridisierungsfähigkeit mit dem nachzuweisenden Target beeinträchtigt. Beim Biotin/(Strept)avidin-System wird die Gensonde unter schonenden Bedingungen biotinyliert, die Hybridisierungseigenschaft der Gensonde bleibt dadurch voll erhalten. Anschließend wird markiertes (Strept)avidin zugeführt. Im Prinzip können für die Markierung die unterschiedlichsten Label bzw. weitere Verstärkungssysteme (z.B. Enzymsysteme) eingesetzt werden. Selbst bei hohem Labelüberschuß bleibt die extrem hohe Affinität zwischen Biotin und (Strept)avidin bestehen.

Ähnlich kann bei den Antigen/markierter Antikörper-Systemen vorgegangen werden. Als Antigen kann grundsätzlich jede Substanz, für die ein entsprechender Antikörper verfügbar ist, verwendet werden. Vorzugsweise handelt es sich hierbei um ein kleineres Molekül (Hapten), welches sich in Analogie zum Biotin leicht für die Herstellung der entsprechenden Gensonde einsetzen läßt. Der Antikörper kann ebenfalls in Analogie zum (Strept)avidin auf unterschiedlichste Weise markiert werden. Allerdings ist die Affinität zwischen Antigen/Antikörper in der Regel um einen Faktor $10^{-3}$ - $10^{-7}$ geringer als beim Biotin/(Strept)-avidin-System. Darüber hinaus kommt es bei einem höheren Labelbeladungsgrad leicht zu einer Verschlechterung der immunologischen Reaktivität. Ein Beispiel für ein Antigen/Antikörpersystem stellt Digoxigenin/Anti Digoxigenin Antikörper dar.

Das "Christmas-tree"-Prinzip sieht wie folgt aus: Eine markierte Gensonde 1 hybridisiert mit der komplementären Sequenz des nachzuweisenden Targets. Eine markierte Gensonde 2 kann entweder mit einer unterschiedlichen Targetsequenz bzw. mit einer Sequenz der Gensonde 1 hybridisieren. Dieser Vorgang läßt sich im Prinzip beliebig fortsetzen. Man kann darüber hinaus 2 Ausführungsformen unterscheiden. In der ersten Ausführungsform hybridisieren die verschiedenen Komponenten in sequentieller Reihenfolge miteinander, in der zweiten Ausführungsform hybridisiert das Target mit einem präformierten Sondenkomplex, der in analoger Weise, wie zuvor beschrieben, hergestellt wurde.

Beim "Catalyzed reporter deposition"-System (z.B. ELAST™ von Du Pont) katalysiert ein an eine Festphase immobilisiertes Enzym eine Reaktion, bei der zusätzliche signalgebende Enzymmoleküle an die Festphase gebunden werden. Beispielsweise katalysiert das Enzym Peroxidase die Umsetzung von Biotintyramid, wobei Biotin-Moleküle an die Festphase gebunden werden. Anschließend wird enzymmarkiertes Streptavidin zugeführt. Die Art der Enzymmarkierung ist frei wählbar, so daß die unterschiedlichsten chromogenen, luminogenen Substanzen verwendet werden können.

Das Liposom-System läßt sich folgendermaßen durchführen: Die Gensonde ist mit einem Acridiniumlabel markiert. Anschließend wird ein labelspezifischer Antikörper zugeführt, der ein mit identischen oder unterschiedlichen Labelmolekülen gefülltes Liposom trägt. Das Liposom läßt sich durch ein Detergenz, z.B. Triton X-100 öffnen. Anschließend wird das Signal der freigesetzten Labelmoleküle ermittelt.

Unter Einsatz der erfindungsgemäßen Gensonden bevorzugte Signalamplifikationssysteme stellen Biotin-markierte (Strept)avidin-Komplexe (Clin. Chem. 37/5, 1991, 632-633), Antigen-markierte Antikörperkomplexe (Analytical Biochemistry 169, 1988, 6-9), das "Christmas-Tree"-Prinzip (1987 Elsevier Science Publishers B.V, 253-264), die "Catalyzed reporter deposition" (Journal of Immunological Methods 125, 1989, 279-285) und labelgefüllte Liposomen (Clin. Chem. 37, 1991, 1519-1520; Journal of Bioluminescence and Chemiluminescence 4, 1989, 88-89) dar.

Die hohe Stabilität der erfindungsgemäßen Gensonden ermöglicht darüber hinaus neuartige Signalamplifikationssysteme. Diese sind dadurch gekennzeichnet, daß sich nach der Hydridisierung der Doppelstrang durch Erhitzen ohne Schädigung des Labels trennen läßt. Auf Basis des wieder zugänglichen Labels wird eine Signalamplifikation aufgebaut. Die Grundzüge eines derartigen Signalamplifikationssystems sehen wie folgt aus: Nach der Hybridisierung und anschließender differentieller Hydrolyse gemäß dem "Hybridization Protection Assay" erfolgt eine Magnetpartikeltrennung. Das Backgroundsignal kann auf diese Weise extrem niedrig gehalten werden. Zwecks Amplifikation des spezifischen Signals wird danach das Hybrid durch Erhitzen gespalten. Ein labelspezifischer Antikörper wird zugefügt. Dieser Antikörper kann auf verschiedenste Art markiert sein, z.B. erfolgt bei einer Biotin-Markierung der Nachweis über gelabeltes (Strept)avidin, bei einer Antigen-Markierung über einen spezifischen gelabelten Antikörper. Das im (Strept)avidin oder Antikörper eingesetzte Label kann eines der in den erfindungsgemäßen Gensonden eingesetzten Acridiniumlabel, aber auch irgend ein anderes Label darstellen. Im Falle eines Enzyms kann zusätzlich ein Enzymamplifikationssystem (Annales de Biologie Clinique 47, 1989, 527-532; "ELAST™" von du Pont) angeschlossen werden.

Der labelspezifische Antikörper kann aber auch ein Liposom tragen, welches mit identischen oder unterschiedlichen Labelmolekülen gefüllt ist. Das Liposom läßt sich durch Zugabe eines Detergenz, z.B. Triton X-100 öffnen. Anschließend wird das Signal der freigesetzten Labelmoleküle ermittelt.

Besonders gut lassen sich die erfindunsgemäßen Gensonden in das Biotin-(Strept)avidin System, das Antigen/Antikörper-System und das Liposom-System einsetzen.

Die Acridiniumverbindungen weisen auch bei bereits eingeführten Amplifikationssystemen deutliche Vorteile gegenüber anderen Labeln auf. Beispielsweise können für das "Christmas-Tree"-Prinzip präformierte Sondenkomplexe verwendet werden. Die Herstellung eines derartigen Komplexes durch sequentielle Hybridisierung verschiedener Gensonden bei erhöhter Temperatur bereitet wegen der herausragenden Stabilität des Labels keine Probleme.

Herstellung der Gensonden:

Die Oligonucleotid-Synthese nach der Festphasen-Methode erfolgt nach an sich bekannten Methoden ausgehend von einem Nucleosid, das über seine 3'-Hydroxy- oder Amino-Gruppe an den polymeren Träger gebunden ist. Der polymere Träger ist beispielsweise CPG (Controlled pore glass) oder ein organisches Harz wie TentaGel (Firma Rapp, Tübingen). Das Nucleosid ist über einen geeigneten Spacer (beispielsweise Succinat, Oxalat oder Bis-Hydroxyethylsulfid-Linker) als Ester oder Amid einer Dicarbonsäure oder eines Phosphats an den funktionalisierten Träger gebunden. Nach erfolgter Synthese muß das Oligonucleotid vom Träger spaltbar sein und die freigesetzte Aminofunktion dient zur Derivatisierung des Oligonucleotids mit dem Chemilumineszenz-Label. Als bevorzugtes Reagenz zur Einführung der chemilumineszierenden Acridinium-Derivate dienen die Hydroxysuccinimidester, die mit der freien Aminofunktion am Oligonucleotid zur Reaktion gebracht werden.

Die Einführung der freien reaktiven Aminofunktion bedarf einer Abwandlung der Oligonukleotid-Synthese. Kommerziell zugängliche Reagenzien erlauben die Einführung der Aminofunktion am 3'-Ende (z.B. 3'-Amino-Modifier CPG, Fa. MWG-Biotech GmbH, Ebersberg, Deutschland), am 5'-Ende (z.B. 5'-Amino-Modifier $C_2$ bis $C_{12}$, Fa. MWG Biotech GmbH) oder internucleotidisch, d.h. an jeder beliebigen Stelle im Oligonukleotid (5'Branched Modifier $C_3$, Fa. MWG, Biotech GmbH) nach Angaben des Herstellers. Eine weitere Möglichkeit besteht über ein modifiziertes Thymin-Derivat (Amino Modifier-dT, Fa. MWG) oder über ähnliche modifizierte Nukleobasen wie bei Goodchild (Bioconjugate Chem. 1, 165 (1990)) beschrieben. Bei Goodchild sind außerdem weitere Reagenzien beschrieben (S. 170-173), die die Einführung von Amino- oder Thiol-Funktionen in Oligonucleotide erlauben.
Die Herstellung von Oligonucleotiden mit amino- oder thiol-modifizierten Nukleotiden, die sich ebenfalls zur Markierung mit Chemilumineszenz-Farbstoffen eignen, ist in der EP-A-0 490 281 beschrieben. Im Beispiel 14a ist die Synthese eines 26-mer Oligonucleotids erläutert, das am 5'-Terminus mit einem Aminoalkylphosphatrest versehen ist. Die Struktur des modifizierten 5'-Endes ist in Formel (64) dargestellt:

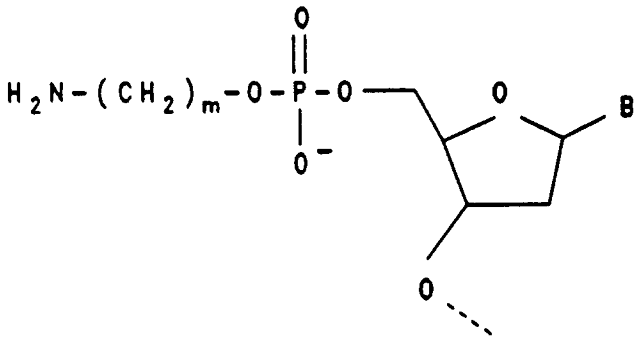

(64)

B = Adenin, Guanin, Thymin, Cytosin, 2,6-Diaminopurin, Inosin, 5-Fluorurcil; m = 6.

Die Herstellung der Einheit (64) erfolgt mit Hilfe des 5'-Amino-Modifiers der Formel (70) nach der Phosphoramidit-Methode.

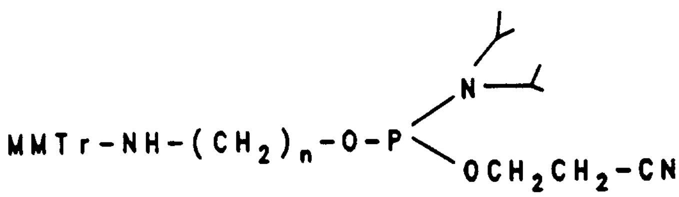

(70)

MMTR = Monomethoxytrityl, n = 2, 3, 6, 12

Beispiel 14b steht für die Herstellung eines 3'-aminomodifizierten Oligonucleotids, das ausgehend vom 3'-Amino-Modifier CPG (Formel 71) und Standard-Phosphoramidit-Chemie erhältlich ist. Die Struktur des modifizierten 3'-Endes ist in Formel (65) dargestellt

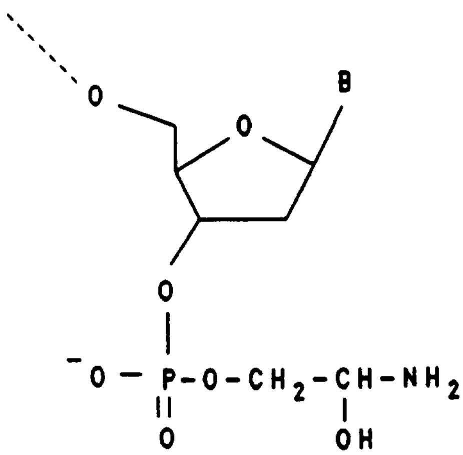

(65)

B = Adenin, Guanin, Thymin, Cytosin, 2,6-Diaminopurin, Inosin, 5-Fluoruracil

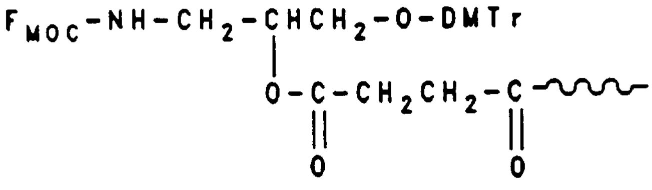

(71)

$F_{MOC}$ = Fluorenylmethyloxycarbonyl

Die Synthese eines Oligonucleotids, das die reaktive Amino-Funktion innerhalb der Oligonucleotid-Sequenz anstelle eines Nukleotids enthält ( = internucleotidisch), ist durch Kondensation mit dem Branched-Modifier der Formel (72) möglich (Beispiel 14c). Die Struktur der internucleotidischen Modifikation ist in Formel (66) dargestellt:

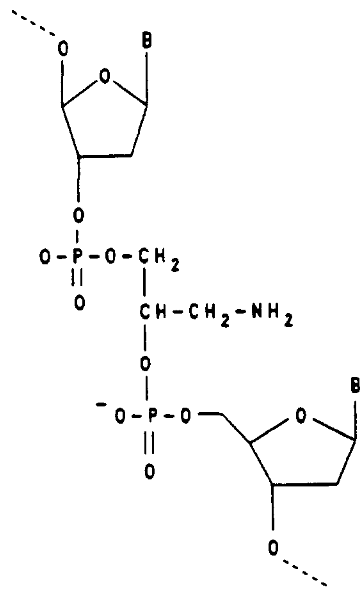

(66)

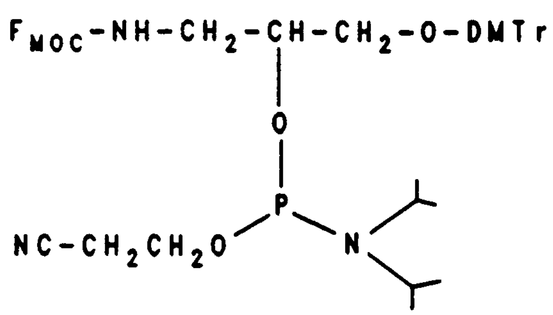

(72)

Die Herstellung von Oligonucleotiden mit einem Aminoalkylphosphoramidat-Rest in der Internucleotidbindung (Formel 67) erfolgt durch Kondensation mit einem Methoxy-Phosphoramidit der Formel (73) das mit 0.1 M Jod und Trifluoracetylhexamethylendiamin (x = NH und n = 6 der Formel 67) zum Phosphoramidat umgesetzt wird (Beispiel 14d)

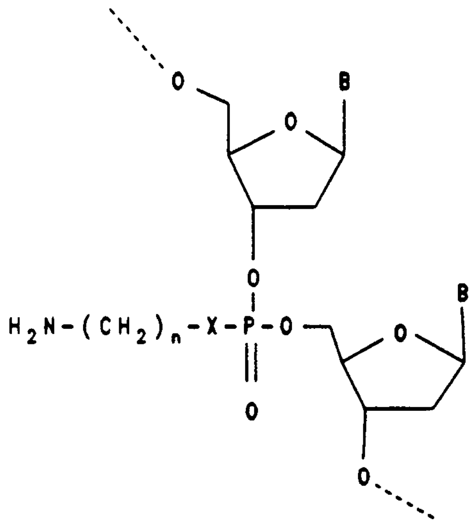

(67)

x = 0, NH, S; n = 2 bis 18

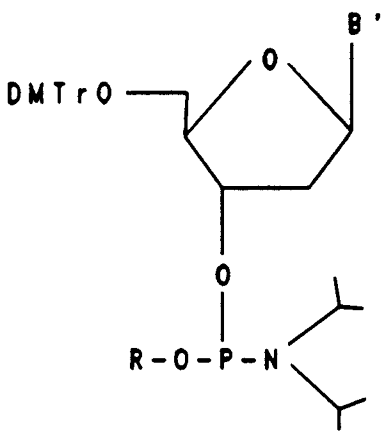

(73)

B' = Thymin, $N^4$-Benzoyl-cyostin, $N^6$-Benzoyladenin, $N^2$-Isobutyrylguanin,
DMTr = Di-methoxytrityl, R = $CH_2CH_2CN$ oder $CH_3$

Entsprechend sind Oligonucleotide der Formel (68) zugänglich, wenn man von einem Träger der Formel (74) ausgeht, der am Ende der Synthese mit $NaJO_4$ oder $H_2O_2/Na_2WO_4$ zum mit Ammoniak spaltbaren Träger oxidiert wird (Beispiel 14e).

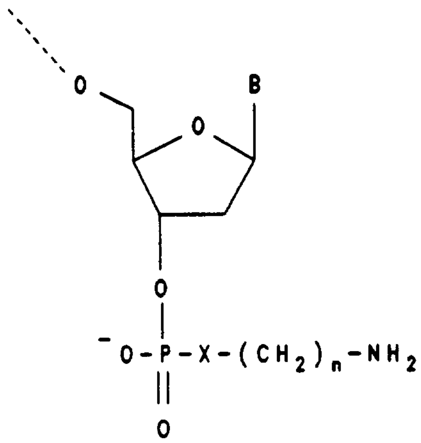

(68)

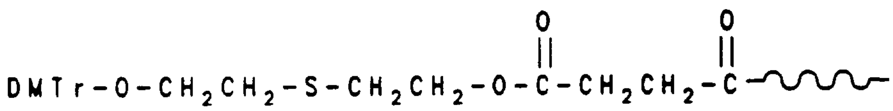

(74)

In Beispiel 12f wird die Herstellung eines Oligonucleotids der Formel (69) ausgeführt, das am 3'-Ende ein 3'-Aminonucleotid enthält. Die Synthese erfolgt ausgehend von einem CPG-Träger derFormel (75), dessen Phosphoramidat-Bindung am Ende der Synthese mit 80 %iger Essigsäure gespalten wird:

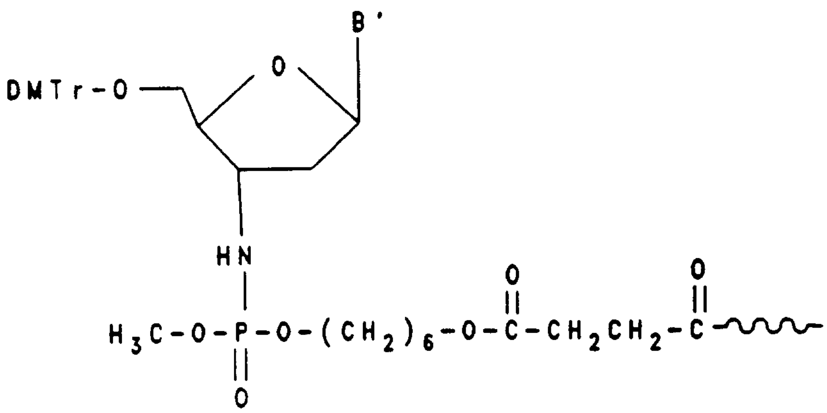

(75)

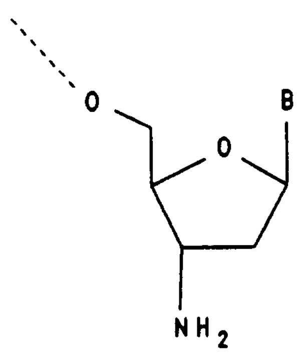

(69)

Die Beispiele 14a bis 14f stellen eine Auswahl dar, wie Oligonucleotide mit einer freien Aminofunktion hergestellt werden können, die anschließend wie in Beispiel 15 beschrieben mit einem aktivierten Derivat des Chemilumineszenzlabels umgesetzt werden. Neben Aminofunktionen können auch andere funktionelle Gruppen wie -SH, -OH, -COOH,

$$-O-\overset{\overset{O}{\|}}{\underset{\underset{O}{\|}}{P}}-X^{\ominus}$$

(X = 0, S)

mit geeigneten reaktiven Gruppen des Labels umgesetzt werden. Als bevorzugtes Regenz zur Einführung von chemiluminescenzierenden Acridinium-Derivaten dienen deren Hydroxysuccinimidester, die wie in Beispiel 15 beschrieben mit der freien Aminofunktion am Oligonucleotid zur Reaktion gebracht werden.

Vergleichsversuche zur Stabilität verschiedener Acridiniumderivate während der Kopplung an ein Oligonucleotid:

Folgende Acridiniumderivate wurden eingesetzt:

1) "Ac-Hoechst", ein zur Herstellung einer erfindungsgemäßen Gensonde verwendbares Acridiniumderivat

2) "Ac-Abbott", ein Acridiniumderivat, das gemäß EP-A-273 115 (Firma Abbott), für eine Anwendung in DNA-Sondentesten geeignet sein sollte.

Setzt man die Succinimidester Ac-Hoechst und Ac-Abbott wie in Beispiel 15 beschrieben unter identischen Bedingungen mit dem Oligonucleotid der Formel (65) um, so ergib nur Ac-Hoechst ein stabiles Produkt, während Ac-Abbott vor Abschluß der Reaktion bereits Zerfallsprodukte aufzeigt. Die Reaktionen wurden mittels HPLC (bei pH 6.8) verfolgt. Gemessen wurde die Absorption in Absorptionseinheiten AUF bei 260 nm gegen die Zeit in Minuten. Bei der Reaktion von (65) (peak A, Fig.3, vgl. Beispiel 14, = Beispiel 76, worin R = H)) mit Ac-Hoechst entsteht kontinuierlich das markierte Oligonucleotid der Formel (76) (peak B, Fig. 3). (Fig. 3a: Absorptionsmessung nach 2 Tagen, Fig. 3b: Messung nach 3 Tagen, Fig. 3c: Messung nach 4 Tagen).

5'd GGC CGT TAC CCC ACC TAC TAG CTA AT $p-O-CH_2-\underset{\underset{OH}{|}}{CH}-CH_2-NH-R$ (76)

R = 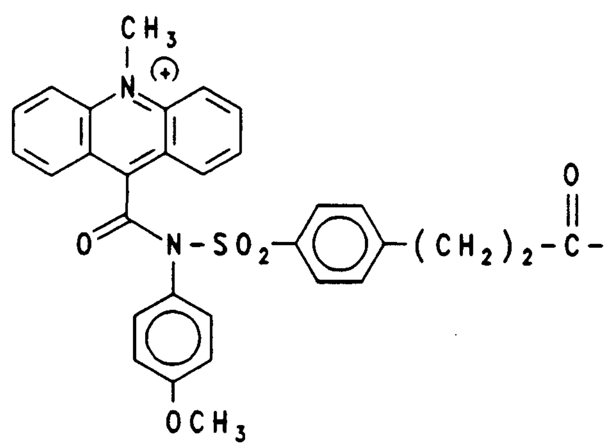

Im Falle der Markierung von (65) mit Ac-Abbott tritt ein mittels HPLC nachweisbares Zerfallsprodukt (peak C, Fig. 4) auf, und zwar zu einem Zeitpunkt, bis zu dem nur wenig Produkt (peak B, Fig. 4) aus dem Edukt (peak A) entstanden ist. (Fig. 4a: Absorptionsmessung nach 2 Tagen, Fig. 4b: Messung nach 3 Tagen, Fig. 4c: Messung nach 4 Tagen.

Nur im Falle des mit Ac-Hoechst markierten Oligonucleotids der Formel (76) ist es gelungen, durch HPLC-Reinigung oder Reinigung mittels Gelelektrophorese ein einheitliches Produkt herzustellen, während sich beispielsweise das mit Ac-Abbott markierte Oligonucleotid, wie im Vergleichsversuch gezeigt, während der Reinigungprozedur bereits wieder zersetzte.

Es wurden noch eine Reihe von Markierungsreaktionen bei unterschiedlichen pH-Werten (pH 6.8, pH 7.3, pH 8.0) sowie in Gegenwart von Pyridin getestet. In allen Fällen war Ac-Hoechst dem Ac-Abbott überlegen. So läßt sich Oligonucleotid (65) nur mit Ac-Hoechst (Fig. 5b) in Pyridin/$H_2O$/Acetonitril (2:2:1, v:v:v)umsetzen, während Ac-Abbott praktisch nicht reagiert (HPLC-Daten s.Fig. 5a). Wiederum ist für Ac-Hoechst kein Zerfallsprodukt in der HPLC nachweisbar.

Beispiele

Beispiel 1:

N-Penyl-N-(4-benzyloxycarbonylbenzolsufonyl)acridin-9-carbonsäureamid(1)

11 g 4-(N-Phenylsulfamido)benzoesäurebenzylester werden in 300 ml Methylenchlorid mit 360 mg 4-(Dimethylamino)pyridin und 16,6 ml Triethylamin versetzt, nach 10 Min. gibt man 8,34 g Acridin-9-carbonsäurechlorid-hyrochlorid zu und erhitzt 16 Std. zum Rückfluß. Die abgekühlte Lösung wird kurz mit 2N NaOH verrührt, die abgetrennte organische Phase mit Wasser gewaschen, über $Na_2SO_4$ getrocknet und eingeengt. Der Rückstand wird aus Toluol/Heptan umkristallisiert.
Ausbeute: 70 % Schmelzpunkt 161 bis 163° C
NMR (DMSO, 100 MHz): $\delta$ = 5,5 ppm (s, 2H), $\delta$ = 6,8 bis 8,6 ppm (m, 22H).

N-Phenyl-N-(4-carboxybenzolsulfonyl)acridin-9-carbonsäureamidhydrobromid(2)

8,58 g N-Phenyl-N-(4-benzyloxycarbonylbenzolsulfonyl)acridin-9-carbonsäureamid (1) werden in 30 ml 33 % HBr in Eisessig 2 Std. auf 60° C erhitzt, nach Abkühlen fügt man 60 ml Diisopropylether hinzu, saugt den Niederschlag ab und trocknet im Vakuum.
Ausbeute: 95 % Schmelzpunkt: 255° C
NMR (DMSO, 100 MHz): $\delta$ = 6,8 bis 9 ppm (m).

N-Phenyl-N-(4-succinimidyloxycarbonylbenzolsulfonyl)acridin-9-carbonsäureamid (3)

5,63 g N-Phenyl-N-(4-carboxybenzolsulfonyl)acridin-9-carbonsäureamidhyrobromid (2) in 250 ml Tetrahydrofuran werden mit 2,8 ml Triethylamin versetzt, auf -15° C abgekühlt und mit 0,96 ml Chlorameisensäureethylester versetzt. Man rührt 20 Min. nach, gibt 1,15 g N-Hydroxysuccinimid zu, rührt 3 Std. bei -15° C, läßt auf Raumtemperatur auftauen und über Nacht nachrühren. Vom Niederschlag wird abgesaugt, das Filtrat eingeengt, der Rückstand mit Methylenchlorid aufgenommen, die resultierende Losung mit Wasser, $NaHCO_3$-Lösung und Wasser gewaschen und über $Na_2SO_4$ getrocknet. Die organische Phase wird eingeengt und der Rückstand aus Toluol umkristallisiert.
Ausbeute: 50 % Schmelzpunkt 226° C (Zersetzung)
NMR (DMSO, 100 MHz): $\delta$ = 2,95 ppm (s, 4H), $\delta$ = 6,8 bis 8,7 ppm (m, 17H).

N-Phenyl-N-(4-succinimidyloxycarbonylbenzolsulfonyl)-10-methylacridinium-9-carbonsäureamid-fluorsulfonat bzw. trifluorracetat (4)

1,16 g N-Phenyl-N-(4-succinimidyloxycarbonylbenzolsulfonyl)acridin-9-carbonsäureamid (3) werden in 60 ml 1,2-Dichlorethan mit 0,3 ml Methylfluorsulfonat 24 Std. bei Raumtemperatur gerührt, der ausfallende Niederschlag wird abgesaugt und im Vakuum getrocknet.
Ausbeute: 65 %
Nach präparativer Mitteldruckchromatographie (MPLC) an RP-18-Kieselgel (Fa. Merck) mit Acetonitril/Wasser-Gemischen, die als Elektrolyt 0,1 Vol-% Trifluoressigsäure enthalten, erhält man das Trifluoracetat als gelbes Pulver.
IR: 3420 $cm^{-1}$(br), 3100(br), 1805(w), 1770(m), 1745(s), 1700(m), 1385(m), 1280(m), 1255(s), 1230(s), 1205-(s).
NMR (DMSO, 100 MHz): $\delta$ = 2,95 ppm (s,4H), $\delta$ = 4,75 ppm (s, br, 3H), $\delta$ = 7,0 bis 9,0 ppm (m, 17H).
Massenspektrum: m/z = 594: $M^+$ (Kation)

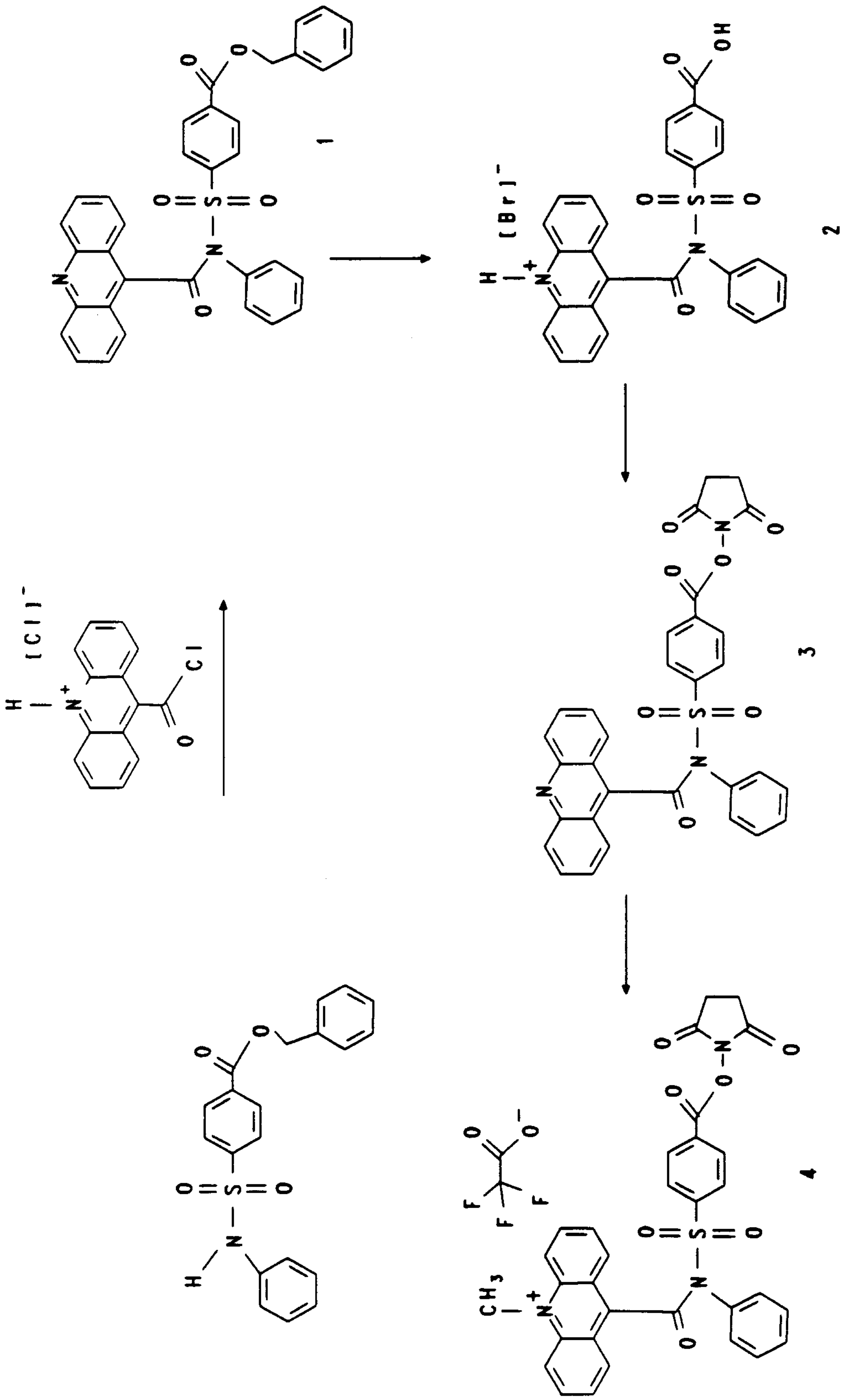

1

2

3

4

Beispiel 2:

Die Darstellung von N-(4-Methoxyphenyl)-N-(4-succinimidyloxycarbonyl-benzolsulfonyl)-10-methylacridinium-9-carbonsäureamid-fluorsulfonat bzw. -trifluoracetat (8) ausgehend von 4-[N-(4'-Methoxyphenyl)-

sulfamido]-benzoesäurebenzylester und Acridin-9-carbonsäurechlorid-hydrochlorid erfolgt analog der Synthese von (4) (s. Beispiel 1). Die Ausbeuten der einzelnen Syntheseschritte sowie die spektroskopische Charakterisierung sind nachfolgend angegeben.

N-(4-Methoxyphenyl)-N-(4-benzyloxycarbonyl-benzolsulfonyl)acridin-9-carbonsäureamid (5)

Ausbeute: 70 % Schmelzpunkt: 182 bis 183° C
NMR (DMSO, 100 MHz): δ = 3,5 ppm (s, 3H), δ = 5,5 ppm (s, 2H), δ = 6,35 bis 6,63 ppm (d, br, 2H), δ = 7,05 bis 7,2 ppm (d, br, 2H), δ = 7,35 bis 8,5 ppm (m, 17H).

N-4-Methoxyphenyl)-N-4-(carboxybenzolsulfonyl)acridin-9-carbonsäureamid-hydrobromid (6)

Ausbeute: 95 % Schmelzpunkt: 273° C (Zersetzung)
NMR (DMSO, 100 MHz): δ = 3,5 ppm (s,3H), δ = 6,4 bis 6,6 ppm (d, br, 2H), δ = 7,05 bis 7,2 ppm (d, br, 2H), δ = 7,7 bis 8,5 ppm (m, 12H).

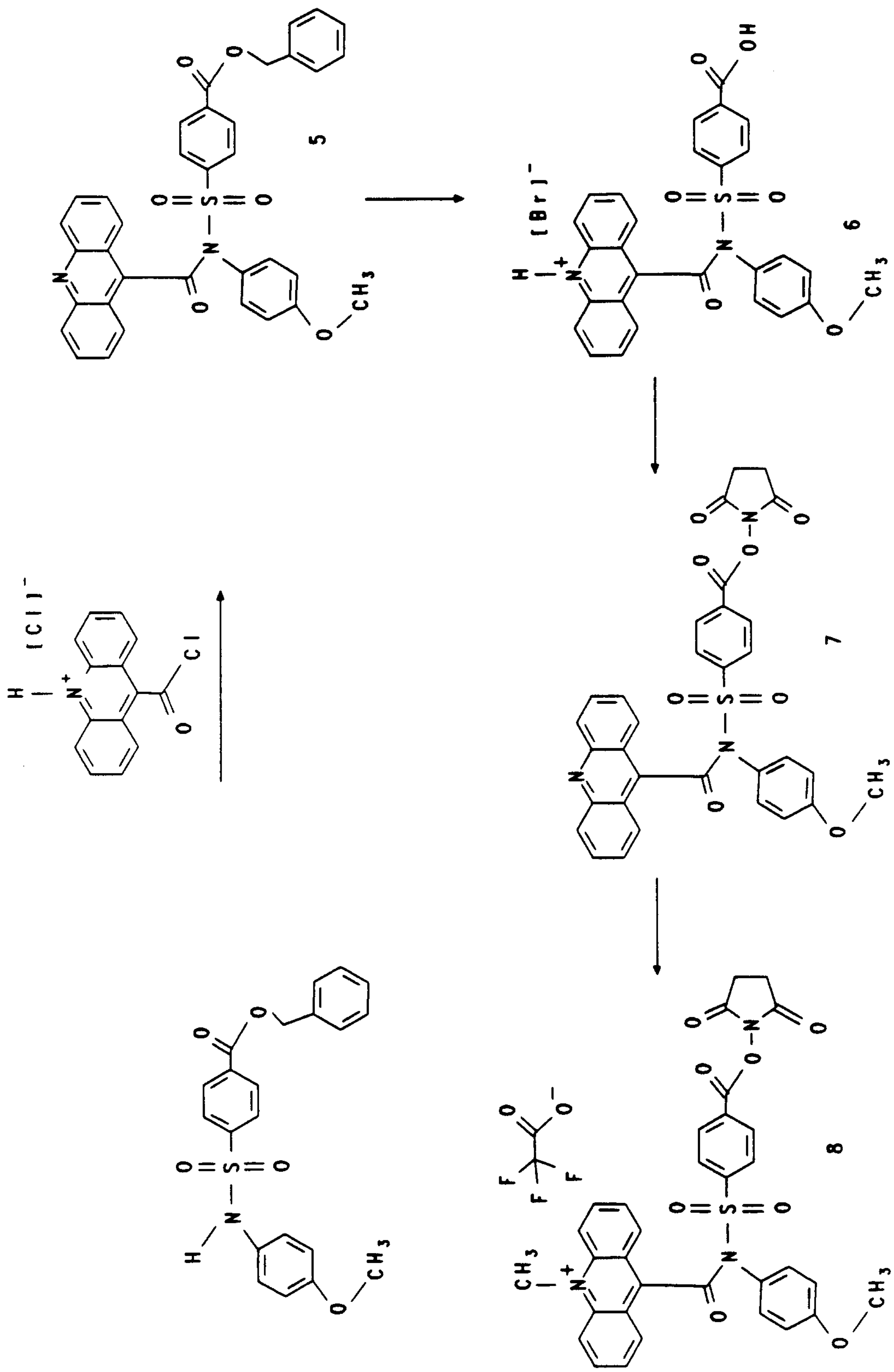

5

6

7

8

N-(4-Methoxyphenyl)-N-(4-succinimidyloxycarbonylbenzolsulfonyl)acridin-9-carbonsäureamid (7)

Ausbeute: 50 % Schmelzpunkt: 232 bis 234° C

NMR (DMSO, 100 MHz): $\delta$ = 2,95 ppm (s, 4H), $\delta$ = 3,5 ppm (s, 3H), $\delta$ = 6,4 bis 6,6 ppm (d, br, 2H), $\delta$ =

7,05 bis 7,25 ppm (d, br, 2H), δ = 7,8 bis 8,6 ppm (m, 12H).
IR: 3050 $cm^{-1}$ 1805(w), 1780(m), 1740(s), 1700(m), 1505(m), 1370(m), 1250(m), 1200(s), 1185.

N-(4-Methoxyphenyl)-N-(4-succinimidyloxycarbonylbenzolsulfonyl)-10-methylacridinium-9-carbonsäureamid-fluorsulfonat bzw. trifluoracetat (8)

Die Substanz fällt bei der Reaktion nicht aus, sie wird durch Einengen der Lösung und Verrühren des Rückstandes mit Diisopropylether gewonnen.
Ausbeute: 80 %
Nach präparativer Mitteldruckchromatographie (MPLC) an RP-18-Kieselgel (Fa. Merck) mit Acetonitril/Wasser-Gemischen, die als Elektrolyt 0,1-Vol-% Trifluoressigsäure enthalten, erhält man das Trifluoroacetat als gelbes Pulver.
NMR (DMSO, 100 MHz): δ = 2,95 ppm (s, 4H), δ = 3,5 ppm (s, 3H), δ = 4,8 ppm (s, br,3H), δ = 6,45 bis 6,7 ppm (d, br, 2H), δ = 7,2 bis 7,4 ppm (d, br, 2H), δ = 7,7 bis 9 ppm (m, 12H).
Massenspektrum: m/z = 624 $M^+$ (Kation)
IR: 3440 $cm^{-1}$ (br), 3100, 2950, 1805(w), 1775(m), 1740(s), 1695(m), 1610(m), 1505(m), 1375(m), 1280(m), 1250(s), 1205(s).

Beispiel 3:

Die Darstellung von N-(4-Methoxyhenyl)-N-(3-succinimidyloxycarbonylbenzolsulfonyl)-10-methylacridinium-9-carbonsäureamid-fluorsulfonat bzw. -trifluoracetat (12) ausgehend vom 3-[N-(4'-Methoxyphenyl)-sulfamido]-benzoesäurebenzylester und Acridin-9-carbonsäurechlorid-hydrochlorid erfolgt analog zur Synthese von (4) (s. Beispiel 1). Die Ausbeute der einzelnen Syntheseschritte sowie die spektroskopische Charakterisierung sind nachfolgend angegeben.

N-(4-Methoxyphenyl)-N-(3-benzyloxycarbonylbenzolsulfonyl)acridin-9-carbonsäureamid (9)

Ausbeute: 70 % Schmelzpunkt 168 bis 170 ° C
NMR (DMSO, 100 MHz): δ = 3,5 ppm (s, 3H), δ = 5,45 ppm (s, 2H), δ 6,5 ppm (s, br, 2H), δ = 7,1 ppm (br, 2H), δ = 7,3 bis 8,8 ppm (m, 17H).

N-(4-Methoxyphenyl)-N-(3-carboxybenzolsulfonyl)acridin-9-carbonsäureamidhydrobromid (10)

Ausbeute: 90 % Schmelzpunkt: 264 ° C
NMR (DMSO, 100 MHz): δ = 3,5 ppm (s, 3H), δ = 6,4 bis 6,6 ppm (d, br, 2H), δ = 7,0 bis 7,2 ppm (d, br, 2H), δ = 7,6 bis 8,8 ppm (m, 12H).

N-(4-Methoxyphenyl)-N-(3-succinimidyloxycarbonylbenzolsulfonyl)acridin-9-carbonsäureamid (11)

Ausbeute 50 % Schmelzpunkt: 223 bis 225 ° C
NMR (DMSO, 100 MHz): δ = 2,95 ppm (s, 4H), δ = 3,5 ppm (s, 3H), δ = 6,4 bis 6,6 ppm (d, br, 2H), δ = 7,0 bis 7,2 ppm (d, br, 2H), δ = 7,4 bis 8,9 ppm (m, 12H).
IR: 3500 $cm^{-1}$ (br), 3060, 2950, 2840, 1805(w), 1785(m), 1740(s), 1700(m), 1510(m), 1380(m), 1250(m), 1205(m), 1165(m).

N-(4-Methoxyphenyl)-N-(3-succinimidyloxycarbonylbenzolsulfonyl)-10-methylacridinium-9-carbonsäureamidfluorsulfonat bzw. trifluoracetat (12)

Ausbeute 90 %
NMR (DMSO, 100 MHz): δ = 2,95 ppm (s, 4H), δ = 3,55 ppm (s, 3H), δ = 4,8 ppm (s, br,3H), δ = 6,45 bis 6,7 ppm (d, br, 2H), δ = 7,05 bis 7,3 ppm (d, br, 2H), δ = 7,5 bis 8,9 ppm (m, 12H).
IR: 3500 $cm^{-1}$ (br), 3080, 2950, 1805(w), 1780(m), 1740(s), 1700(m), 1610(m), 1510(m), 1380(m), 1250(s), 1205(s), 1170(s).
Masse: m/z = 624 $M^+$ (Kation)
Nach präparativer Mitteldruckchromatographie (MPLC) an RP-18-Kieselgel (Fa. Merck) mit Acetonitril/Wasser-Gemischen, die als Elektrolyt 0,1-Vol-% Trifluoressigsäure enthalten, erhält man aus dem rohen Fluorsulfonat das Trifluoracetat als gelbes Pulver.

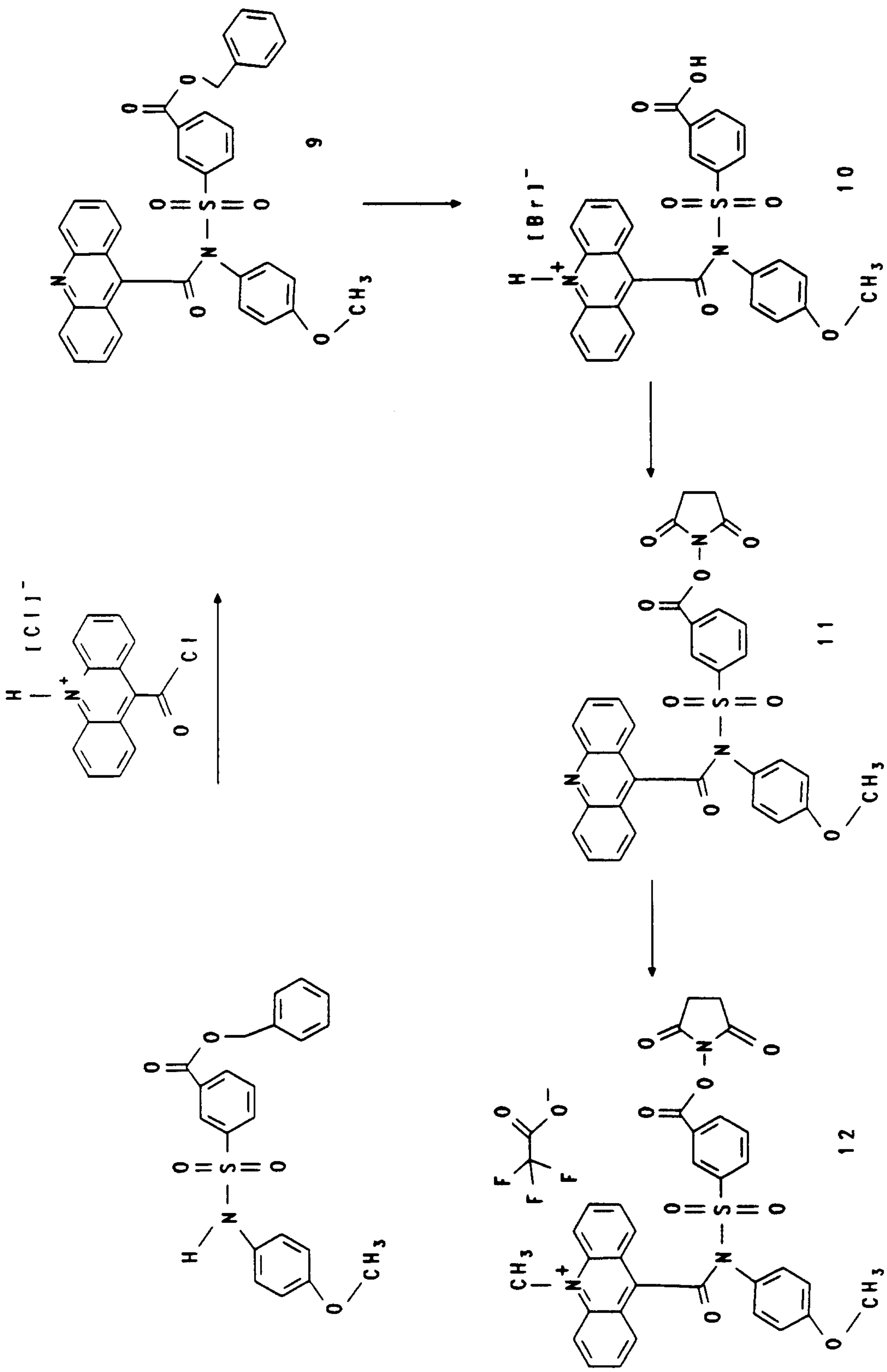

9

10

11

12

Beispiel 4:

N-(4-Methoxyphenyl)-N[4-(2-benzyloxycarbonylethyl)benzolsufonyl]acridin-9-carbonsäureamid (13)

17 g 4'-[N-(4-methoxyphenyl)sulfamido]-3-phenylpropion säurebenzylester in 400 ml Dichlormethan werden mit
460 mg 4-(N,N-Dimethylamino)pyridin und 22,1 ml Triethylamin versetzt, nach 10 Min. gibt man 11,12 g Acridin-9-carbonsäurechlorid-hydrochlorid zu und erhitzt 6 Std. zum Rückfluß. Die abgekühlte Lösung wird kurz mit 2 N NaOH verrührt, die abgetrennte organische Phase mit $H_2O$ gewaschen, über Magnesiumsulfat getrocknet und eingeengt. Der Rückstand wird durch Säulenchromatographie gereinigt.

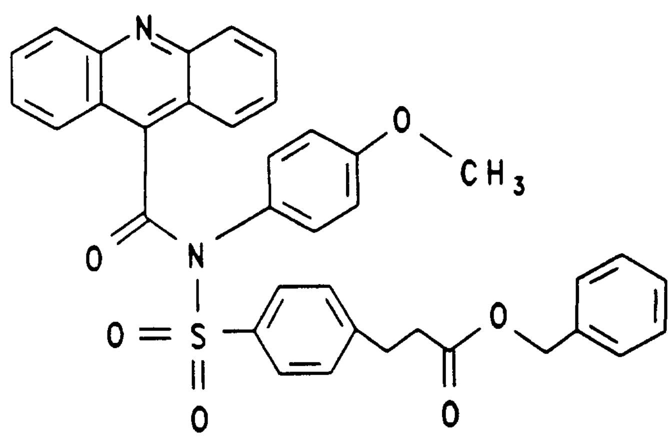

(13)

Ausbeute: 60 % Schmelzpunkt: 130 bis 132° C
NMR(DMSO, 100 MHz): δ = 2,7 bis 3,0 ppm (d, br, 2H), δ = 3,0 bis 3,3 ppm (d, br, 2H), δ = 5,1 ppm (s, 2H), δ = 6,5 ppm (d, br, 2H), δ = 7,1 ppm (d, br, 2H), δ = 7,35 ppm (s, 5H), δ = 7,5 bis 8,3 ppm (m, 12H).

N-(4-Methoxyphenyl)-N-[4-(2-carboxyethyl)benzolsulfonyl]acridin-9-carbonsäureamid-hydrobromid (14)

6,3 g (13) werden in 30 ml 33 % HBr/Eisessig 2 Std. auf 60° C erhitzt. Nach Abkühlen fügt man 60 ml Diisopropylether hinzu, saugt den Niederschlag ab und trocknet im Vakuum.

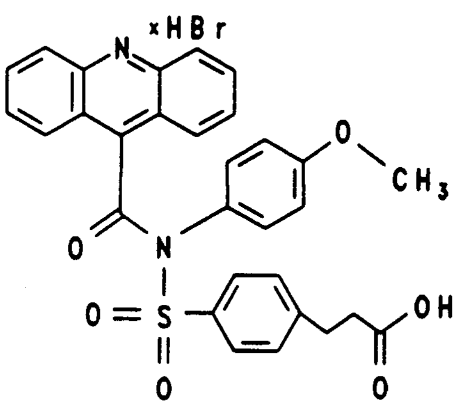

(14)

Ausbeute: 90 % Schmelzpunkt: Zersetzung 237° C
NMR(DMSO, 100 MHz): δ = 2,7 ppm (d, br, 2H), δ = 3,1 ppm (d, br, 2H), δ = 3,5 ppm (s, br, 3H), δ = 6,5 ppm (d, br, 2H), δ = 7,0 bis 8,4 ppm (m, 15H).

N-(4-Methoxyphenyl)-N-[4-(2-succinimidyloxycarbonylethyl)benzolsulfonyl]acridin-9-carbonsäureamid (15)

3,1 g (14) in 50 ml Tetrahydrofuran werden mit 1,41 ml Triethylamin versetzt, auf -20° C abgekühlt und mit 0,474 ml Chlorameisensäureethylester versetzt. Man rührt 20 Min. nach, gibt 575 mg N-Hydroxysuccinimid zu, rührt 3 Std. bei - 20° C und läßt über Nacht unter Rühren auf Raumtemperatur kommen. Vom Niederschlag wird abgesaugt, das Filtrat eingeengt, der Rückstand in Dichlormethan oder Ethylacetat aufgenommen, die resultierende Losung mit Wasser, $NaHCO_3$-Lösung und Wasser gewaschen und über $MgSO_4$ getrocknet. Die

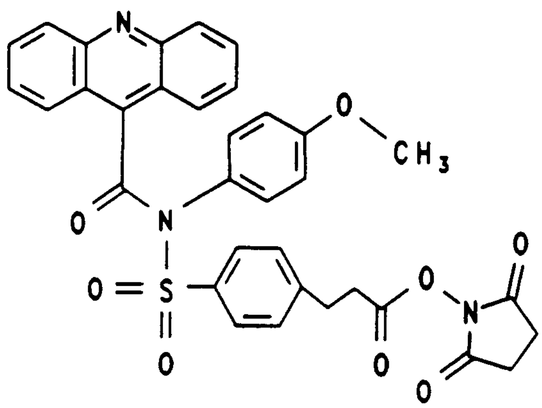

(15)

organische Phase wird eingeengt und der Rückstand aus Toluol umkristallisiert.
Ausbeute: 50 %
NMR (DMSO, 100 MHz): δ = 2,8 ppm (s, 4H), δ = 3,2 ppm (s, br, 4H), δ = 3,5 ppm (s, br, 3H), δ = 6,5 ppm (d, br, 2H), δ = 7,2 ppm (d, br, 2H), δ = 7,6 bis 8,4 ppm (m, 12H).
IR: 3400 $cm^{-1}$ (br), 3060, 2930, 1815(w), 1780(w), 1740(s), 1690(m), 1600(w), 1510(m), 1370(m), 1250(m), 1203(m), 1175(m).

N-(4-Methoxyphenyl)-N-[4-(2-succinimidyloxycarbonylethyl)benzolsulfonyl]-10-methylacridinium-9-carbonsäureamidfluorsulfonat bzw. trifluoracetat (16).

1,27 g (15) in 60 ml Dichlormethan werden bei -20° C mit 0,4 ml Methylfluorosulfonat versetzt. Man läßt 2 Std. bei -20° C rühren und über Nacht auf Raumtemperatur auftauen. Auf Zusatz von Toluol fällt ein gelber Feststoff aus, der abgesaugt und im Vakuum getrocknet wird.
Ausbeute: 80 %
Hochreines (16) erhält man nach präp. MPLC wie in den Beispielen 1 bis 3 beschrieben als Trifluoracetat.
NMR (DMSO, 100 MHz): δ = 2,9 ppm (s, 4H), δ = 3,2 ppm (s, br, 4H), δ = 3,5 ppm (s, br, 3H), δ = 4,8 ppm (s, br, 3H), δ = 6,5 ppm (br, 2H), δ = 7,2 ppm (br, 2H), δ = 7,6 bis 9,0 ppm (m, 12H).
IR: = 3400 $cm^{-1}$ (br), 3160, 2970, 1810(w), 1785(w), 1740(s), 1695(m), 1600(w), 1555(w), 1510(m), 1370-(m), 1290(m), 1250(m), 1210(m), 1170(m).
Massenspektrum: m/z: 652 $M^+$ (Kation)

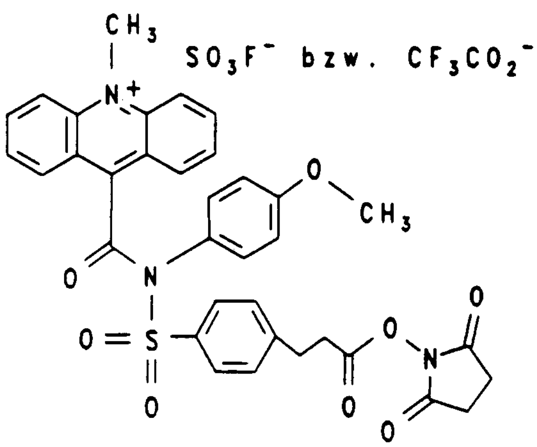

(16)

Beispiel 5:

Die Darstellung von N-(4-methoxyphenyl)-N-[4-(4-succinimidyloxycarbonylbutyl)benzolsulfonyl]-10-methylacridinium-9-carbonsäureamidfluorsulfonat (20) ausgehend von 4-[N-(4-Methoxyphenyl)sulfamido]-5-phenylvaleriansäurebenzylester und Acridin-9-carbonsäurechlorid-hydrochlorid erfolgt analog zur Synthese von (16). Die Ausbeuten der einzelnen Syntheseschritte sowie die spektroskopische Charakterisierung sind nachfolgend angegeben.

N-(4-Methoxyphenyl)-N-[4-(4-benzyloxycarbonylbutyl)benzolsulfonyl]acridin-9-carbonsäureamid (17)

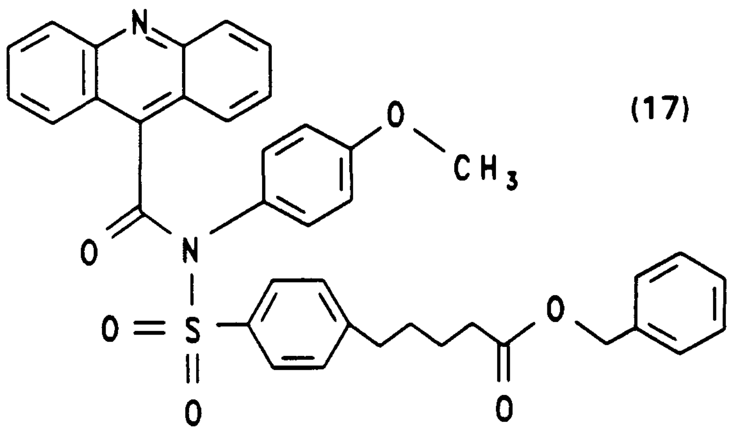

(17)

Ausbeute: 40 % zähes Öl erstarrt teilweise
NMR ($CDCl_3$, 100 MHz): $\delta$ = 2,85 (m, 4H), $\delta$ = 2,45 ppm (t, br, 2H), $\delta$ = 2,8 ppm (t, br, 2H), $\delta$ = 3,5 ppm (s, 3H), $\delta$ = 5,15 ppm (s, 2H), $\delta$ = 6,3 ppm (d, 2H), $\delta$ = 6,9 ppm (d, 2H), $\delta$ = 7,3 bis 8,3 ppm (m, 17H).

N-(4-Methoxyphenyl)-N-[4-(4-carboxybutyl)benzolsulfonyl]acridin-9-carbonsäureamid-hydrobromid (18)

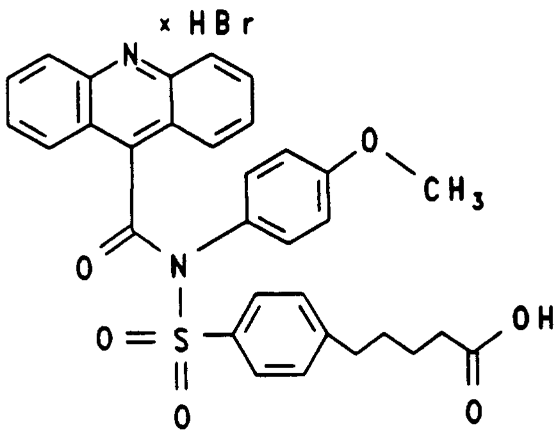

(18)

Ausbeute: 95 % Schmelzpunkt: Zersetzung 153 bis 155° C
NMR (DMSO, 100 MHz): $\delta$ = 1,7 ppm (s, br, 4H), $\delta$ = 2,3 (m, br, 2H), $\delta$ = 2,8 ppm (s, br, 2H), $\delta$ = 3,5 ppm (s, 3H), $\delta$ = 6,5 ppm (br, 2H), $\delta$ = 7,05 ppm (br, 2H), $\delta$ = 7,5 bis 8,5 ppm (m, 12H).

N-(4-Methoxyphenyl)-N-[4-(4-succinimdyloxycarbonylbutyl)benzolsulfonyl]acridin-9-carbonsäureamid (19)

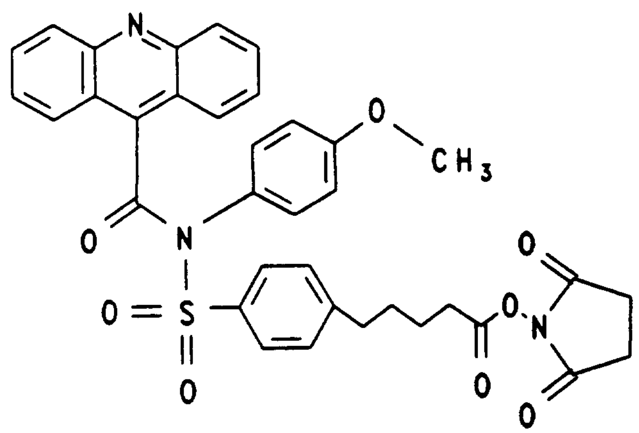

(19)

Ausbeute: 25 % Schmelzpunkt: Zersetzung 75 bis 80° C
NMR (DMSO, 100 MHz): δ = 1,8 ppm (br, 4H), δ = 2,3 ppm (s, 2H), δ = 2,85 ppm (s, br, 6H), δ = 3,5 ppm (s, br, 3H), δ = 6,5 ppm (d, br, 2H), δ = 7,05 ppm (d, br, 2H), δ = 7,5 bis 8,3 ppm (m, 12H).

N-(4-Methoxyphenyl)-N-[4-(4-succinimidyloxycarbonylbutyl)benzolsulfonyl]-10-methylacridinium-9-carbonsäureamidfluorsulfonat (20)

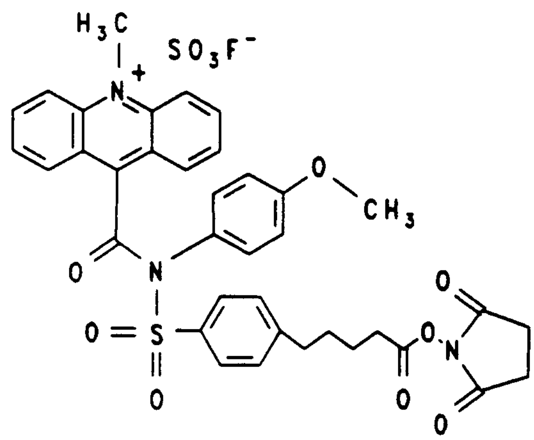

(20)

Ausbeute: 90 %
NMR (DMSO, 100 MHz): δ = 1,8 ppm (br, 4H), δ = 2,3 ppm (s, br, 2H), δ = 2,8 ppm (s, br, 6H), δ = 3,5 ppm (s, br, 3H), δ = 4,8 ppm (br, 3H), δ = 6,5 ppm (br, 2H), δ = 7,05 ppm (br, 2H), δ = 7,5 bis 9,0 ppm (m, 12H).
IR: 3340 $cm^{-1}$ (br), 3060(w), 2930(m), 2870(w), 1810(w), 1785(w), 1740(s), 1695(m), 1600(w), 1550(w), 1510-(m), 1460(w), 1370(m), 1290(s), 1250(s), 1205(s), 1170(s).
Massenspektrum: m/z = 680 $M^+$ (Kation)

Beispiel 6:

Die Darstellung von N-(2,4-Dimethoxyphenyl)-N-[4-(2-succinimidyloxycarbonylethylbenzolsulfonyl]-10-methylacridinium-9-carbonsäureamid-fluorsulfonat (24) ausgehend von 4'-[N-(2,4-Dimethoxyphenyl)-sulfamido]-3-phenylpropionsäurebenzylester und Acridin-9-carbonsäurechloridhydrochlorid erfolgt analog zur Synthese von (16). Die Ausbeuten der einzelnen Syntheseschritte sowie die spektroskopische Charakterisierung sind nachfolgend angegeben.

N-(2,4-Dimethoxyphenyl)-N-[4-(2-benzyloxycarbonylethyl)benzolsulfonyl]acridin-9-carbonsäureamid (21)

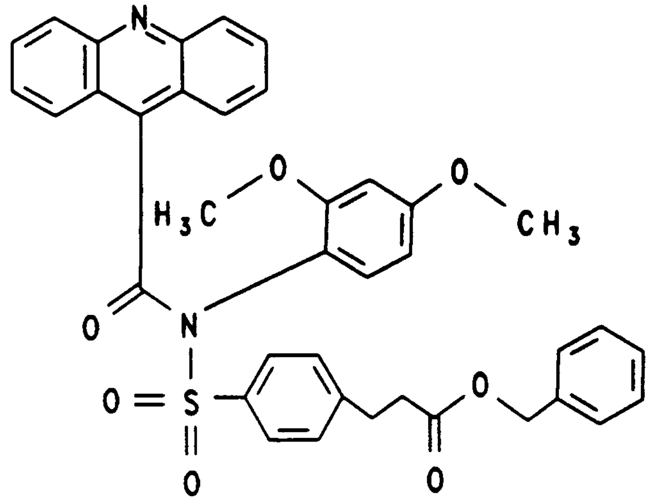

(21)

Ausbeute: 50 % Schmelzpunkt: 74° C
NMR (DMSO, 100 MHz): δ = 2,9 ppm (d, br, 2H), δ = 3,1 ppm (d, br, 2H), δ = 3,3 ppm (s, 3H), δ = 3,4 ppm (s, 3H), δ = 5,1 ppm (s, 2H), δ = 5,9 bis 6,2 ppm (m, 2H), δ = 7,0 ppm (d, 1H), δ = 7,35 ppm (s, 5H), δ = 7,5 bis 8,2 ppm (m, 12H).

N-(2,4-Dimethoxyphenyl)-N-[4-(2-carboxyethyl)benzolsulfonyl]acridin-9-carbonsäureamid-hydrobromid (22)

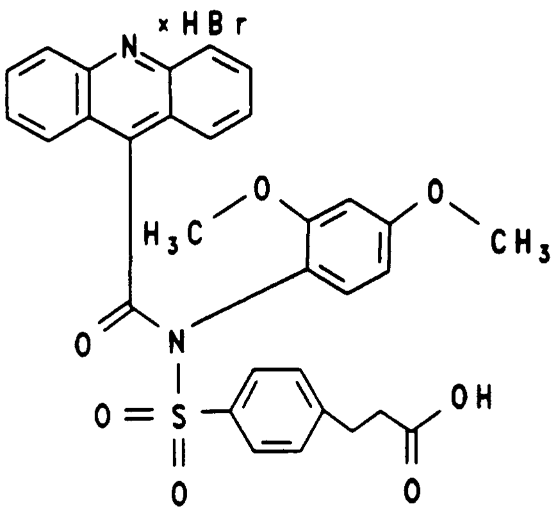

(22)

Ausbeute: 95 %
NMR (DMSO, 100 MHz): δ = 2,75 ppm (d, br, 2H), δ = 3,05 ppm (d, br, 2H), δ = 3,3 ppm (s, 3H), δ = 3,5 ppm (s, 3H), δ = 5,95 bis 6,3 ppm (m, 2H), δ = 7,05 ppm (d, 1H), δ = 7,6 bis 8,6 ppm (m, 12H), δ = 9,2 ppm (s, br, 2H).

N-(2,4-Dimethoxyphenyl)-N-[4-(2-succinimidyloxycarbonylethyl)benzolsulfonyl]acridin-9-carbonsäureamid (23)

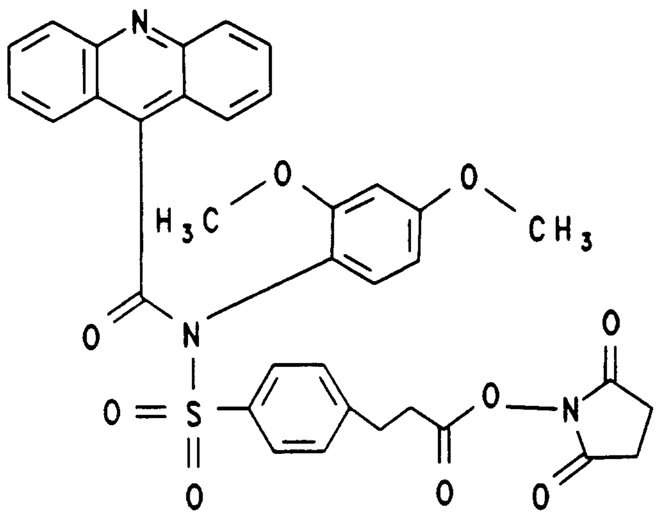

(23)

Ausbeute: 45 % Schmelzpunkt: ~ 105 ° C Zersetzung
NMR (DMSO, 100 MHz): δ = 2,9 ppm (s, 4H), δ = 3 ppm (br, 2H), δ = 3,2 ppm (s, 3H), δ = 3,4 ppm (s, 3H), δ = 5,9 bis 6,3 ppm (m, 2H), δ = 7,0 ppm (d, 1H), δ = 7,5 bis 8,4 ppm (m, 12H).
IR (KBr-Pressling): 3440 $cm^{-1}$ (br), 3060(w), 2930(w),
2850(w), 1815(w), 1785(w), 1740(s), 1695(m), 1600(w), 1510(m), 1460(w), 1440(w), 1365(m), 1320(w), 1290-(w), 1240(m), 1210(s), 1165(m), 1085(m).

N-(2,4-Dimethoxyphenyl)-N-[(2-succinimidyloxycarbonylethyl)benzolsulfonyl]-10-methylacridinium-9-carbonsäureamid-fluorsulfonat (24) bzw. trifluoracetat

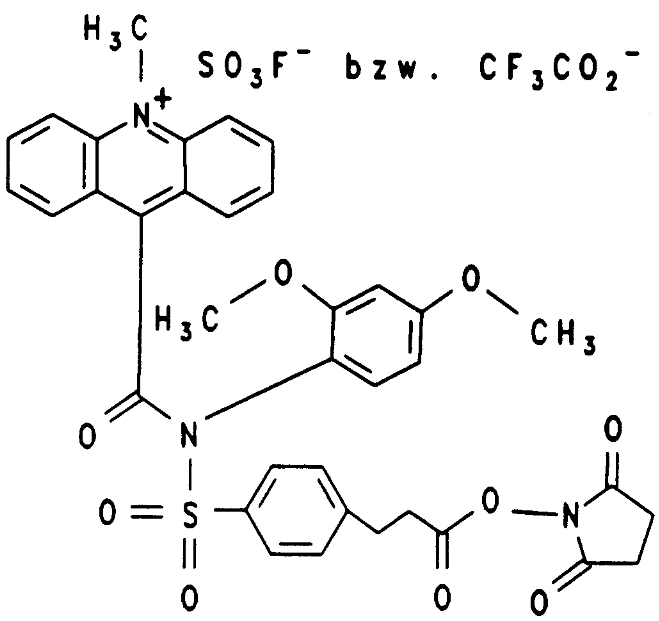

(24)

Ausbeute: 80 % Schmelzpunkt: ~ 135 ° C Zersetzung
NMR (DMSO, 100 MHz): δ = 2,9 ppm (s, 4H), δ = 2,95 bis 4,2 ppm (m, 10H), δ = 4,8 bis 5,0 ppm (s, br,

3H), δ = 6,05 bis 6,25 ppm (m, 1H), δ = 7,6 bis 9,0 ppm (m, 14H).
IR (KBr-Pressling): 3430 $cm^{-1}$ (m), 2950(w), 2870(w), 2825(w), 1810(w), 1780(m), 1750(s), 1695(m), 1610-(m), 1555(w), 1510(m), 1465(m), 1380(m), 1285(m), 1250(m), 1210(s), 1170(m).
Hochreines (24) als Trifluoracetat erhält man durch präparative MPLC wie in den Beispielen 1 bis 3 beschrieben.

Beispiel 7:

Die Synthese von N-(3,4-Ethylendioxyphenyl)-N-[4-(2-succinimidyloxycarbonylethyl)benzolsulfonyl]-10-methylacridinium-9-carbonsäureamid-fluorsulfonat (28) ausgehend von 4'-[N-(3,4-Ethylendioxyphenyl)-sulfamido]-3-phenylpropionsäurebenzylester und Acridin-9-carbonsäurechloridhydrochlorid erfolgt analog Beispiel 4. Die Ausbeuten der einzelnen Schritte sowie die spektroskopische Charakterisierung sind nachfolgend angegeben.

N-(3,4-Ethylendioxyphenyl)-N-[4-(2-benzyloxycarbonylethyl)benzolsulfonyl]acridin-9-carbonsäureamid (25)

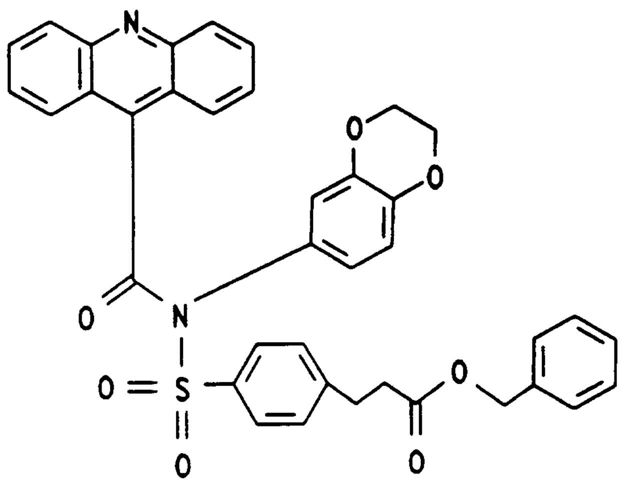

(25)

Ausbeute: 50 % Schmelzpunkt: 91,5° C
NMR (DMSO, 100 MHz): δ = 2,9 ppm (d, br, 2H), δ = 3,1 ppm (d, br, 2H), δ = 4,0 ppm (s, br, 4H), δ = 5,1 ppm (s, 2H), δ = 6,3 bis 6,8 ppm (m, 3H), δ = 7,3 ppm (s, 5H), δ = 7,6 bis 8,3 ppm (m, 12H).

N-(3,4-Ethylendioxyphenyl)-N-[4-(2-carboxyethyl)benzolsulfonyl]acridin-9-carbonsäureamid-hydrobromid (26)

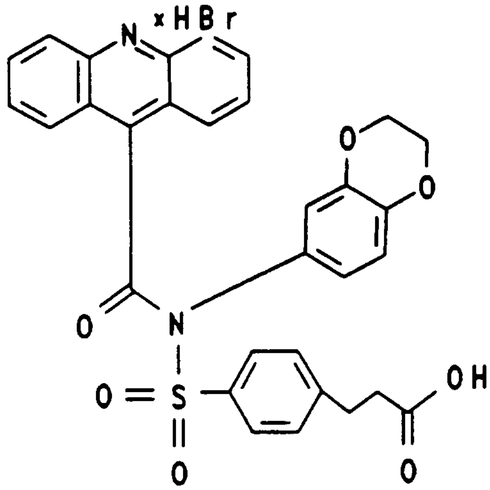

(26)

Ausbeute: 95 % Schmelzpunkt: > 200 ° C
NMR (DMSO, 100 MHz): δ = 2,7 ppm (m, 2H), δ = 3,05 ppm (m, 2H), δ = 4,0 ppm (s, br, 4H), δ = 6,3 bis 6,8 ppm (m, 3H), δ = 7,5 bis 8,6 ppm (m, 12H), δ = 9,6 ppm (s, br, 2H).

N-(3,4-Ethylendioxyphenyl)-N-[4-(2-succinimidyloxycarbonylethyl)benzolsulfonyl]acridin-9-carbonsäureamid (27)

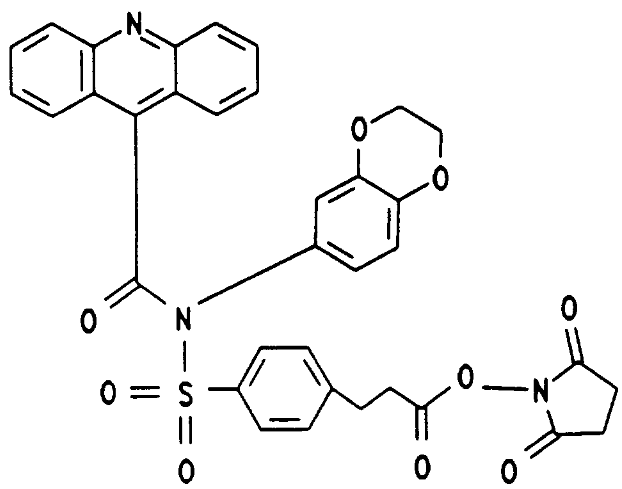

(27)

Ausbeute: 45 % Schmelzpunkt: 140 ° C Zersetzung
NMR (DMSO, 100 MHz): δ = 2,7 bis 2,9 ppm (d, s, überlagert, 6H), δ = 3,0 ppm (d, 2H), δ = 4,0 ppm (s, br, 4H), δ = 6,3 bis 6,8 ppm (m, 3H), δ = 7,5 bis 8,4 ppm (m, 12H).
IR (KBr-Pressling): 3420 $cm^{-1}$ (br), 3060(m), 2980(m), 2980(m), 1810(w), 1790(w), 1740(m), 1695(s), 1590-(m), 1460(w), 1430(w), 1410(w), 1370(m), 1300(m), 1225(s), 1175(s).

N-(3,4-Ethylendioxyphenyl)-N-[4-(2-succinimidyloxycarbonylethyl)-benzolsulfonyl]-10-methylacridinium-9-carbonsäureamid-fluorsulfonat (28)

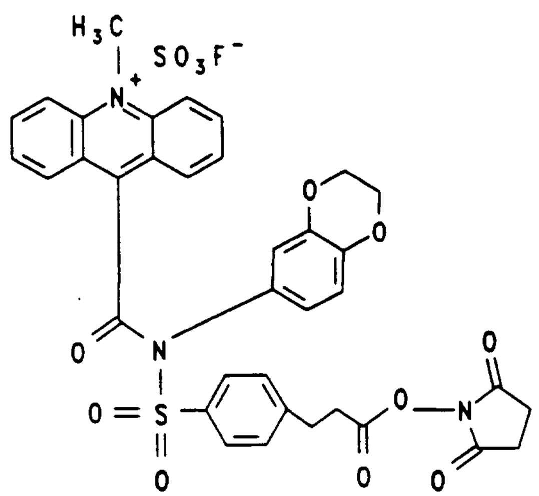

(28)

Ausbeute: 80 % Schmelzpunkt: 110 ° C, Zersetzung
NMR (DMSO, 100 MHz): δ = 2,85 ppm (s, 4H), δ = 3,0 bis 3,3 ppm (s, s, br, 4H), δ = 3,8 bis 4,5 ppm (m, br, 4H), δ = 4,75 bis 5,1 ppm (s, br mit Schulter, 3H), δ = 6,3 bis 9,0 ppm (m, 15H).

Beispiel 8:

N-(4-Carboxyphenyl)-4-toluolsulfonsäureamid (29)

Zu einer Mischung von 252 g (3 Mol) Natriumhydrogencarbonat und 139,1 g (1 Mol) 4-Aminobenzoesäure in 2,5 l Wasser tropft man bei 20 bis 30° C eine solche von 190,5 g (1 Mol) 4-Toluolsulfochlorid in 300 ml i-Propylether. Man rührt intensiv 2 bis 4 Std., bis das Sulfochlorid verbraucht ist. Die abgetrennte wäßrige Lösung stellt man mit konz. Salzsäure auf pH 1 und nimmt die Fällung in Propylacetat auf. Den Extrakt wäscht man 2x mit 2n-Salzsäure, 1x mit Wasser aus, trocknet über Natriumsulfat und dampft ein. Man erhält 240 g (82,5 % d. Th.) N-(4-Carboxyphenyl)-4-toluolsulfonsäureamid

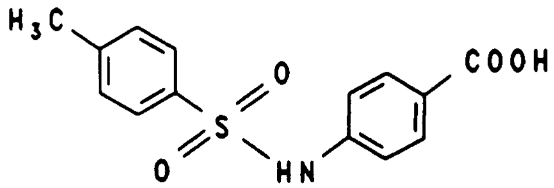

(29)

$^1$H-NMR (DMSO $d_6$): δ = 2,3 (s, CH), 7,1 (d, Aromat, 2H), 7,3 (d, Aromat, 2H), 7,6 bis 7,9 (m, Aromat, 4H), 10,75 (breit), 12,7 (breit).

N-(4-Benzyloxycarbonylphenyl)-4-toluolsulfonsäureamid (30)

Eine Lösung von 11,64 g (40 mMol) N-(4-Carboxyphenyl)-4-toluolsulfonsäureamid, 5,06 g (40 Mmol) Benzylchlorid, 5,20 g (44 Mmol) Di-i-propyl-ethylamin in 100 ml Dimethylformamid erhitzt man 4 Std. auf 140° C. Nach beendeter Reaktion dampft man in Vakuum ein. Man erhält 11,8 g (78 % d. Th.) N-(4-Benzyloxycarbonylphenyl)-4-toluolsulfonsäureamid, der aus Methanol umkristallisiert wird.

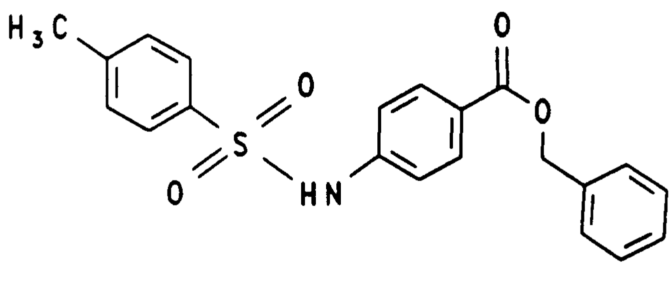

(30)

$^1$H-NMR (DMSO) $d_6$): δ = 2,3 (s, $CH_3$), 5,3 (s, $CH_2$), 7,1 bis 7,3 (dd, 4 aromat. H), 7,4 (s, $C_6H_5$), 7,7 bis 7,9 (dd, 4 aromat. H), 10,8 (breit, NH).

N-(4-Benzyloxycarbonylphenyl)-N-(4-toluolsulfonyl)-acridin-9-carbonsäureamid (31)

Zu der Lösung von 1,5 g (4 Mmol) N-(4-Benzyloxycarbonylphenyl)-4-toluolsulfonsäureamid, 1,23 g (4,4 Mmol) Acridincarbonsäurechloridhydrochlorid und 0,02 g Dimethylaminopyridin in 20 ml wasserfreiem Tetrahydrofuran tropft man bei 25° C 2,1 ml (15 Mmol) Triethylamin in 10 ml Tetrahydrofuran. Man steigert die Temperatur auf 60° C.

Das auskristallisierende Produkt wird nach beendeter Reaktion mit Methanol verrührt, abgesaugt und aus Ethylacetat umkristallisiert.
Ausbeute: 1,57 g (67,0 % d. Th.)

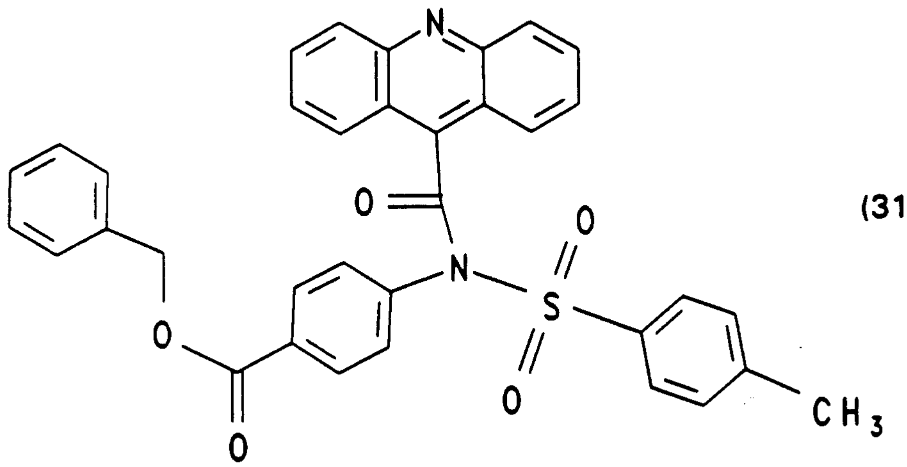

(31)

$^1$H-NMR ($CDCl_3$): $\delta$ = 2,5 (s, $CH_3$), 5,2 (s, $CH_2$), 7,3 (s, $C_6H_5$), 7,0 bis 8,2 (m, 16 aromat. H).

N-(4-Carboxyphenyl)-N-(4-toluolsulfonyl)-acridin-9-carbonsäureamid-hydrobromid (32)

1,17 g (2 mMol) N-(4-Benzyloxycarbonylphenyl)-N-(4-toluolsulfonyl)-acridin-9-carbonsäureamid werden mit 10 ml 33 %iger Bromwasserstoff-Eisessiglösung unter Rühren 4 Std. auf 60° C erwärmt. Nach Abkühlung saugt man die Fällung ab und trocknet i. V.

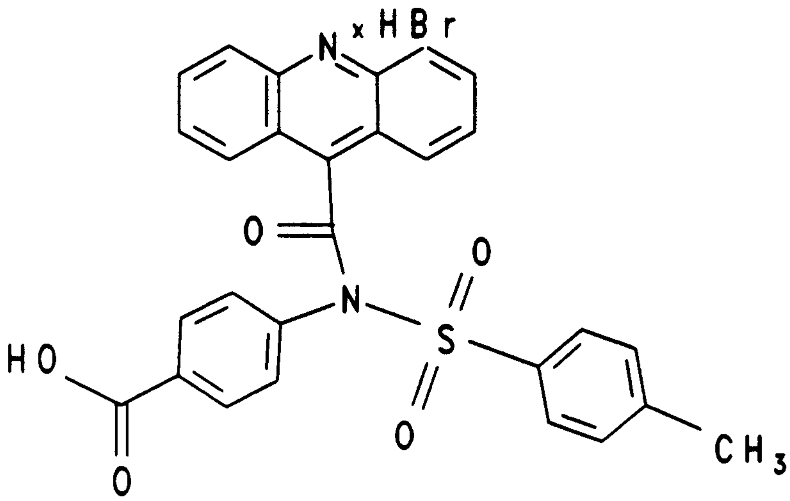

(32)

Ausbeute: 1,00 g (87 % d. Th.)
$^1$H-NMR (TFA): $\delta$ = 2,6 (s, $CH_3$), 7,3 bis 8,6 (m, 16 aromat. H), 11,65 (S, NH).
MS: 496 ($M^+$)

N-(4-Succinimidyloxycarbonylphenyl)-N-(4-toluolsulfonyl)acridin-9-carbonsäureamid (33)

Zu einer Lösung von 0,57 g (1 mMol) N-(4-Carboxyphenyl)-N-(4-toluolsulfonyl)-acridin-9-carbonsäureamid-hyrobromid und 0,21 g (2 mMol) Triethylamin in 25 ml wasserfreiem Tetrahydrofuran gibt man unter Rühren bei -15° C
0,11 g (1 mMol) Chlorameisensäureethylester. Man rührt 1 Std. bei gleicher Temperatur und setzt dann 0,12 g (1 mMol) N-Hydroxysuccinimid zu. Nach einer weiteren Stunde läßt man den Ansatz bei Raumtemperatur 15 Std. stehen. Man dampft i. V. ein, nimmt in Ethylacetat auf, wäscht mit Wasser, Natriumhydrogencarbonatlösung und Wasser aus und trocknet über Natriumsulfat. Nach dem Eindampfen erhält man 0,42 g (70,8 % d. Th.) des Zielproduktes.

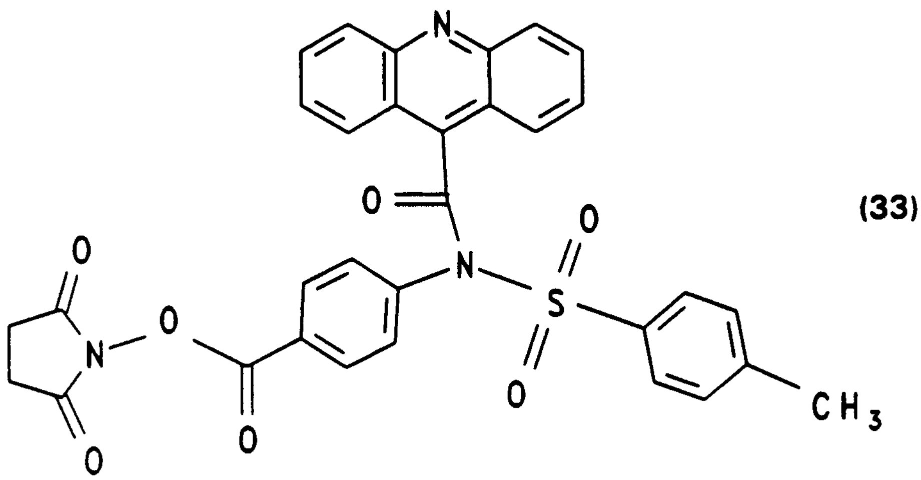

(33)

$^1$H-NMR (TFA): $\delta$ = 2,6 (s, $Ch_3$), 3,1 (s, $CH_2$-$CH_2$), 7,0 bis 8,6 (m, 16 aromat. H).

N-(4-Succinimidyloxycarbonylphenyl)-N-(4-toluolsulfonyl)-10-methylacridinium-9-carbonsäureamid-fluorsulfonat (34)

Zu der Lösung von 0,59 g (1 mMol) N-(4-succinimidyloxycarbonylphenyl)-N-(4-toluolsulfonyl)-acridin-9-carbonsäureamid in 30 ml 1,2-Dichlorethan gibt man unter Rühren bei 25° C 0,85 g (7,5 mMol] Fluorsulfonsäuremethylester. Das Reaktionsprodukt fällt innerhalb von 4 Std. aus. Nach Absaugen und Trocknen erhält man 0,43 g (60,8 % d. Th.) des Zielproduktes.

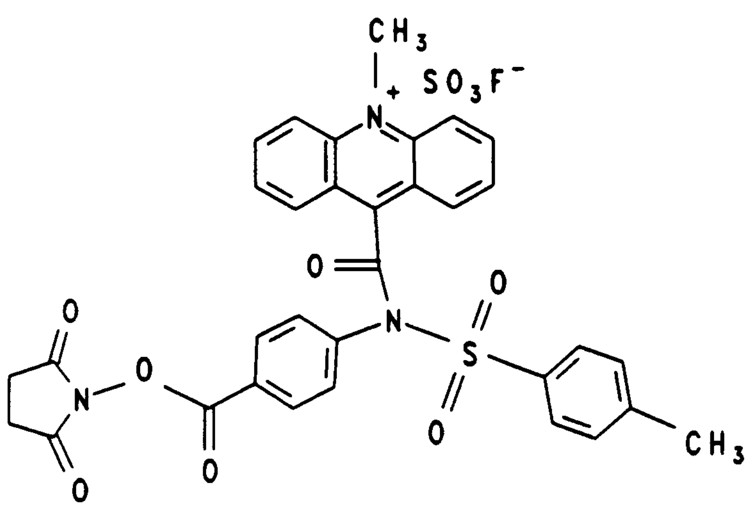

(34)

$^1$H-NMR (TFA): $\delta$ = 2,6 (s, AR-$CH_3$), 3,1 (s, $CH_2$-$CH_2$), 4,9 (s, N-$CH_3$), 7,3 bis 8,8 (m, 16 aromat. H).

Beispiel 9:

In gleicher Weise wie in Beispiel 8 erhält man aus N-(4-Carboxymethylphenyl)-4-toluol-sulfonsäureamid N-(4-Succinimidyloxycarbonylmethylphenyl)-N-(-4-toluolsulfonyl)-10-methylacridinium-9-carbonsäureamid-fluorosulfonat

N-(4-Carboxymethylphenyl)-4-toluolsufonsäureamid (35)

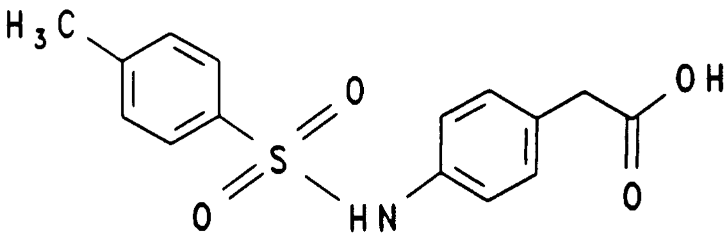

(35)

$^1$H-NMR (DMSO): δ = 2,3 (s, $CH_3$), 3,5 (s, $CH_2$), 7,0 (AB, $C_6H_4$), 7,3 bis 7,6 (AB, $C_6H_4$), 10,2 (s, NH).

N-(4-Benzyloxycarbonylmethylphenyl)-4-toluolsufonsäureamid (36)

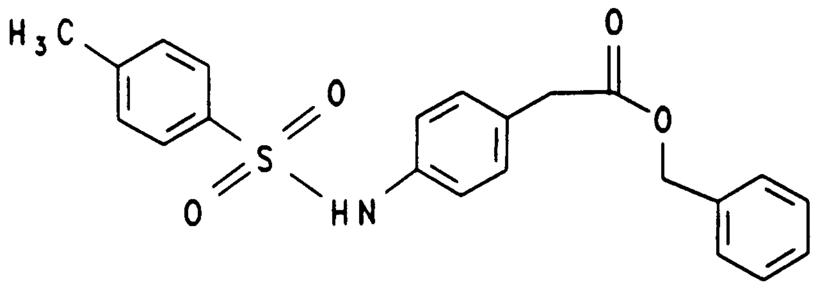

(36)

Ausbeute: 35 % d. Th.
$^1$H-NMR (DMSO): δ = 2,3 (s, $CH_3$), 3,6 (s, $COCH_2$), 5,1 (s, $OCH_2$), 6,9 bis 7,7 (m, 13 aromat. H), 10,2 (s, NH).

N-(4-Benzyloxycarbonylmethylphenyl)-N-(4-toluolsufonyl)acridin-9-carbonsäureamid (37)

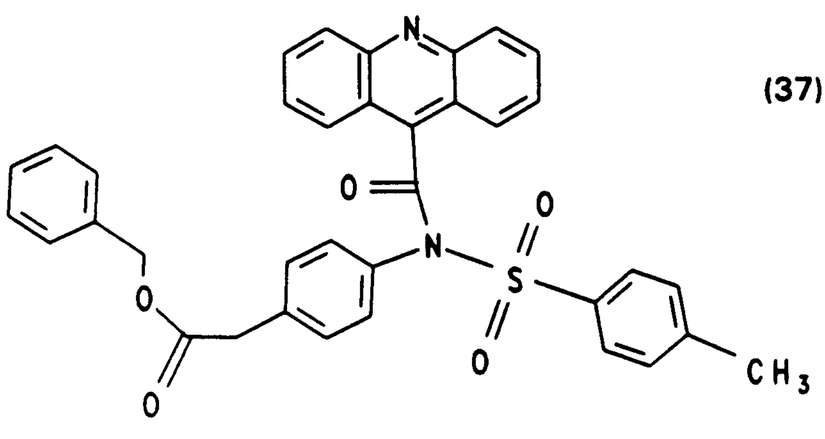

(37)

Ausbeute: 35 % d. Th.
$^1$H-NMR ($CDCl_3$): δ = 2,55 (s, $CH_3$), 3,3 (s, $COCH_2$), 5,0 (s, O-$CH_2$), 6,7 bis 8,2 (m, 21 aromat. H).

N-(4-Carboxymethylphenyl)-N-(4-toluolsulfonyl)-acridin-9-carbonsäureamid-hydrobromid (38)

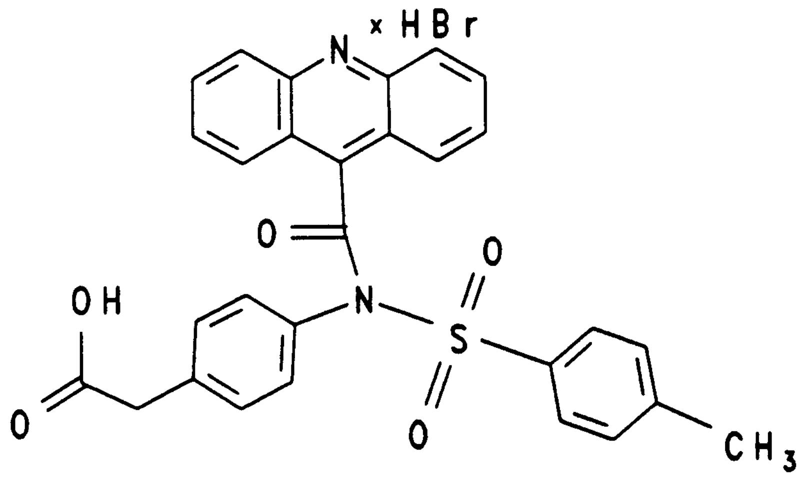

(38)

Ausbeute: 95 % d. Th.
$^1$H-NMR (TFA): $\delta$ = 2,65 (s, $CH_3$), 3,55 (s, $CH_2$), 6,8 bis 8,6 (m, 16 aromat. H).

N-(4-Succinimidyloxycarbonylmethylphenyl)-N-(4-toluolsulfonyl)-acridin-9-carbonsäureamid (39)

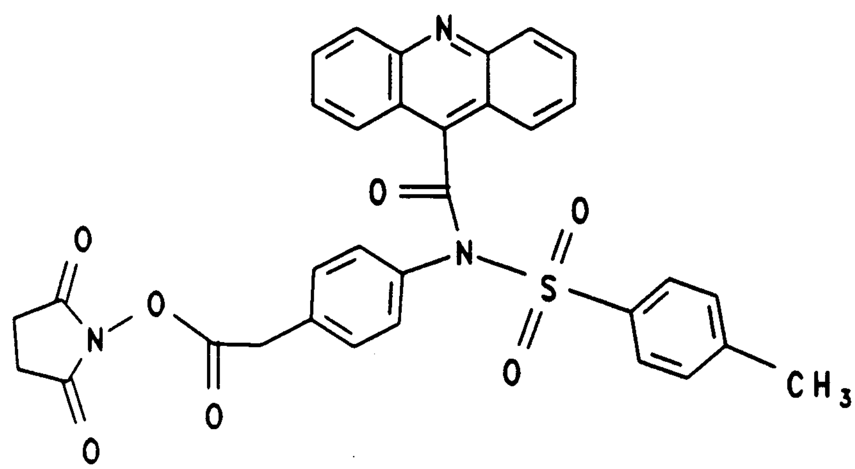

(39)

Ausbeute: 71 % d. Th.
$^1$H-NMR (TFA): $\delta$ = 2,65 (s, Toluol-$CH_3$), 3,0 (s, $CH_2$-$CH_2$), 3,6 (breit, $COCH_2$), 6,9 bis 8,5 (m, Aromat).

N-(4-Succinimidyloxycarbonylmethylphenyl)-N-(4-toluolsulfonyl)-10-methylacridinium-9-carbonsäureamidfluorsulfonat (40)

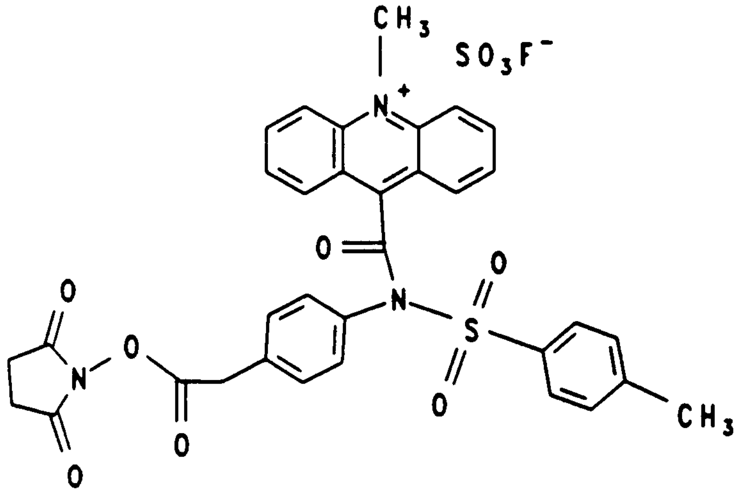

(40)

Ausbeute: 80 % d. Th.
$^{1}$H-NMR (TFA): $\delta$ = 2,6 (s, Toluol-$CH_3$), 2,9 (s, $CH_2$-$CH_2$), 3,6 (breit, $COCH_2$), 4,9 (s, N-$CH_3$), 6,8 bis 8,9 (m, Aromat).

Beispiel 10:

In gleicher Weise wie in Beispiel 8 erhält man aus N-[4-(2-Carboxyethyl)-phenyl]-4-toluolsulfonsäureamid N-[4-(2-Succinimidyloxycarbonylethyl)-phenyl]-N-(4-toluolsulfonyl)-10-methyl-acridinium-9-carbonsäureamidfluorosulfonat.

N-[4-(2-Carboxyethyl)-phenyl]-4-toluolsulfonsäureamid (41)

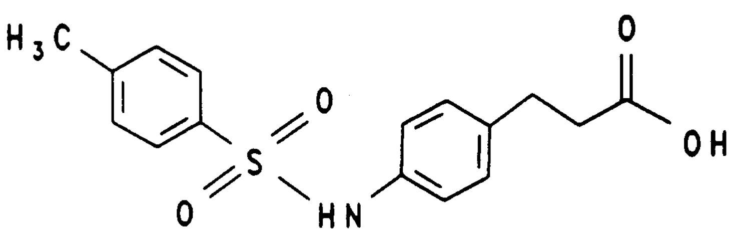

(41)

Ausbeute: 44 % d. Th.
$^{1}$H-NMR (DMSO): $\delta$ = 2,3 (s, $CH_3$), 2,4 bis 2,8 (m, $CH_2$-$CH_2$), 7,0 (AB, 4H), 7,2 bis 7,8 (AB, 4H), 10,1 (s, NH).

N-[4-(2-Benzyloxycarbonyethyl)-phenyl]-4-toluolsulfonsäureamid (42)

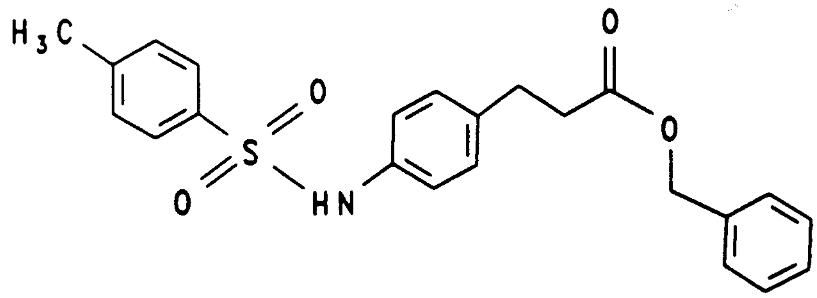

(42)

Ausbeute: 78 % d. Th.
H-NMR ($CDCl_3$): δ = 2,35 (s, $CH_3$), 2,4 bis 3,0 (m, $CH_2$-$CH_2$), 5,1 (s, O-$CH_2$), 7,0 bis 7,8 (13 aromat. H, NH).

N-[4-(2-Benzyloxycarbonylethyl)-phenyl]-N-(4-toluolsulfonyl)acridin-9-carbonsäureamid (43)

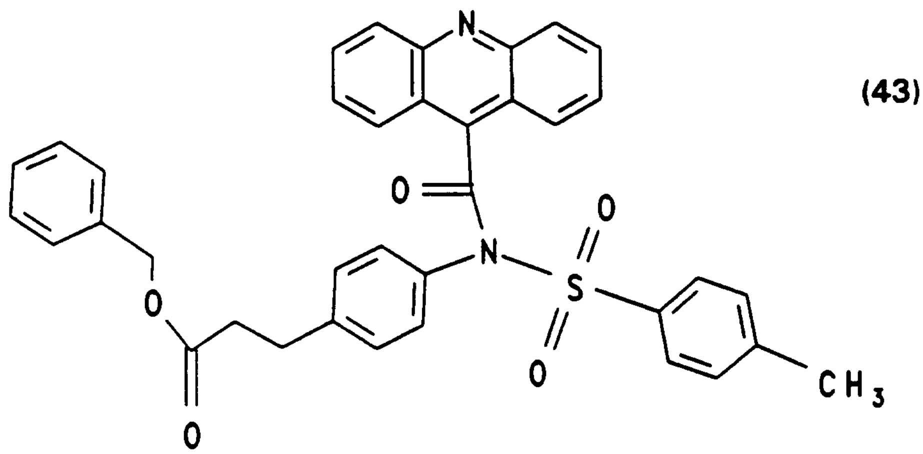

(43)

Ausbeute: 77 % d. Th.
$^1$H-NMR ($CDCl_3$): δ = 2,2 bis 2,8 (m, 7H), 5,0 (s, $CH_2$), 6,65 bis 7,0 (AB, 4 aromat. H), 7,3 bis 8,3 (m, 17 aromat. H).

N-[4-(2-Carboxyethyl)-phenyl]-N-(4-toluolsulfonyl)acridin-9-carbonsäureamid-hydrobromid (44)

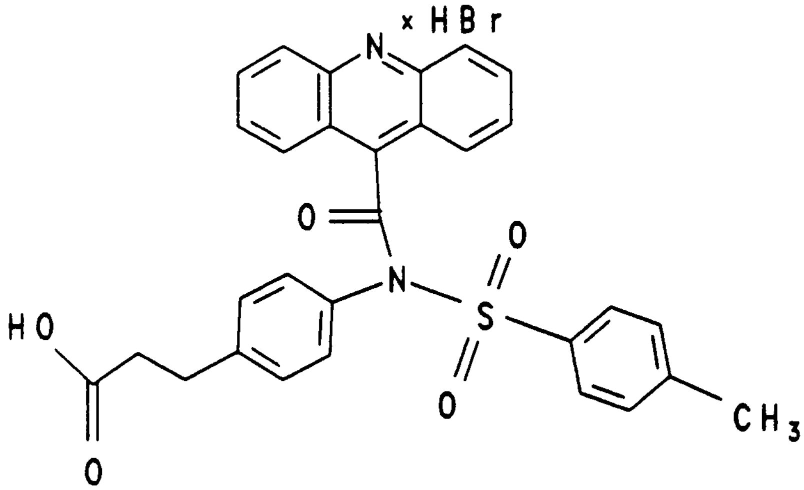

(44)

Ausbeute: 65 % d. Th.
$^1$H-NMR ($CD_3OD$): $\delta$ = 2,1 bis 2,8 (m, $CH_2$-$CH_2$), 2,5 (s, $CH_3$), 6,7 bis 8,5 (16 aromat. H).

N-[4-(2-Succinimidyloxycarbonylethyl)-phenyl]-N-(4-toluolsulfonyl)acridin-9-carbonsäureamid (45)

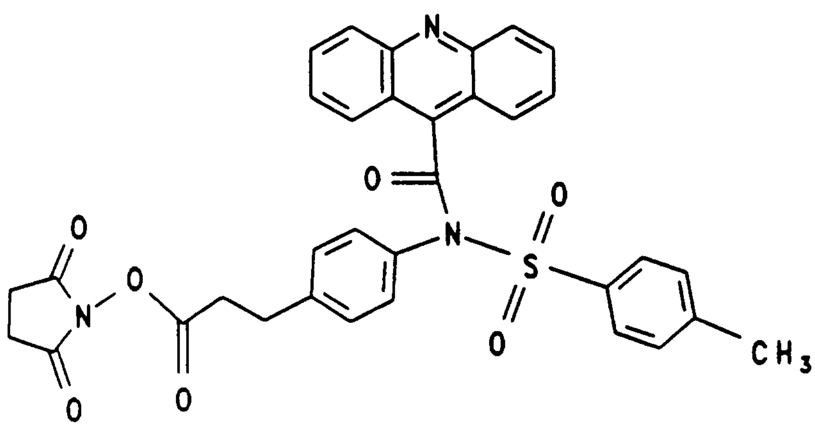

(45)

Ausbeute: 90 % d. Th.
$^1$H-NMR ($CDCl_3$): $\delta$ = 2,6 (s, $CH_3$), 2,7 (breit, $CH_2$-$CH_2$), 2,8 (s, $CH_2$-$CH_2$), 6,6 bis 8,3 (16 aromat. H).

N-[4-(2-Succinimidyloxycarbonylethyl)-phenyl]-N-(4-toluolsulfonyl)acridin-9-carbonsäureamidfluorosulfonat (46)

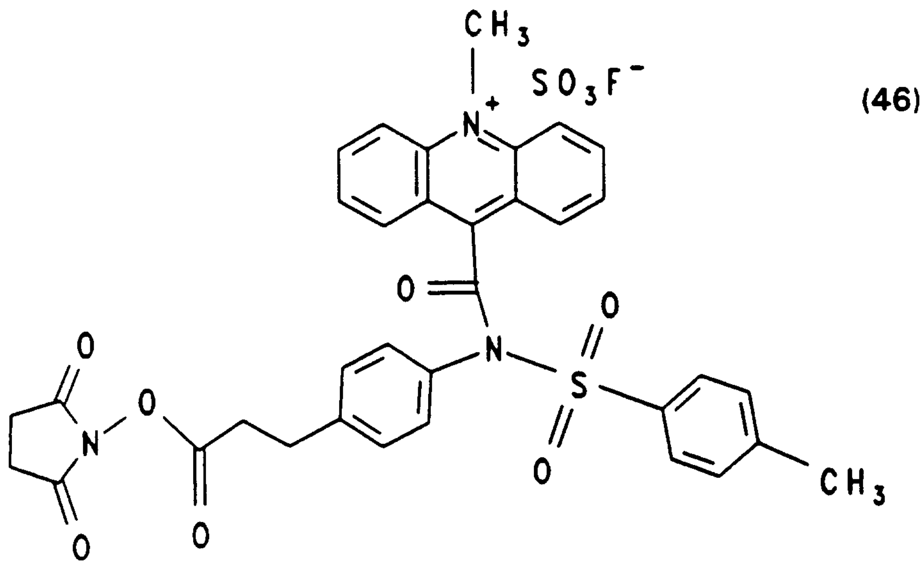

(46)

Ausbeute: 84 % d. Th.
$^1$H-NMR (TFA): $\delta$ = 2,6 (s, $CH_3$), 2,3 bis 3,3 (breiter Untergrund, 11H, s, 3,1), 4,9 (s, N-$CH_3$), 6,7 bis 8,8 (16 aromat. H).

Beispiel 11:

In analoger Weise wie in den vorangegangenen Beispielen erhält man N-[4-(N-Methylmorpholino-N-2-ethoxy)-phenyl]-N-[4-(2-succinimydyl-oxycarbonylethyl)-phenylsulfonyl]-10-methylacridin-9-carbonsäureamid-diium-di-fluorsulfonat. Abweichende Verfahrensschritte sind in den folgenden Reaktionsstufen beschrieben.

3-(4-Chlorsulfonyl-phenyl)-propionsäure-tert.butylester (47)

25 g (0,1 Mol) 3-(4-Chlorsulfonyl-phenyl)-propionsäure, 12 ml tert.Butanol, 60 ml i-Buten und 3 ml konz. Schwefelsäure werden bei -15° C vereinigt und in einem Autoklaven bei Raumtemperatur 24 Std. intensiv gerührt. Die erneut auf -15° C abgekühlte Reaktionsmischung wird in überschüssige Natriumhydrogencarbonatlösung einerührt, mit Methylenchlorid extrahiert und zuletzt unter Vakuum eingedampft.

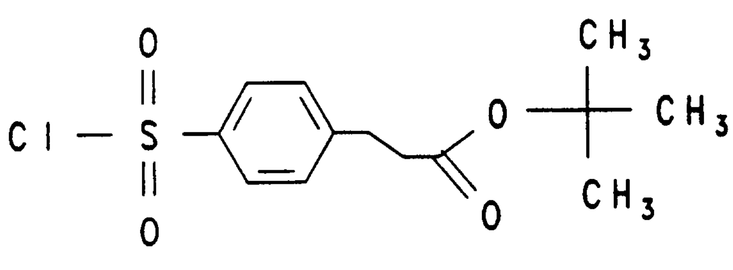

(47)

Ausbeute: 20,4 g (67 % d. Th.). Das Produkt wird aus Hexan umkristallisiert.
$^1$H-NMR ($CDCl_3$): $\delta$ = 1,4 (s), 2,6 (m), 3,0 (t), 7,4 (m), 7,95 (m).
MS: m/z = 305 ($M^+H$)
3-(4-Chlorsulfonyl-phenyl)-propionsäure wurde in bekannter Weise aus 3-Phenylpropionsäure und Chlorsulfonsäure hergestellt.

4-(Morpholino-N-2-ethoxy)anilin (48)

25 g (0,1 M) 4-(Morpholino-N-2-ethoxy)-nitrobenzol werden mit 75 g Zinngranulat in 400 ml halbkonzentrierter Salzsäure 4 Std. unter Rückfluß gekocht. Nach Abkühlen wird in 400 ml 33 %ige Salzsäure eingegossen, mit i-propylether extrahiert und die organische Phase nach Trocknen eingedampft. Man erhält 20 g (90 % d. Th.) Zielprodukt.
$^1$H-NMR ($CDCl_3$): $\delta$ = 2,5 (t), 2,7 (t), 3,5 (breit), 3,7 (t),4,0 (t), 6,6 (m). MS: m/z = 222 ($M^+$)
Das gleiche Produkt erhält man durch Hydrierung der Nitroverbindung in Methanol über Palladium-Aktivkohle.

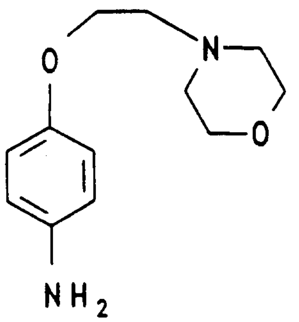

(48)

Die Nitroverbindung wurde gemäß Bull. Soc. chim. France 1955, 1353 - 62 hergestellt.

N-[4-(Morpholino-N-2-ethoxy)-phenyl]-N-[4-(2-t-butoxycarbonylethyl)-phenylsulfonamid] (49)

Die klare Lösung von 9,3 g (30 Mmol) 4-chlorsulfonyl-phenylpropionsäure-t-butylester, 6,9 g 4-(Morpholino-N-2-ethoxy)anilin und 0,3 g Dimethylaminopyridin in 150 ml Methylenchlorid wird nach 10 stündigem Stehen bei Raumtemperatur mit gesättigter Natriumhydrogencarbonatlösung ausgewaschen, auf 1/3 eingeengt und über eine Kieselgursäule mit einer Mischung von 90 % Methylenchlorid und 10 % Methanol chromatographiert. Die Hauptfraktion des Eluats wird eingedampft.

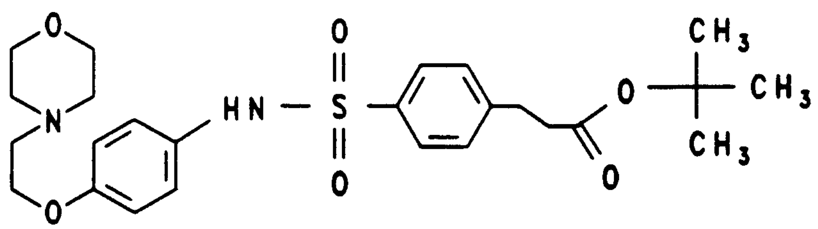

(49)

Ausbeute: 9 g (61,2 % d. Th.).
$^1$H-NMR ($CDCl_3$): $\delta$ = 1,35 (s), 2,5 (m), 2,7 bis 3,0 (m), 3,7 (m), 4,0 (t), 6,7 bis 7,0 (m), 7,2 bis 7,7 (m).
MS: m/z = 491 ($M^+H$)

N-[4-(Morpholino-N-2-ethoxy)-phenyl]-N-[4-(2-t-butoxycarbonylethyl)-phenylsulfonyl]-9-acridincarbonsäureamid (50)

Eine Lösung von 2,0 g (4,1 mMol) N-[4-(Morpholino-N-2-ethoxy)-phenyl]-N-[4-(2-t-butoxycarbonylethyl)-phenylsulfonamid] in 50 ml Methylenchlorid versetzt man unter kräftigem Rühren nacheinander mit 50 ml 33 %iger Natronlauge, 30 mg Dimethylaminopyridin, 1,2 g Tetrabutylammoniumchlorid und 1,55 g (5,6 mMol) 9-Acridincarbonsäurechloridhydrochlorid. Nach 6 Std. trennt man die organische Phase ab, wäscht mit Wasser aus, trocknet über Natriumsulfat und dampft ein.

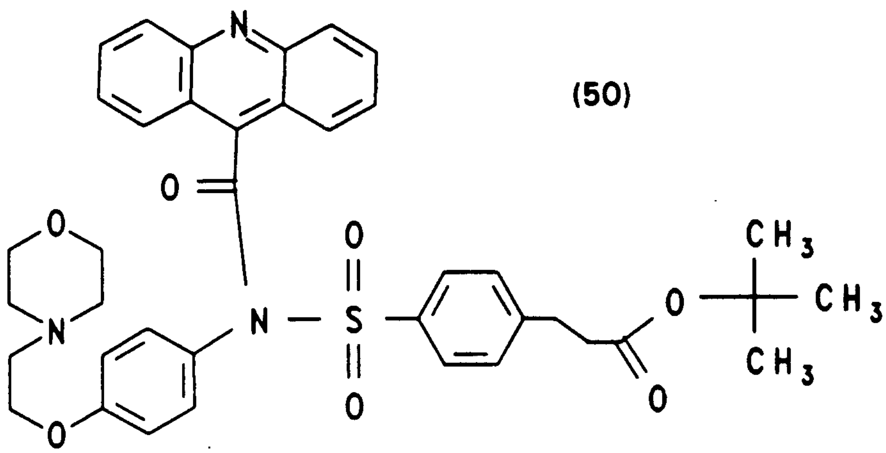

(50)

Ausbeute: 2,8 g (98 % d. Th.).
$^1$H-NMR ($CDCl_3$): $\delta$ = 1,4 (s), 2,3 bis 2,5 (m), 2,5 bis 2,8 (m), 3,0 bis 3,3 (t), 3,6 bis 3,9 (m), 6,3 (d), 6,9 (d), 7,4 bis 8,3 (m).
MS: m/z = 695 ($M^+H$)

N-[4-(Morpholino-N-2-ethoxy)-phenyl-N-[4-(2-carboxyethyl)-phenylsulfonyl]-9-acridincarbonsäureamid (51)

0,7 g (1 mMol) N-[4-(Morpholino-2-ethoxy)-phenyl]-N-[4-(2-t-butoxycarbonylethyl)-phenylsulfonyl]-9-acridincarbonsäureamid werden in 5 ml Trifluoressigsäure gelöst und über Nacht bei Raumtemperatur stehen gelassen. Man dampft an der Wasserstrahlpumpe ein, löst in Wasser und stumpft die filtrierte Lösung mit Natriumacetat auf pH 4 ab. Das angefallene Produkt extrahiert man mit Methylenchlorid, trocknet über Natriumsulfat und dampft ein.

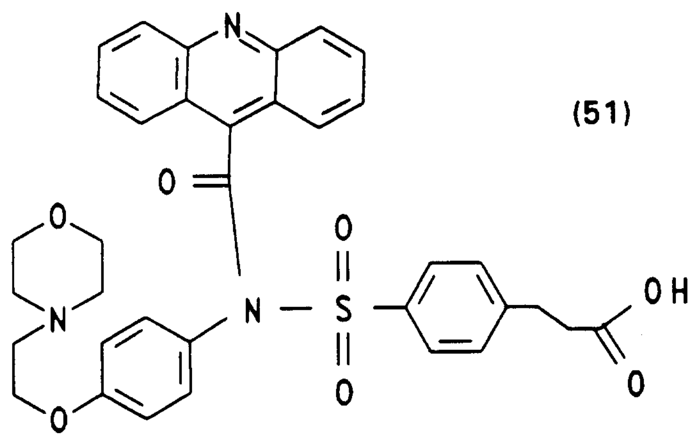

(51)

Ausbeute: 0,6 g (94 % d. Th.).
$^1$H-NMR (DMSO): $\delta$ = 2,6 bis 3,0 (m), 3,0 bis 3,3 (m), 3,6 bis 4,0 (m), 4,0 bis 4,4 (m), 5,8 bis 6,6 (m), 6,8 bis 7,0 (m), 7,3 bis 8,4 (m).
MS: m/z = 639 ($M^+$)

N-[4-(Morpholino-N-2-ethoxy)-phenyl-N-[4-(2-succinimidyloxycarbonylethyl)-phenylsulfonyl]-9-acridincarbonsäureamid (52)

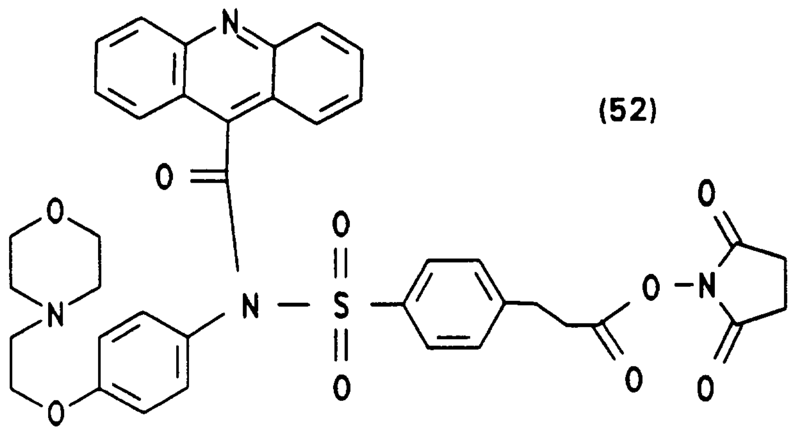

(52)

Ausbeute: 95 % d. Th.
$^1$H-NMR ($CDCl_3$): $\delta$ = 2,3 bis 2,7 (m), 2,8 (s), 2,9 bis 3,4 (m), 3,5 bis 3,9 (m), 3,9 bis 4,3 (m), 6,2 bis 7,0 (m), 7,3 bis 8,3 (m).
MS: m/z = 736 ($M^+$)

N-[4-(Methylmorpholino-N-2-ethoxy]-phenyl-N-[4-(2-succinimidyloxycarbonylethyl)-phenyl-sulfonyl]-10-methylacridin-9-carbonsäureamiddiium-di-fluorsulfonat (53)

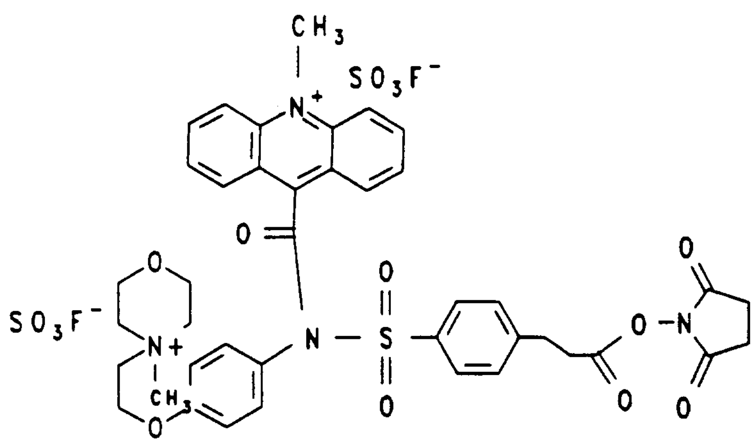

(53)

Ausbeute: 84 % d. Th. Die Verbindung ist mit gelber Farbe klar wasserlöslich.
$^1$H-NMR (DMSO): $\delta$ = 2,85 (s), 3,1 (s), 3,2 bis 4,4 (m), 4,75 (s), 6,4 bis 8,3 (m).
IR (KBr) [$cm^{-1}$]: 3120, 3080, 3060, 2960, 1815, 1785, 1740, 1695, 1610, 1555, 1510, 1380, 1270, 1215, 1140.

Beispiel 12

Die Darstellung von N-Methoxy-N-[4-(2-succinimidyloxycarbonylethyl)-benzolsulfonyl]-10-methylacridinium-9-carbonsäureamidfluorsulfonat bzw. trifluoracetat (57) ausgehend von 4-(N-Methoxysulfamido)-3-phenylpropionsäurebenzylester (53) und Acridin-9-carbonsäurechlorid-hydrochlorid erfolgt analog zur Synthese von Verbindung 16 (Beispiel 4). Die Ausbeuten der einzelnen Syntheseschritte sowie die spektroskopische Charakterisierung sind nachfolgend angegeben.

N-Methoxy-N-[4-(2-benzyloxycarbonylethyl)benzolsulfonyl]-acridin-9-carbonsäureamid (54)

Ausbeute: 72 %;
IR (KBr) [$cm^{-1}$]: 3080(w), 3040(w), 3010(w), 2960(m), 2950(m), 1730(s), 1700(s), 1595(m),1455(m), 1380(s), 1295(m), 1230(s), 1195(s), 1180(vs), 1145(m), 1095(m), 980(m), 955(m), 765(s).
$^1$H-NMR (100-MHz; D-Trichlormethan): $\delta$ [ppm] = 2,69-2,90 (m,2H); 3,05-3,29 (m,2H]; 3,61 (s,br,3H); 5,13 (s,2H); 7,26-7,43 (m,9H); 7,48 (d,br,2H), 7,65-7,85 (m,2H); 8,04 (d,br,2H); 8,15-8,30 (m,2H).

N-Methoxy-N-[4-(2-carboxyethyl)benzolsulfonyl]-acridin-9-carbonsäureamid-hydrobromid (55)

Ausbeute: quantitativ; Schmp.: 175-177 °C (Zers.)
IR (KBr) [$cm^{-1}$]: 3650-3300 (m,br), 3080 (w), 3060 (w), 3040 (w), 3200-2000 (br), 2960 (m), 2910 (m), 2880 (m), 2720 (m), 2660 (m), 2620 (m), 1700 (vs), 1640 (s), 1470 (m), 1430 (m), 1375 (s), 1250 (m), 1170 (s), 1195 (s), 950 (m), 755 (m).
$^1$H-NMR (100-MHz; $D_6$-DMSO): $\delta$ [ppm] = 2,63-2,88 (m,2H]; 2,95-3,20 (m, 2H); 3,64 (s,br,3H);4,02 (s,1H); 7,18-7,53(AA'XX'-Spektrum,4H); 7.60-8,59 (m,8H); 14,20 (s,br,1H).
Massenspektrum [EI, 70 eV; CI (Isobutan): m/z: 464 $M^+$ (Kation).

N-Methoxy-N-[4-(2-succinimidyloxycarbonylethyl]benzolsulfonyl]acridin-9-carbonsäureamid (56)

Ausbeute 55 %
$^1$H-NMR (100 MHz, $D_6$-DMSO]: $\delta$ [ppm] = 2,84 (s,4H]; 2,90 bis 3,38 (m,4H); 3,73 (s,br,3H); 7,28 bis 7,88 (m,br,8H); 7.96 bis 8,31 (m,br,4H).

N-Methoxy-N-[4-(2-succinimidyloxycarbonylethyl]benzolsulfonyl]-10-methylacridinium-9-carbonsäureamidfluorsulfonat bzw. -trifluoracetat (57)

Ausbeute: 75 %
$^1$H-NMR (100 MHz, $D_6$-DMSO]: $\delta$ [ppm] = 2,75 bis 2,90 (m,4H); 3,24 (s,br,4H); 3,65 (s,br,3H); 4,93 (s,br,3H); 7,38 bis 8,19 (m,br,8H); 8,38 bis 8,63 (m,br,2H); 8,93 (d,br,2H).
IR(KBr) [$cm^{-1}$]: 3090 (w), 2960 (w), 2930 (w), 2870 (w), 1815 (m), 1790 (s), 1745(vs), 1695 (s), 1615 (m), 1600 (w), 1555 (m), 1465 (m), 1430 (m), 1380 (s), 1210 (vs), 1190 (vs), 1095 (s), 770 (s), 725 (s), 670 (s).
Massenspektrum (FAB; Matrix: 3-Nitrobenzylalkohol, ohne und mit KCl-Zusatz): m/z: 576 $M^+$ (Kation).

Beispiel 13

Analog zu Beispiel 11 erhält man N-[4-(3,6,9-Trioxa-1-undecycloxy(phenyl]-N-[4-(2-succinimidyloxycarbonylethyl)-benzolsulfonyl]-10-methylacridinium-9-carbonsäureamidfluorsulfonat bzw. - trifluoracetat (63). Die Ausbeute der einzelnen Syntheseschritte sowie die spektroskopische Charakterisierung sind nachfolgend angegeben. 4-(3,6,9-Trioxa-1-undecyloxy)anilin (58) erhält man durch Reduktion der Nitroverbindung, wie für Verbindung (47) in Beispiel 11 beschrieben. Die Nitroverbindung wurde gemäß US 2,485,712 (1949) [CA 44 (1950) 2556i] hergestellt.

4-(3,6,9-Trioxa-1-undecycloxy)anilin (58)

$^1$H-NMR (100 MHz, $CDCl_3$) $\delta$ [ppm] = 1,20 (t,3H), 2,97 (s,br,2H), 3,51 (q,2H), 3,54-4,13 (m,14H), 6.50-6,84 (m,4H).

N-[4-(3,6,9-Trioxa-1-undecyloxy)phenyl]-N-[4-(2-t-butoxycarbonylethyl)-phenylsulfonamid] (59)

$^1$H-NMR (100 MHz, $CDCl_3$) $\delta$ [ppm] = 1,20 (t,3H), 1,39 (s,9H), 2,41-2,63 (m,2H), 2,80-3,05 (m,2H), 3,53 (q,2H), 3,60-3,75 (m,8H), 3,77-3,91 (m,2H), 3,98-4,13 (m,2H), 6,75; 6,98 (AA'XX'-Spektrum,4H), 7,24, 7,61 (AA'XX'-Spektrum,4H).
IR (KBr) [$cm^{-1}$]: 3280 (m,br), 3070 (w), 2980 (s), 2930 (s), 2880 (s), 1725 (s), 1600 (m), 1510 (s), 1555 (m), 1370 (s), 1340 (m), 1300 (m), 1250 (m), 1170 (s), 1115 (m), 1095 (m).

N-[4-(3,6,9-Trioxa-1-undecyloxy)phenyl]-N-[4-(2-t-butoxycarbonylethyl]-phenylsulfonyl]-9-acridincarbonsäureamid (60)

Ausbeute 70 %;
$^1$H-NMR (100 MHz, $CDCl_3$) $\delta$ [ppm] = 1,17 (t,3H), 1,45 (s,9H), 2,55-2,78 (m,2H), 3,01-3,25 (m,2H), 3.50 (q,2H), 3,54-3,88 (m,12H), 6,33, 6,89 (AA'XX'-Spektrum,4H), 7,41-7,83 (m,8H), 8,02-8,18 (m,4H).
IR (KBr) [$cm^{-1}$]: 3080 (w), 3060 (w), 2980 (m), 2920 (m), 2880 (m), 2860 (m), 1740 (s),1690 (s), 1600 (m), 1510 (m), 1360 (s), 1250 (s), 1175 (s), 1160 (s), 1150 (s), 1060 (m), 950 (m).
Massenspektrum (FAB; Matrix: 3-Nitrobenzylalkohol, ohne und mit KCl-Zusatz): m/z: 742 $M^+$ (Kation).

N-[4-(3,6,9-Trioxa-1-undecyloxy)phenyl]-N-[4-(2-carboxyethyl]phenylsulfonyl]-9-acridincarbonsäureamid (61)

$^1$H-NMR (100 MHz, $CDCl_3$) $\delta$ [ppm] = 1,18 (t,3H), 2,70-2,95 (m,2H), 3,05-3,28 (m,2H), 3,50 (q, überlagert, 2H), 3,52-3,90 (m,12H), 6,35, 6,90 (AA'XX'-Spektrum,4H), 7,36-7,85 (m,8H), 8,02-8,28 (d,br,4H).
IR (KBr) [$cm^{-1}$]: 3450 (br), 3300-2400 (br), 3080 (m), 3060 (m), 2980 (s), 2920 (s), 2880 (s),1735 (vs), 1690 (vs), 1600 (s), 1505 (vs), 1450 (m), 1355 (vs), 1250 (vs), 1210 (s), 1170 (vs), 1140 (s), 1125 (s), 1105 (s)-,1050 (s), 950 (s), 930 (m), 860 (m).

N-[4-(3,6,9-Trioxa-1-undecyloxy)phenyl]-N-[4-(2-succinimidyloxycarbonylethyl)-phenylsulfonyl]-9-acridincarbonsäureamid (62)

Ausbeute: 45 %;
$^1$H-NMR (100 MHz, $CDCl_3$) $\delta$ [ppm] = 1,18 (t,3H); 2,83 (s,br,4H); 3,04-3,30 (m,4H); 3,49 (q,2H); 3,53-3,89 (m,12H); 6,33; 6,86 (AA'XX'-Spektrum,4H); 7.50-7,85 (m,8H); 8,00-8,25 (m,4H).
IR (KBr) [$cm^{-1}$]: 3070 (w), 3060 (w), 2980 (m), 2930 (m), 2860 (m), 1815 (m), 1785 (m), 1740 (vs), 1690 (vs), 1600 (m), 1505 (vs), 1450 (m), 1360 (vs), 1290 (s), 1250 (vs), 1205 (vs), 1170 (vs), 1140 (s), 1110 (s), 1080 (s), 945 (m), 855 (m), 770 (m), 760 (m), 660 (m), 650 (m).
Massenspektrum (FAB; Matrix: 3-Nitrobenzylalkohol, ohne und mit KCl-Zusatz): m/z: 783 $M^+$ (Kation).

N-[4-(3,6,9-Trioxa-1-undecyloxy)phenyl]-N-[4-(2-succinimidyloxycarbonylethyl)-phenylsulfonyl]-10-methylacridinium-9-carbonsäureamid-fluorsulfonat bzw. - trifluoracetat (63)

Ausbeute: 41 %;
Die Reinigung durch präp- MPLC erfolgt wie in den Beispielen 1-3 beschrieben.
IR (KBr) [$cm^{-1}$]: 3120 (w), 3070 (w), 2980 (m), 2960 (m), 2930 (m), 2900 (m), 2880 (m), 1810 (m), 1785 (m), 1740 (s), 1690 (s), 1610 (m), 1505 (m), 1380 (s), 1255 (s), 1210 (s), 1175 (s), 1130 (s), 1090 (s), 935 (m), 770 (m), 670 (m).
Massenspektrum (FAB; Matrix: 3-Nitrobenzylalkohol, ohne und mit KCl-Zusatz): m/z: 798 $M^+$ (Kation).

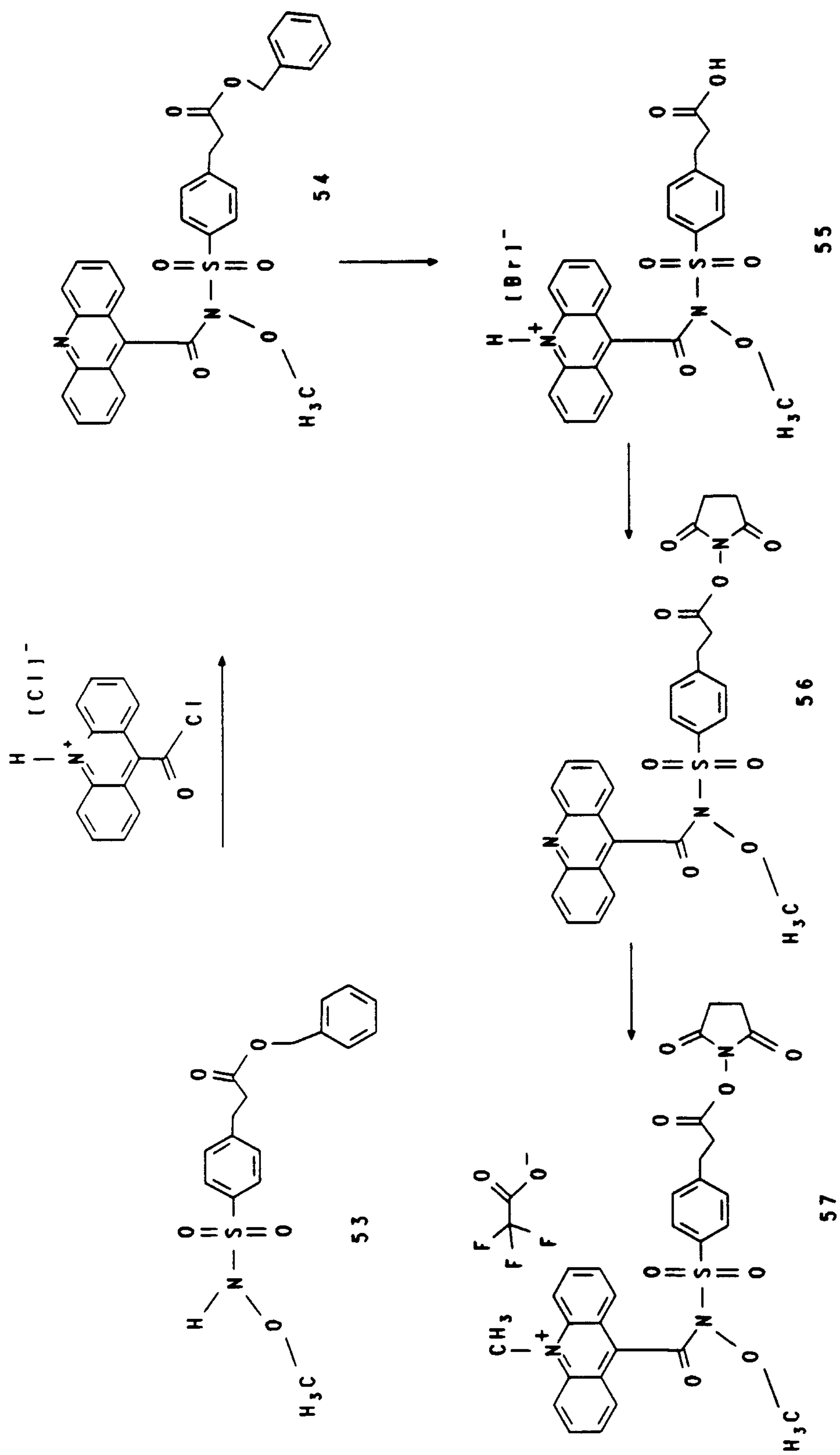

54

55

56

53

57

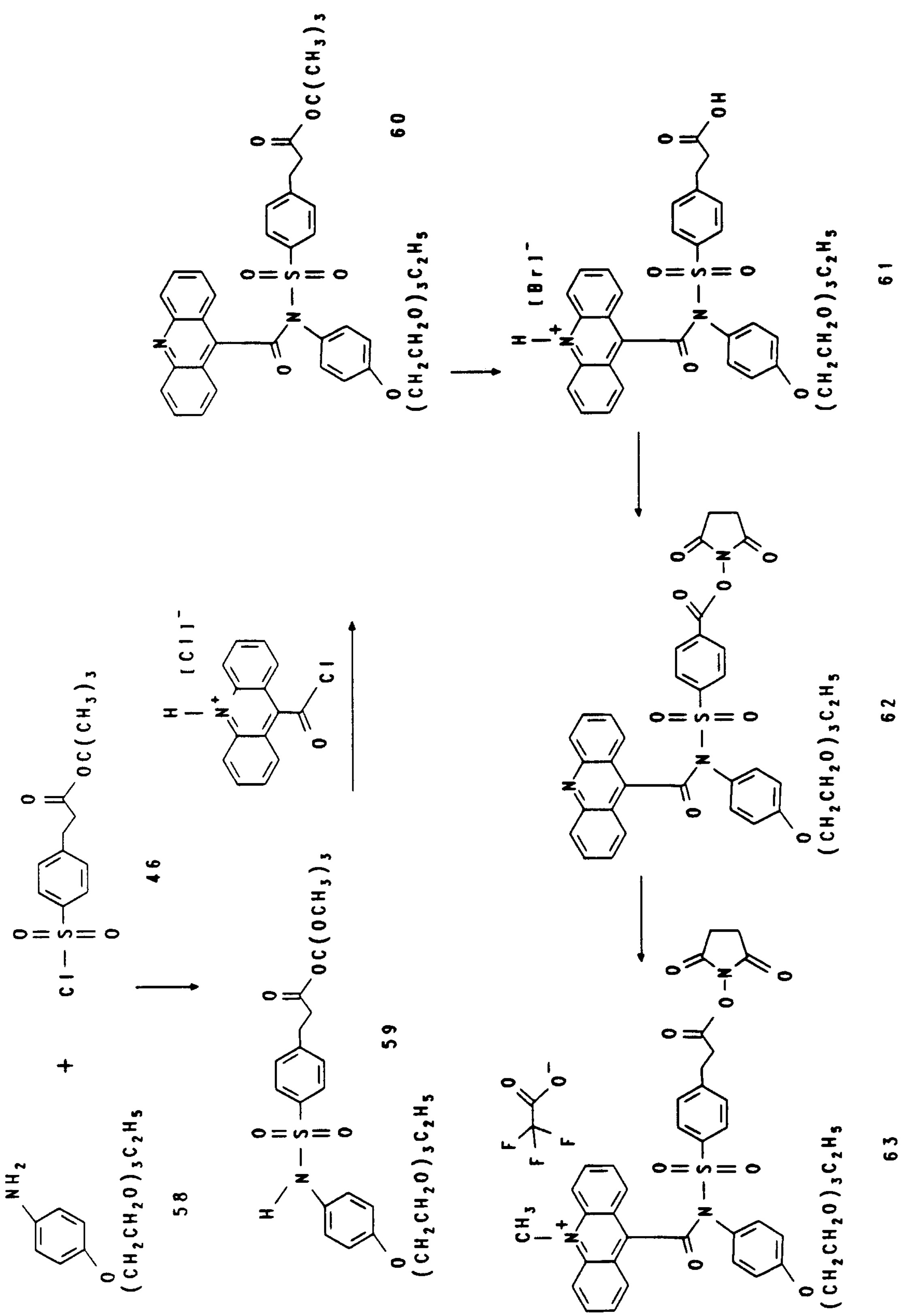

58

46

59

60

61

62

63

Beispiel 14: Herstellung von Oligonucleotiden der Formeln (64) bis (69)

a) Herstellung eines Oligonucleotids der Formel (64)

5'd($H_2N$-$(CH_2)_6$-O-pGGCCGTTACCCCACCTACTAGCTAAT)3' (64)

Der Träger, welcher 0,2 µmol 5'-O-Dimethoxytrityl-thymidin als 3'-O-Succinat gebunden hält, wird nacheinander mit folgenden Reagenzien behandelt:

1. Acetonitril abs.
2. 3 % Trichloressigsäure in Dichlormethan
3. Acetonitril abs.
4. 4 µmol 5'-O-Dimethoxytritylnucleosid-3'-phosphorigsäure-β-cyanoethylester-diisopropylamidit der Formel (73) (im 1. Reaktionszyklus ist B' = $N^6$-Benzoyladen-1-yl) und 25 µmol Tetrazol in 0,15 ml Acetonitril abs.
5. Acetonitril
6. 20 % Acetanhydrid in THF mit 40 % Lutidin und 10 % Dimethylaminopyridin.
7. Acetonitril
8. Jod (1,3 g in THF/Wasser/Pyridin; 70 : 20 : 5 = v : v : v)

Im Kondensationsschritt Nr. 4 werden das Adenin als $N^6$-Benzoyl-, das Cytosin als $N^4$-Benzoyl-, das Guanin als $N^2$-Isobutyryl-Nucleosidderivat und das Thymin ungeschützt entsprechend obiger Sequenz eingesetzt.

Die Schritte 1 bis 8, zur Vereinfachung ein Reaktionszyklus genannt, werden zum Aufbau der obigen Sequenz 24 mal wiederholt. Im folgenden 26. Reaktionszyklus wird im Schritt 4 anstelle des 5'-O-Dimethoxytrityl-2'-desoxynucleosid-3'-phosphoramidits das 5'-Aminomodifier-phosphoramidit der Formel (70) zur Kondensation eingesetzt. Nach abgeschlossener Synthese erfolgt die Abspaltung der Dimethoxytritylgruppe wie in den Schritten 1 bis 3 beschrieben. Durch 1,5-stündige Behandlung mit Ammoniak wird das Oligonucleotid vom Träger gespalten und zugleich werden die β-Cyanoethyl-Gruppen eliminiert. Da das Oligonucleotid noch Amino-Schutzgruppen enthält, ist eine weitere Ammoniak-Behandlung von 24 Stunden bei ca. 25° C notwendig. Das erhaltene Rohprodukt an 5'-Aminoalkylphosphat-Oligonucleotid wird durch Polyacrylamid-Gelelektrophorese, FPLC oder HPLC gereinigt. Die Charakterisierung erfolgt durch Elektrospray Negativ-Ionen Massenspektrometrie.

b) Herstellung des Oligonucleotids der Formel (65)

5'd(GGCCGTTACCCCACCTACTAGCTAATp-O-$CH_2$-CH(OH)-$CH_2$-$NH_2$)3' (65)

Die Synthese des 3'-aminopropandiol-modifizierten Oligonucleotids erfolgt wie in Beispiel a) beschrieben mit der Ausnahme, daß zum Start der Synthese der 3'-Amino-Modifier-Träger der Formel (71) eingesetzt wird, die erste Kondensation mit 5'-O-Dimethoxytritylthymidin-3-'phosphoramidit erfolgt und 26 Reaktionszyklen entsprechend obiger Sequenz erfolgen. Die Kondensation mit dem 5'-Amino-Modifier der Formel (70) entfällt.

c) Herstellung des Oligonucleotids der Formel (66)

5'd(GGCCGTTACCCCp-O-$CH_2$-CH($CH_2NH_2$)-O-pCCTACTAGCTAAT)3' (66)

Bei dieser Sequenz wurde das 14. Nucleotid vom 3'Ende durch eine Aminopropandiol-Einheit ersetzt. Die Synthese erfolgt analog Beispiel a) mit der Ausnahme, daß im 14. Kondensationsschritt anstelle des $N^6$-Adenosin-3'-phosphoramidits der Branched-Modifier der Formel (72) eingesetzt wird und die 5'-Derivatisierung entfällt.

d) Herstellung des Oligonucleotids der Formel (67)

5'd(GGCCGTTACCCCp{-NH-$(CH_2)_6$-$NH_2$}ACCTACTAGCTAAT)3' (67)

Bei dieser Sequenz wurde zwischen dem 14. und 15. Nucleotid vom 3'-Ende die Internucleotid-Phosphodiester-Gruppe durch eine Aminoalkyl-phosphoramidat-Gruppe ersetzt. Die Synthese erfolgt analog Beispiel a) mit der Ausnahme, daß im 14. Kondensationsschritt ein $N^6$-Adenosin-3'-methoxy-phosphoramidit der Formel (73) eingesetzt wird und die Oxidation des Schrittes Nr. 8 nicht mit Jodwasser, sondern mit 0,1M Jod in N-Trifluoracetyl-hexamethylendiamin/THF (1: 2, v : v) ausgeführt wird. Die 5'-Derivatisierung entfällt.

e) Herstellung des Oligonucleotids der Formel (68)

5'd(GGCCGTTACCCCACCTACTAGCTAATp{-NH-$(CH_2)_6$-$NH_2$})3' (68)

Bei dieser Sequenz wurde das 3'-Ende des Oligonucleotids als Aminoalkylphosphoramidat derivatisiert. Die Synthese erfolgt analog Beispiel d) mit der Ausnahme, daß zum Start der Synthese der Träger der Formel (74) eingesetzt wird, die erste Kondensation mit 5'-O-Dimethoxytritylthymidin-3'-methoxyphosphoramidit (Formel (73), B = Thymin) erfolgt und die anschließende Oxidation (Schritt Nr. 8) nicht mit Jodwasser, sondern mit 0,1M Jod in N-Trifluoracetyl-hexamethylendiamin/THF (1:2 = v : v) ausgeführt wird. Die 5'-Derivatisierung entfällt. Am Ende der Synthese wird der Bishydroxyethylsulfid-Linker in an sich bekannter Weise mit $NaWO_4/H_2O_2$ zum basenlabilen Bishydroxyethylsulfonyl-Linker oxidiert.

f) Herstellung des Oligonucleotids der Formel (69)

5'd(GGCCGTTACCCCACCTACTAGCTAAT-$NH_2$)3' (69)

Die Synthese des 3'-Amino-oligonucleotids erfolgt wie in Beispiel b) beschrieben mit der Ausnahme, daß zum Start der Synthese der 3'-Desoxy-3'-aminothymidin-Träger der Formel (75) eingesetzt wird. Am Ende der Synthese erfolgt eine zusätzliche Säurebehandlung (12 Stunden, 80 %ige Essigsäure, 25° C) zur Spaltung der Phosphoramidat-Bindung.

Beispiel 15: Herstellung markierter der Oligonucleotide der Formel (XI) mit dem Acridinium-Derivat (16)

Das Oligonucleotid der Formel (65) (5- $OD_{260}$ = 150 µg) wird in 500 µl Markierungspuffer gelöst, mit 500 µl einer 1 %igen Lösung von (16) in Acetonitril gemischt und die Mischung zwei Tage bei Raumtemperatur gerührt. Der Markierungspuffer wurde durch Lösen von 0,89 g Kaliumdihydrogenphosphat, 16,64 g Dinatriumhydrogenphosphat und 8,77 g Natriumchlorid in 900 ml Wasser, Zugabe von Natriumhydroxid bis pH 8,0 und Verdünnen mit Wasser auf 1 l Volumen hergestellt. Zur Vervollständigung der Markierungsreaktion gibt man nochmals 500 µl (16) in zwei Portionen zu und rührt jeweils noch einen Tag bei 25° C weiter. Nach vollständiger Umsetzung wird die Reaktionsmischung lyophilisiert und der Rückstand über eine PD-10 Säule (Firma Pharmacia, Freiburg) entsalzt. Zur weiteren Reinigung wurde das erhaltene Produkt durch Polyacrylamid-Gelelektrophorese (17 % Polyacrylamid, 7 M Harnstoff, 3200 Voltstunden) gereinigt und über PD-10 entsalzt.

Beispiel 16

Homogener Gensondenassay zum Nachweis auf E. coli

50 µl Standard bzw. Kontrolle werden in Polystyrolröhrchen pipettiert. Danach werden 50 µl einer im Beispiel 15 dargestellten 3'-terminal markierten Gensonde (2,5 x $10^6$ RLU, 1 M Tris-Puffer, pH 7) zugefügt und für 15 min bei 60° C hybridisiert. Anschließend wird 300 µl eines Selektionsreagenzes (0,2 M Tetraborat, pH 8) zugegeben und für 2 x 3 Sekunden geschüttelt. Daraufhin läßt man die Röhrchen 5 min abkühlen. Die Messung erfolgt durch Zugabe von jeweils 300 µl Analyzer Reagenz 1 (0,1 M $HNO_3$, 0,1 % $H_2O_2$) und Analyzer Reagenz 2 (1 M NaOH, 1 M Harnstoff, 1 % Cetyltrimethylammoniumbromid) im Autoclinilumaten der Fa. Berthold. Die Meßzeit beträgt 1 sec/Probe.

Beispiel 17

Heterogener Assay zum Nachweis auf E. coli

50 µl Standard bzw. Kontrolle werden in Polystyrolröhrchen pipettiert. Danach werden 50 µl einer analog Beispiel 15 aus Oligonukleotid (66) dargestellten intern markierten Gensonde (2,5 x $10^6$ RLU, 1 M Tris-Puffer, pH 7) zugefügt und für 15 min bei 60° C hybridisiert. Anschließend wird 1 ml eines Trennreagenzes (Magnetpartikel-Aktivator bestehend aus 0,4 M Phosphatpuffer, 0,5 % Triton x-100, pH 6 und Magnetpartikel der Fa. Advanced Magnetics bestehend aus 4,6 % Feststoff und 82 % Magnetitgehalt mit einer Endkonzentration von 0,01 mg/ml zugefügt, 2 x geschüttelt und danach für 10 min bei 60° C

inkubiert. Es folgt für 5 min eine Separation der Magnetpartikel. Der Überstand wird dekantiert und die Röhrchen werden mit 5 ml Waschpuffer (NaOH, pH 11, Triton X-100) gefüllt. Der nachfolgende Separationsschritt dauert 20 min.
Danach wird wiederum dekantiert. Mit dem verbleibenden Waschpufferrest werden die Magnetpartikel aufgewirbelt.
Die Lichtauslösung und Messung erfolgt wie im Beispiel 16 beschrieben.

Tabelle 1:

| Bestimmung der Gensondenstabilität | | | | | |
|---|---|---|---|---|---|
| Gensonde (Acridiniumester) aus Chlamydia trachomatis Kit Fa. Gen Probe | | | | | |
| Temperatur: 37° C | | | Temperatur: 50° C | | |
| Meßtag | RLU[1] | Aktivität[2] | Meßtag | RLU[1] | Aktivität[2] |
| 0 | 1208710 | 100,00 | 0 | 1208710 | 100,00 |
| 1 | 1142141 | 94,49 | 1 | 1038752 | 85,94 |
| 3 | 994406 | 82,27 | 3 | 636693 | 52,68 |
| 6 | 854844 | 70,72 | 6 | 374743 | 31,00 |
| 7 | 928994 | 76,86 | 7 | 405610 | 33,56 |
| 8 | 903655 | 74,76 | 8 | 339018 | 28,05 |
| 9 | 896953 | 74,21 | 9 | 299335 | 24,76 |
| 10 | 853065 | 70,58 | 10 | 273958 | 22,67 |
| | | | | | |
| Gensonde mit eigenem Label (Markierung am 3'-Ende) | | | | | |
| Temperatur: 37° C | | | Temperatur: 50° C | | |
| Meßtag | RLU[1] | Aktivität[2] | Meßtag | RLU[1] | Aktivität[2] |
| 0 | 1165773 | 100,00 | 0 | 1165773 | 100,00 |
| 1 | 1242315 | 106,57 | 1 | 1234001 | 105,85 |
| 3 | 1374165 | 117,88 | 3 | 1352509 | 116,02 |
| 6 | 1429686 | 122,64 | 6 | 1382378 | 118,58 |
| 7 | 1235129 | 105,95 | 7 | 1217517 | 104,44 |
| 8 | 1233814 | 105,84 | 8 | 1202993 | 103,19 |
| 9 | 1229303 | 105,45 | 9 | 1195099 | 102,52 |
| 10 | 1227636 | 105,31 | 10 | 1191566 | 102,21 |

| Gensonde mit eigenem Label (internukleotidische Markierung) | | | | | |
|---|---|---|---|---|---|
| Temperatur: 37° C | | | Temperatur: 50° C | | |
| Meßtag | RLU[1] | Aktivität[2] | Meßtag | RLU[1] | Aktivität[2] |
| 0 | 1426089 | 100,00 | 0 | 1426089 | 100,00 |
| 1 | 1468217 | 102,95 | 1 | 1469095 | 103,02 |
| 3 | 1567396 | 109,91 | 3 | 1575094 | 110,45 |
| 6 | 1567467 | 109,91 | 6 | 1592136 | 111,64 |
| 7 | 1451830 | 101,81 | 7 | 1439256 | 100,92 |
| 8 | 1437295 | 100,79 | 8 | 1414663 | 99,20 |
| 9 | 1445500 | 101,36 | 9 | 1397278 | 97,98 |
| 10 | 1445081 | 101,33 | 10 | 1369992 | 96,07 |

[1]) RLU = Relative Light Units
[2]) Aktivitätsangabe in %, bezogen auf Meßtag 0

Tabelle 2:

| Vergleich Label-/Gensondenstabilität | | | | | |
|---|---|---|---|---|---|
| Eigenes Label | | | Gensonde mit eigenem Label | | |
| Temperatur: 37° C | | | Temperatur: 37° C | | |
| Meßtag | Licht-signal* | Signal-aktivität° | Meßtag | Licht-signal* | Signal-aktivität° |
| 0 | 1111179 | 100,00 | 0 | 1165773 | 100,00 |
| 1 | 1140331 | 102,62 | 1 | 1242315 | 106,57 |
| 2 | 1053373 | 94,80 | | | |
| 3 | 1042208 | 93,79 | 3 | 1374165 | 117,88 |
| 6 | 920935 | 82,88 | 6 | 1429686 | 122,64 |
| | | | 7 | 1235192 | 105,95 |
| 8 | 1042684 | 93,84 | 8 | 1233814 | 105,84 |
| 9 | 986248 | 88,76 | 9 | 1229303 | 105,45 |
| | | | 10 | 1227636 | 105,31 |
| 12 | 937889 | 84,40 | | | |
| 13 | 986589 | 88,79 | | | |
| | | | | | |
| Temperatur: 50° C | | | Temperatur: 50° C | | |
| Meßtag | Licht-signal* | Signal-aktivität° | Meßtag | Licht-signal* | Signal-aktivität° |
| 0 | 1111179 | 100,00 | 0 | 1165773 | 100,00 |
| 1 | 939137 | 84,52 | 1 | 1234001 | 105,85 |
| 2 | 944192 | 84,97 | | | |
| 3 | 925376 | 83,28 | 3 | 1352509 | 116,02 |
| 6 | 878369 | 79,05 | 6 | 1382378 | 118,58 |
| | | | 7 | 1217517 | 104,44 |
| 8 | 934485 | 84,10 | 8 | 1202993 | 103,19 |
| 9 | 950994 | 85,58 | 9 | 1195099 | 102,52 |
| | | | 10 | 1191566 | 102,21 |
| 12 | 904204 | 81,37 | | | |
| 13 | 926408 | 83,37 | | | |

*) Relative Light Units °) Signalaktivität in %, bezogen auf Meßtag 0

Tabelle 3:

| Vergleich Label-/Gensondenstabilität | | | | | |
|---|---|---|---|---|---|
| Eigenes Label | | | Gensonde mit eigenem Label | | |
| Temperatur: 70° C | | | Temperatur: 70° C | | |
| Meßzeit in min | Licht-signal* | Signal-aktivität° | Meßzeit in min | Licht-signal* | Signal-aktivität° |
| 0 | 1111179 | 100,00 | 0 | 1150000 | 100,00 |
| 15 | 1217798 | 109,60 | 15 | | |
| 30 | 1180330 | 106,22 | 30 | 1165240 | 101,33 |
| 60 | 1208075 | 108,72 | 60 | 1143899 | 99,47 |
| 120 | 1166918 | 105,02 | 120 | 1176993 | 102,35 |
| 240 | 1125228 | 101,26 | 240 | 1313937 | 114,26 |
| 480 | 987609 | 88,88 | 480 | 1259207 | 109,50 |
| | | | | | |
| Temperatur: 95° C | | | Temperatur: 95° C | | |
| Meßzeit in min | Licht-signal* | Signal-aktivität° | Meßzeit in min | Licht-signal* | Signal-aktivität* |
| 0 | 1111179 | 100,00 | 0 | 1150000 | 100,00 |
| 15 | 1181645 | 106,34 | 15 | 1131188 | 98,36 |
| 30 | 1068157 | 96,13 | 30 | 1141822 | 99,29 |
| 60 | 741911 | 66,77 | 60 | 1322910 | 115,04 |
| 120 | 583928 | 52,55 | 120 | | |
| 240 | 560462 | 50,44 | 240 | 1153200 | 100,28 |
| 480 | 618776 | 55,69 | 480 | | |

*) Relative Light Units

°) Signalaktivität in %, bezogen auf Meßtag 0

**Patentansprüche**

1. Gensonde gemäß Formel (XI)

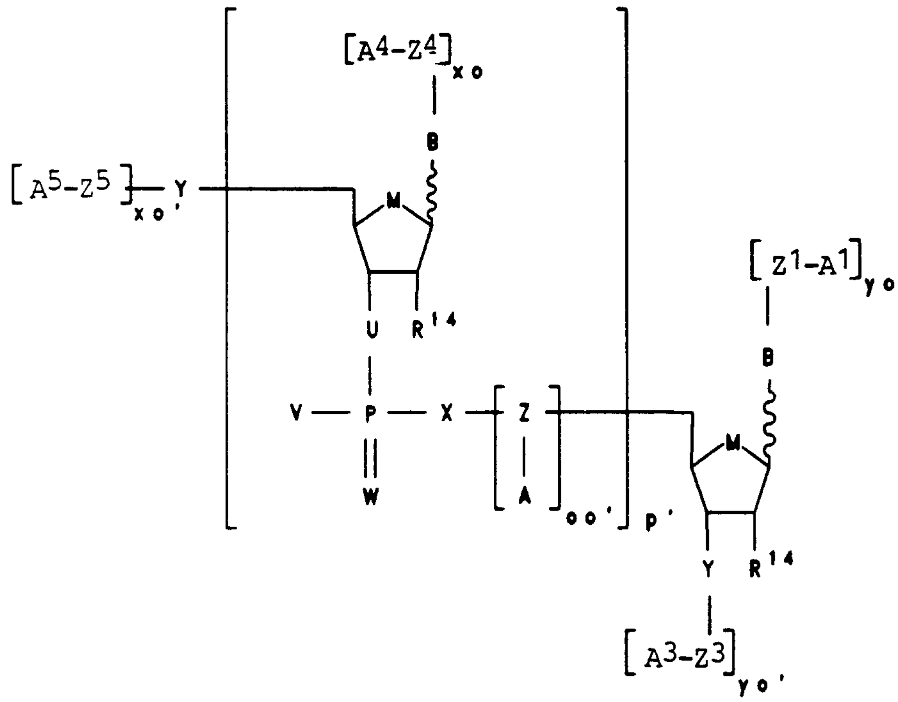

(XI)

worin

| | |
|---|---|
| $R^{14}$ | Wasserstoff, Hydroxy, Amino, $NHR^{15}$, Azid, Halogen oder eine geschützte Hydroxyl- oder Methoxygruppe in $\alpha$- oder $\beta$-Stellung bedeutet, wobei |
| $R^{15}$ | für $C_1$- bis $C_{18}$-Alkyl, vorzugsweise $C_1$- bis $C_8$-Alkyl, $C_6$- bis $C_{20}$-Aryl, ($C_6$- bis $C_{14}$)-Aryl-($C_1$- bis $C_8$)-alkyl, $-(CH_2)_c-[NH(CH_2)_c]_d-NR^{16}R^{16'}$ steht, worin |
| c | eine ganze Zahl von 2 bis 6 und |
| d | eine ganze Zahl von 0 bis 6 ist und |
| $R^{16}$, $R^{16'}$ | unabhängig voneinander Wasserstoff, $C_1$- bis $C_6$-Alkyl oder ($C_1$- bis $C_4$)-Alkoxy-($C_1$- bis $C_6$)-alkyl, bevorzugt Methoxyethyl bedeutet und Halogen für Fluor, Chlor, Brom oder Iod steht, oder einen Rest $-[Z^6-A^6]_{xo''}$ bedeutet, |
| $A^1$, $A^2$, $A^3$, $A^4$, $A^5$, $A^6$ | Acridiniumderivate, welche an $Z^1$, $Z^2$, $Z^3$, $Z^4$, $Z^5$, $Z^6$ der Formel (XI) gebunden sind, |
| B | für eine in der Nukleotidchemie übliche Base, außer Uracil, steht, |
| M | Oxy, Methylen, Fluormethylen oder Difluormethylen ist, |
| U | Oxy, Sulfandiyl, Imino oder Methylen bedeutet, |
| V | für Hydroxy, Mercapto, SeH, $C_1$- bis $C_{18}$-Alkoxy, vorzugsweise $C_1$- bis $C_6$-Alkoxy, $C_1$- bis $C_{18}$-Alkyl, vorzugsweise $C_1$-bis $C_6$-Alkyl, $C_6$- bis $C_{20}$-Aryl, ($C_6$- bis $C_{14}$)-Aryl-($C_1$- bis $C_8$)-alkyl, $NHR^{15}$, $NR^{16}R^{17}$ oder einen Rest der Formel $(OCH_2CH_2)_m,O(CH_2)_n,CH_2R^{18}$ bedeutet, worin $R^{15}$ und $R^{16}$ die oben angegebenen Bedeutungen haben, |
| $R^{17}$ | $C_1$- bis $C_{18}$-Alkyl, vorzugsweise $C_1$-bis $C_8$-Alkyl und besonders $C_1$-bis $C_4$-Alkyl, $C_6$- bis $C_{20}$-Aryl oder ($C_6$-bis $C_{10}$)-Aryl-($C_1$- bis $C_8$)-alkyl bedeutet oder im Falle von $NR^{16}R^{17}$ zusammen mit $R^{16}$ und dem sie tragenden Stickstoffatom einen 5-bis 6-gliedrigen heterozyklischen Ring bedeutet, der zusätzlich ein weiteres Heteroatom aus der Reihe O, S, N enthalten kann, |
| $R^{18}$ | Wasserstoff oder eine funktionelle Gruppe wie Hydroxy, Amino, $NHR^{16}$, COOH, $CONH_2$, $COOR^{19}$ oder Halogen bedeutet, worin |
| $R^{19}$ | $C_1$- bis $C_4$-Alkyl, vorzugsweise Methyl, bedeutet und |
| $R^{16}$ | und Halogen die oben angegebenen Bedeutungen besitzen, |
| m' | eine ganze Zahl von 1 bis 100, bevorzugt 3 bis 20 und besonders bevorzugt 3 bis 8 ist, |
| n' | eine ganze Zahl von 0 bis 18, beorzugt 0 bis 15, ist, |
| W | Oxo, Thioxo oder Selenoxo ist, |

| | |
|---|---|
| X | Oxy, Thio, NH oder Methylen bedeutet, |
| Y | für Oxy, Thio, NH, Methylen oder eine Schutzgruppe steht, |
| $Z^1$, $Z^2$, $Z^3$, $Z^4$, $Z^5$ | unabhängig voneinander $C_1$- bis $C_{30}$-Alkyl, $C_6$- bis $C_{30}$-Aryl, ($C_6$- bis $C_{12}$)-Aryl-($C_1$- bis $C_{20}$)-alkyl, $C_1$- bis $C_{30}$-Alkoxy bedeutet, welche Substituenten gegebenenfalls einfach oder mehrfach mit $C_1$- bis $C_8$-Alkyl, $C_6$- bis $C_{20}$-Aryl, ($C_6$- bis $C_{14}$)-Aryl-($C_1$- bis $C_8$)-alkyl, $C_1$ - bis $C_8$-Alkoxy, Imino, Hydroxy, $NHR^{15}$, Thio, Phosphat, Pyrophosphat oder Triphosphat substituiert sind, wobei $R^{15}$ die oben angegebene Bedeutung hat, |
| oo' | eine ganze Zahl von 0 bis 10 ist, |
| xo, yo, xo', yo', xo'' | unabhängig voneinander 0 oder 1 bedeuten, mit der Maßgabe, daß mindestens eine der Variablen oo', xo, yo, xo', yo' oder xo'' ungleich Null ist, |
| p' | für eine ganze Zahl von 1 bis 100 steht |

und

die geschweifte Klammer für p' in 2'-oder 3' -Stellung bedeutet.

**2.** Gensonde nach Anspruch 1, worin

| | |
|---|---|
| B | für eine in der Nukleotidchemie üblichen Base, einschließlich Uracil, steht und |
| xo, yo | jeweils Null bedeutet. |

**3.** Gensonde nach den Ansprüchen 1 und 2, worin

| | |
|---|---|
| B | für eine Base in $\beta$-Stellung und |
| M | für 0 und |
| $R^{14}$ | für H, OH und |
| Y | für Oxy oder NH steht und |
| M, U, V, W | für 0 stehen und |
| $Z^1$, $Z^2$, $Z^3$, $Z^4$, $Z^5$ | unabhängig voneinander $C_1$- bis $C_{30}$-Alkyl, $C_6$- bis $C_{30}$-Aryl, ($C_6$- bis $C_{12}$)-Aryl-($C_1$- bis $C_{20}$)-alkyl oder $C_1$- bis $C_{30}$-Alkoxy bedeuten, gegebenenfalls mit Imino und/oder Hydroxy substituiert und |
| p' | eine ganze Zahl von 5 bis 50 und |
| oo' | eine ganze Zahl von 0 bis 3 bedeutet und |
| | zwei der Variablen xo', yo', xo'', oo' ungleich Null sind. |

**4.** Gensonde nach einem oder mehreren der Ansprüche 1 bis 3, worin

| | |
|---|---|
| p' | für eine ganze Zahl von 10 bis 30 steht und |
| oo' | Null oder 1 bedeutet und |
| | eine Variable xo', yo', xo'', oo' 1 sein kann und die jeweils übrigen Variablen Null bedeuten. |

**5.** Gensonde nach einem oder mehreren der Ansprüche 1 bis 4, worin

| | |
|---|---|
| oo' | für 1 steht und |
| xo, yo, xo', yo', xo'' | Null bedeuten. |

**6.** Gensonde nach einem oder mehreren der Ansprüche 1 bis 5, worin

| | |
|---|---|
| xo' | für 1 steht und |
| xo, yo, yo', xo'', oo' | Null bedeuten. |

**7.** Gensonde nach einem oder mehreren der Ansprüche 1 bis 6, worin

| | |
|---|---|
| yo' | für 1 steht<br>und |
| xo, yo, xo', xo", oo' | Null bedeuten. |

**8.** Gensonde nach einem oder mehreren der Ansprüche 1 bis 7, enthaltend ein oder mehrere Acridiniumderivate $A^1$ bis $A^6$ der Formel I

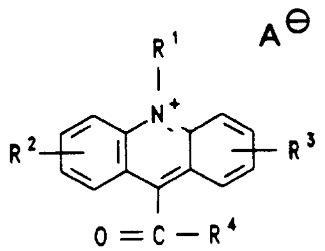

(I)

in der $R^4$ eine gegebenenfalls über einen Spacer erfolgte Bindung zu einem Oligonucleotid oder einen Rest der Formel (II) oder (III) darstellt

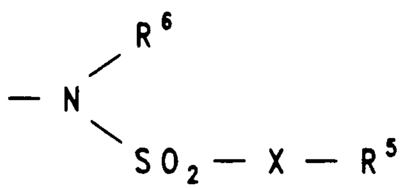

(II)

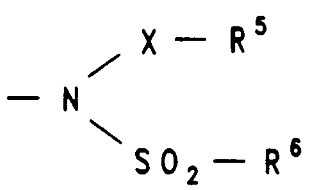

(III)

worin $R^5$ eine reaktive Gruppe ist, die zu einer Bindung mit einem Oligonukleotid wie insbesondere Oligo- und Polynukleotidsequenzen (Targets) in DNA und RNA übergeführt ist,
$R^6$ Wasserstoff, ein Alkyl-, Alkenyl- oder Alkoxyrest mit 1 bis 10 C-Atomen, eine substituierte Aminogruppe, eine Benzylgruppe, eine Arylgruppe, eine Heteroalkylgruppe oder ein Heterocyclus ist, der/die auch mit Hydroxy-, Amino-, Alkylamino-, Alkyl-, Alkenyl- oder Alkoxy mit 1 bis 10 C-Atomen, Polyalkoxy-oder Aryloxy-gruppen oder einer heterocyclischen Gruppe substituiert sein können, wobei die zuletzt genannten Substituenten ihrerseits mit einer heterocyclischen Verbindung oder einem Amin substituiert sein können oder zusammen einen O- und/oder S- und/oder NH- oder N-Alkyl-aufweisenden Heterocyclus bilden können, und
X eine Arylgruppe, die direkt oder über eine Alkyl- oder Oxyalkylgruppe an das Stickstoff- oder Schwefelatom gebunden ist und über eine Alkyl- oder Oxyalkylgruppe an den Rest $R^5$ gebunden ist und die auch mit Alkyl-, Alkenyl-, Hydroxy-, Amino-, Alkoxy-, Polyalkoxy- oder Aryloxy-Gruppen und/oder Heteroatomen einfach oder mehrfach substituiert sein kann oder den Rest einer aliphatischen, araliphatischen oder aromatischen, Aminocarbonsäure, bedeutet oder eine Phenylgruppe ist, wenn $R^6$ eine Phenylgruppe ist, die einfach der mehrfach mit $C_1$-$C_6$-Alkyl substituiert ist,
und
$R^1$ Wasserstoff, ein Alkyl-, Alkenyl- oder Alkinylrest mit 1 bis 10 Kohlenstoffatomen, eine Benzyl- oder Arylgruppe ist,
$R^2$ und $R^3$ Wasserstoff, eine Alkylgruppe mit 1 bis 10 Kohlenstoffatomen, eine substituierte oder unsubstituierte Aminogruppe, eine Carboxy-, Alkoxy-, Cyano-, Nitrogruppe oder Halogen ist.

**9.** Gensonde nach Anspruch 8, dadurch gekennzeichnet, daß X eine Gruppe der Formel (IV) ist,

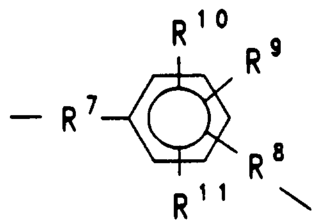

(IV)

in der $R^7$ ein Substituent der Formel $-(CH_2)n-$ oder $((CH_2)_m-O)n-$ ist mit n = 0 bis 4 und m = 1 bis 6, $R^8$ ein Substituent in ortho-, meta- oder para-Stellung zu $R^7$ der Formel $-(CH_2)_p-$, eine Polyalkylenoxid-Gruppe der Formel $-(O-(CH_2)_m-)_p$ oder - $((CH_2)_m-O-)_p$ bevorzugt mit p = 1 bis 6, oder ein verzweigter oder unverzweigter Kohlenwasserstoffrest mit 1 bis 4 C-Atomen ist und die Substituenten $R^9$ bis $R^{11}$ Wasserstoff oder geradkettige oder verzweigte Kohlenwasserstoffreste mit bis zu 30 C-Atomen sind, wobei eine oder mehrere - $CH_2$-Einheiten auch durch O, S, SO, $SO_2$, NH oder N-Alkyl ersetzt sein können und zwei dieser Substituenten ringförmig verknüpft sein können.

**10.** Verfahren zur Herstellung einer Gensonde gemäß einem oder mehreren der Ansprüche 1 bis 9, dadurch gekennzeichnet, daß eine Verbindung gemäß Formel (XI), in der mindestens eine der Gruppen A1 bis A6 Wasserstoff bedeutet umgesetzt wird mit einer Verbindung gemäß Formel (I), in der $R^5$ eine reaktive Gruppe ist, die mit einer funktionellen Gruppe der Verbindung der Formel (XI) eine chemische Bindung ausbildet.

**11.** Gensondenassay zur Bestimmung eines Oligo- bzw. Polynukleotid-Targets (RNA bzw. DNA) in einer Flüssigkeitsprobe, dadurch gekennzeichnet, daß mit einer Gensonde nach einem oder mehreren der Ansprüche 1 bis 9 ein homogenes Assayprinzip durchgeführt wird.

**12.** Gensondenassay nach Anspruch 11, dadurch gekennzeichnet, daß mit einer Gensonde nach einem oder mehreren der Ansprüche 1 bis 9 ein heterogenes Assayprinzip durchgeführt wird.

**13.** Gensondenassay nach den Ansprüchen 11 oder 12, dadurch gekennzeichnet, daß das Target durch Hybridisierung nach einer Targetamplikation bestimmt wird.

**14.** Gensondenassay nach einem oder mehreren der Ansprüche 11 bis 13, dadurch gekennzeichnet, daß zusätzlich ein oder mehrere Signalamplikationssysteme eingesetzt werden.

Fig. 1

Untere Nachweisgrenze des Acridiniumderivats in der erfindungsgemäßen Gensonde

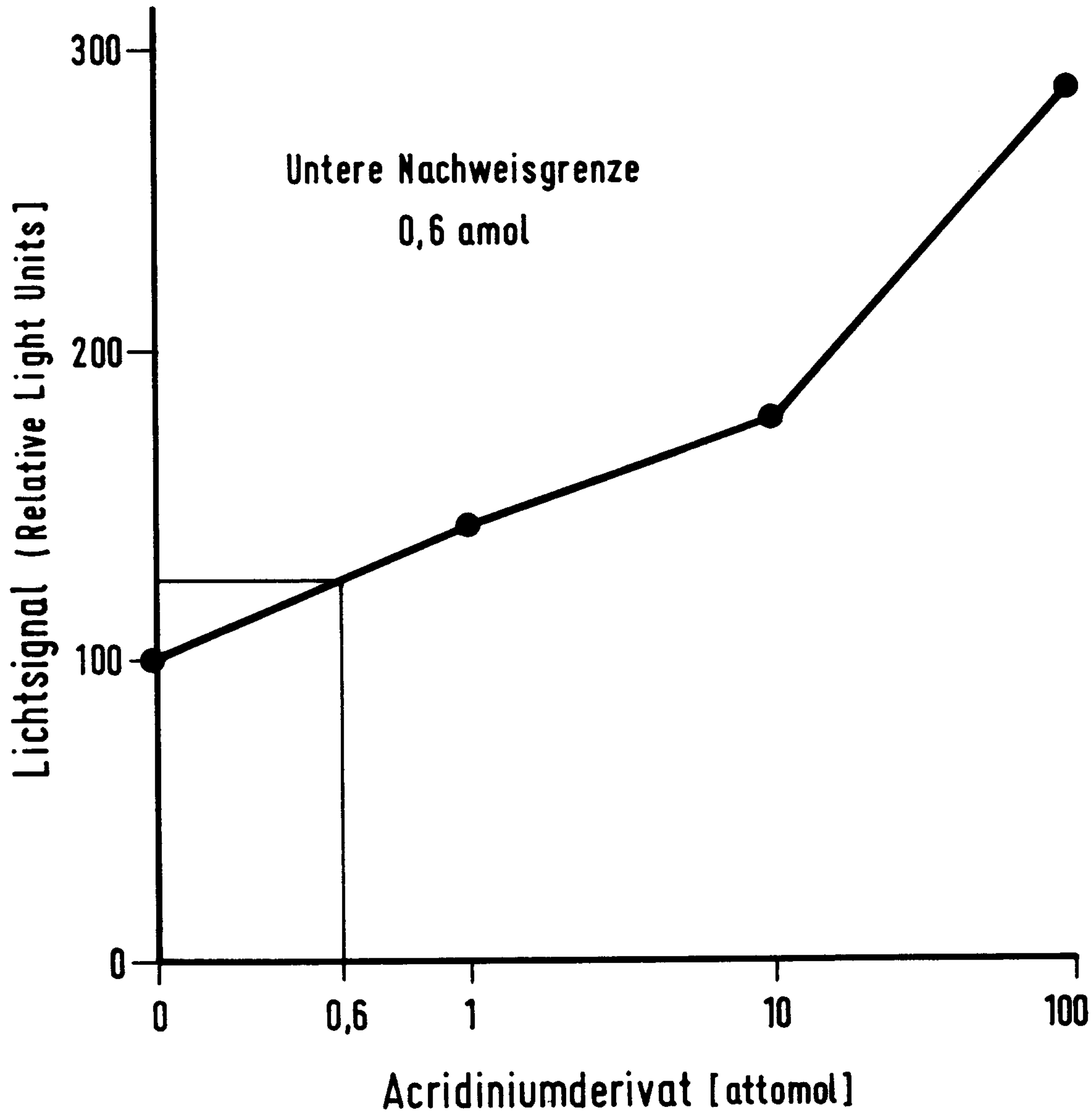

Fig. 2

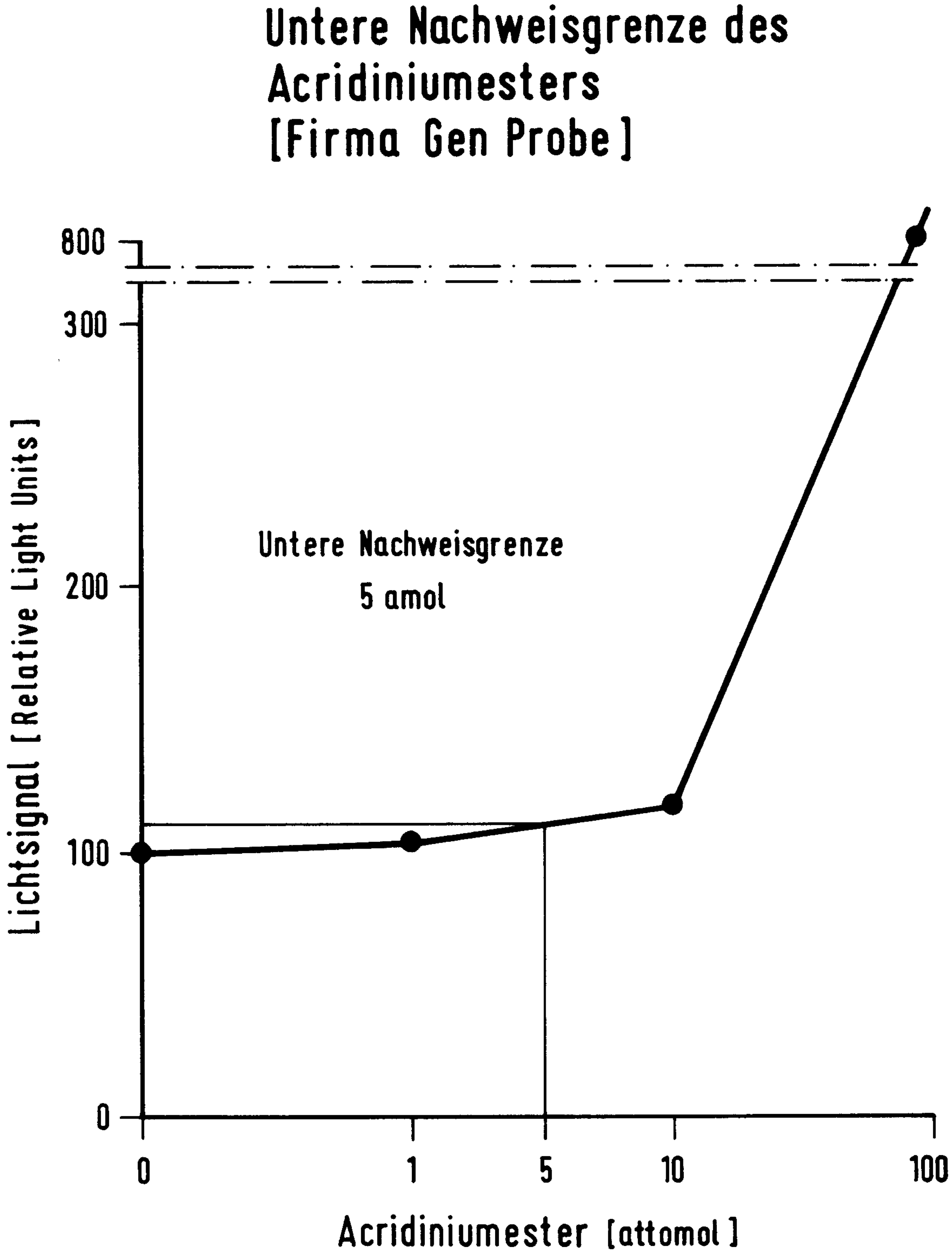
Untere Nachweisgrenze des
Acridiniumesters
[Firma Gen Probe]
800
300
200
100
0
Lichtsignal [Relative Light Units]
Untere Nachweisgrenze
5 amol
0
1
5
10
100
Acridiniumester [attomol]

Fig. 3a:

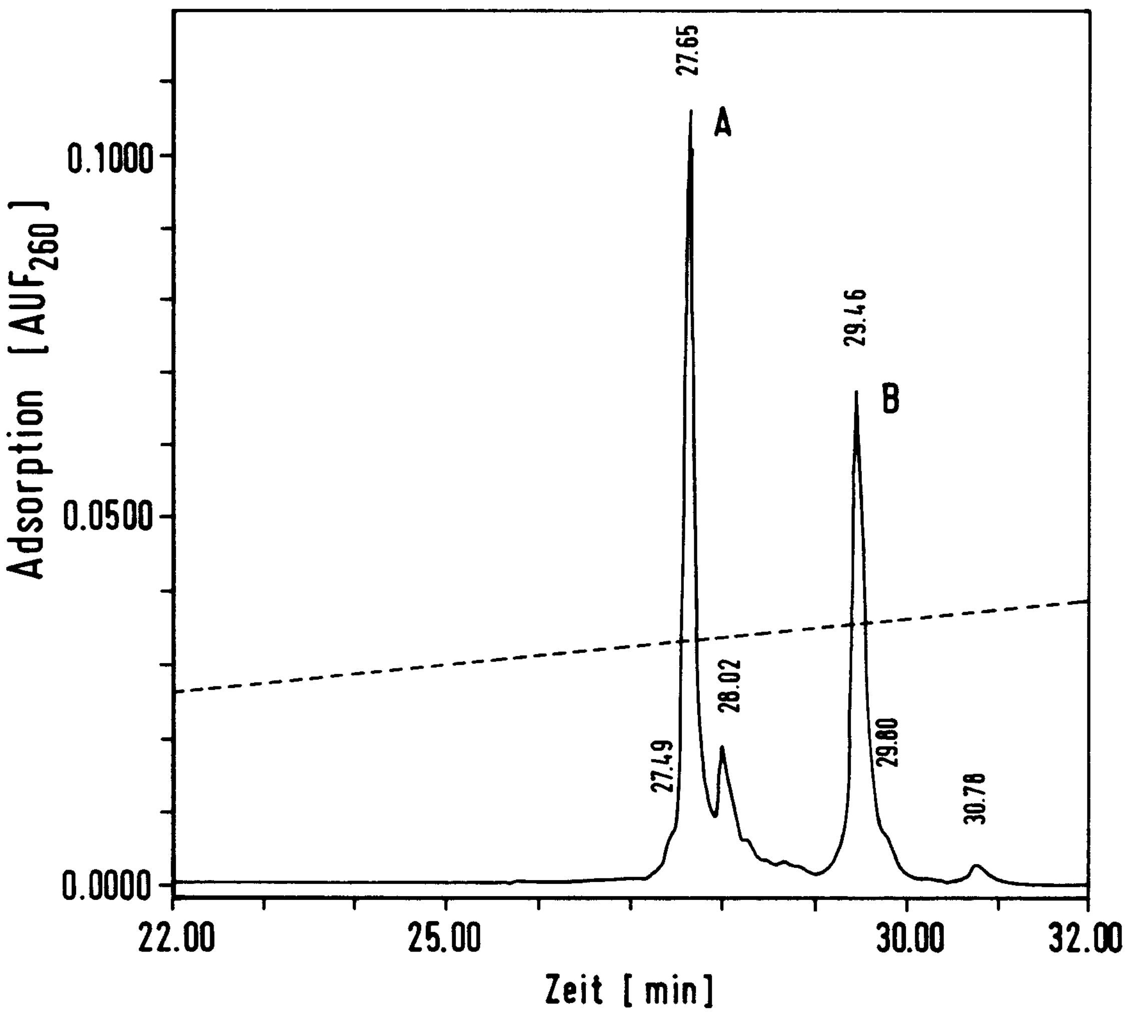
27.65
A
29.46
B
28.02
27.49
29.80
30.78
0.1000
0.0500
0.0000
Adsorption [$AUF_{260}$]
22.00
25.00
30.00
32.00
Zeit [min]

Fig. 3b:

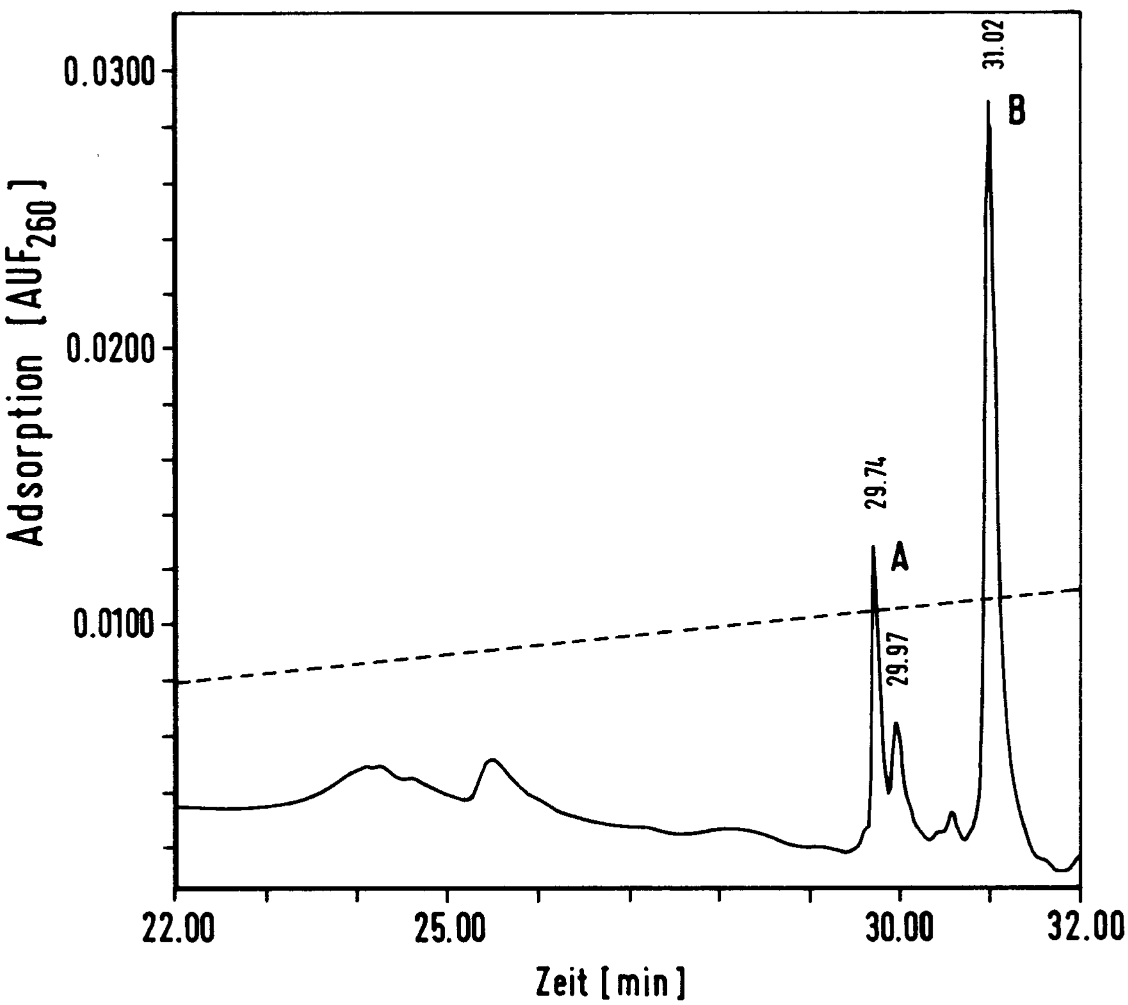
0.0300
0.0200
0.0100
Adsorption [AUF260]
31.02
B
29.74
A
29.97
22.00
25.00
30.00
32.00
Zeit [min]

Fig. 3c:

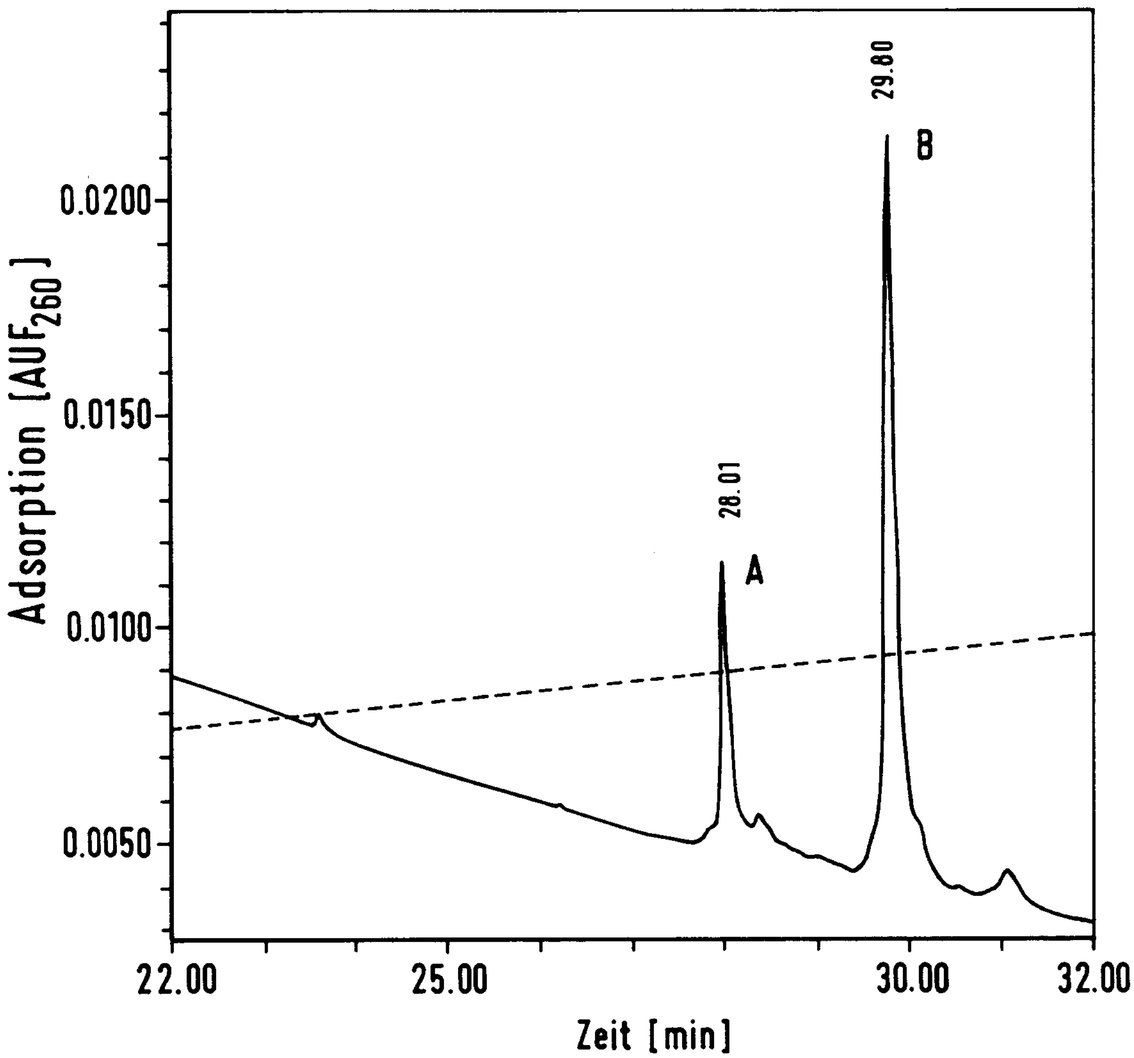
0.0200
0.0150
0.0100
0.0050
Adsorption [AUF260]
28.01
A
29.80
B
22.00
25.00
30.00
32.00
Zeit [min]

Fig. 4a:

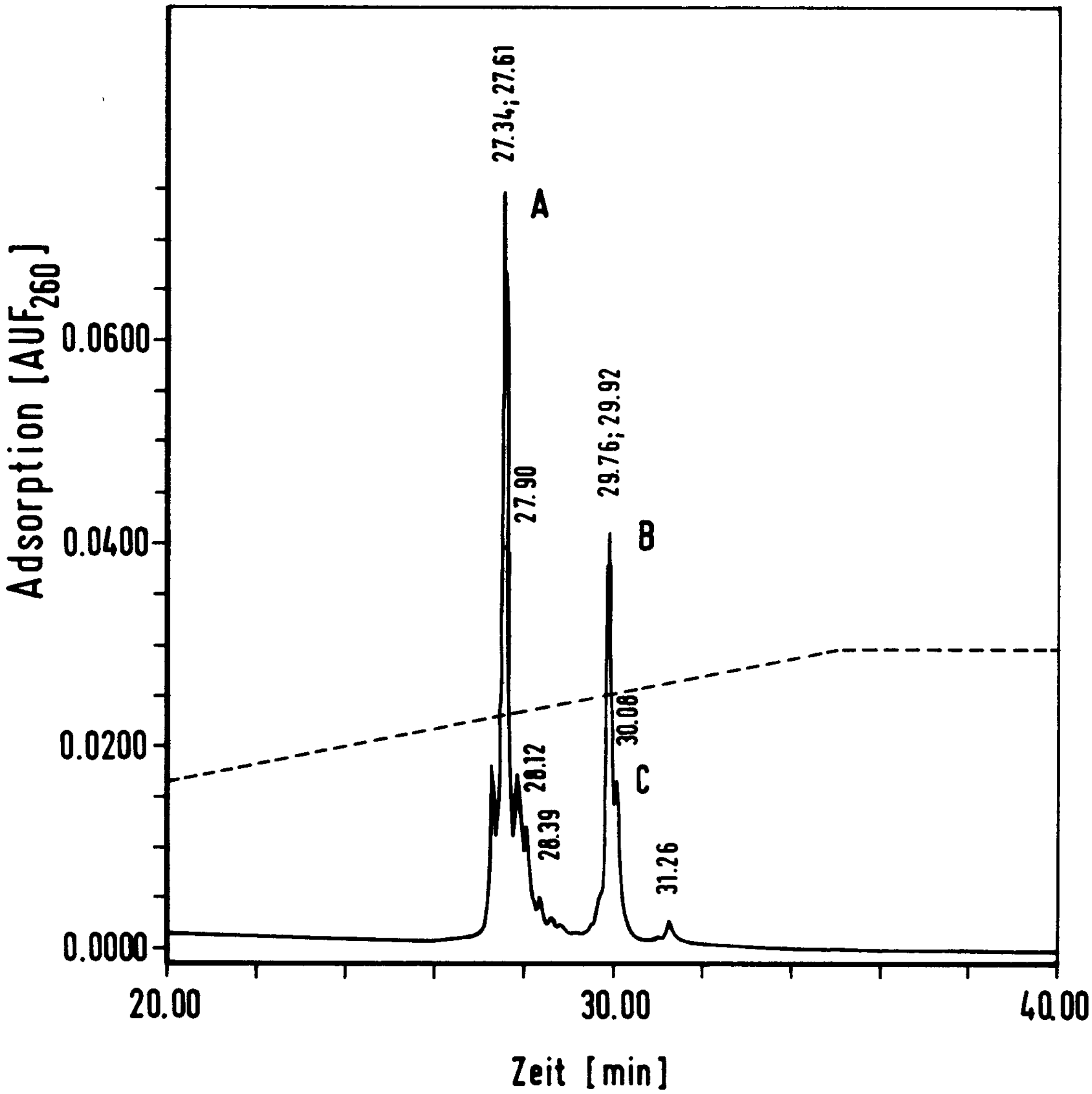
27.34; 27.61
A
27.90
28.12
28.39
29.76; 29.92
B
30.06
C
31.26
Adsorption [AUF260]
0.0600
0.0400
0.0200
0.0000
20.00
30.00
40.00
Zeit [min]

Fig. 4b:

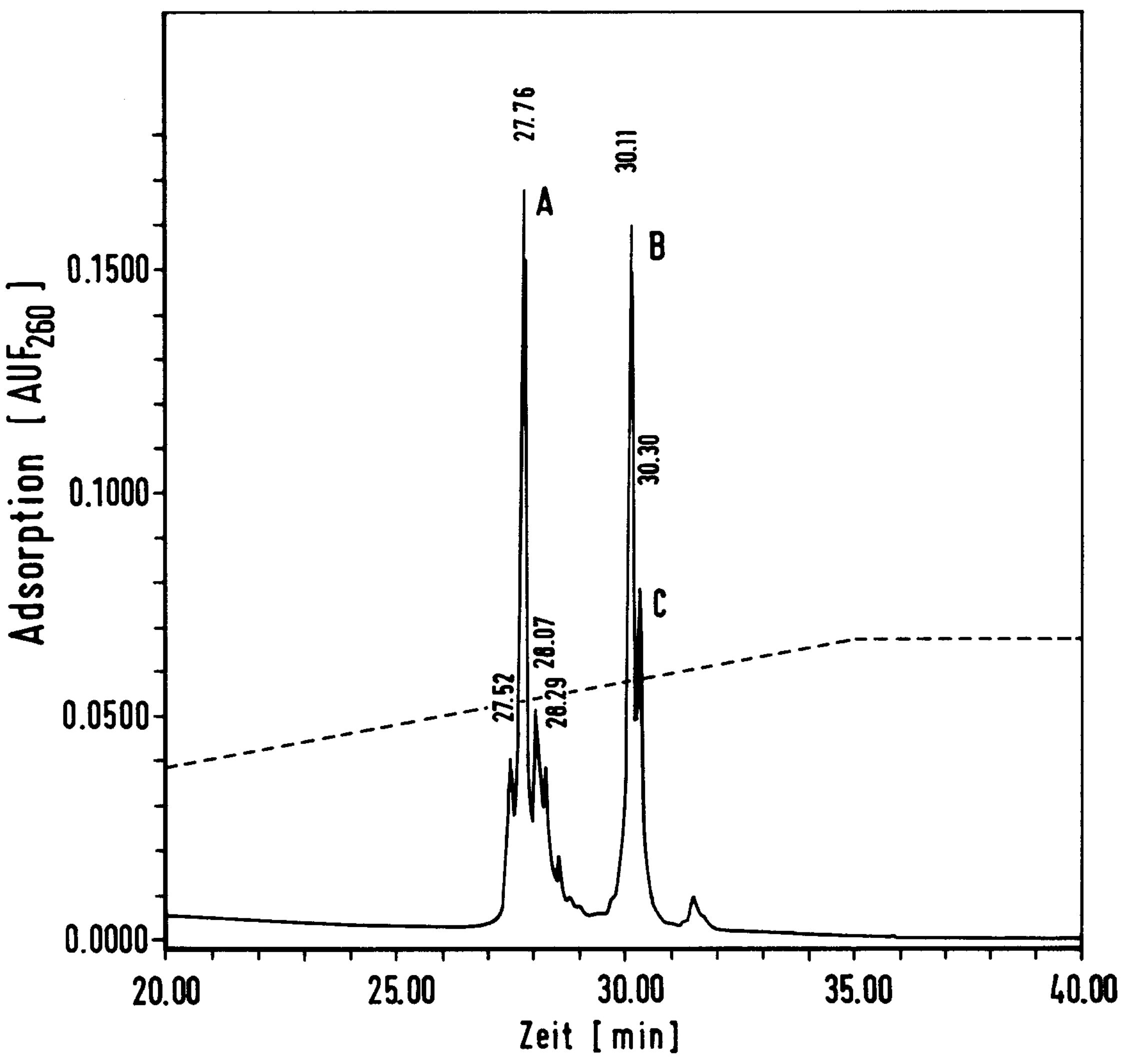
27.76
30.11
A
B
30.30
C
27.52
28.07
28.29
0.1500
0.1000
0.0500
0.0000
Adsorption [ $AUF_{260}$ ]
20.00
25.00
30.00
35.00
40.00
Zeit [min]

Fig. 4c:

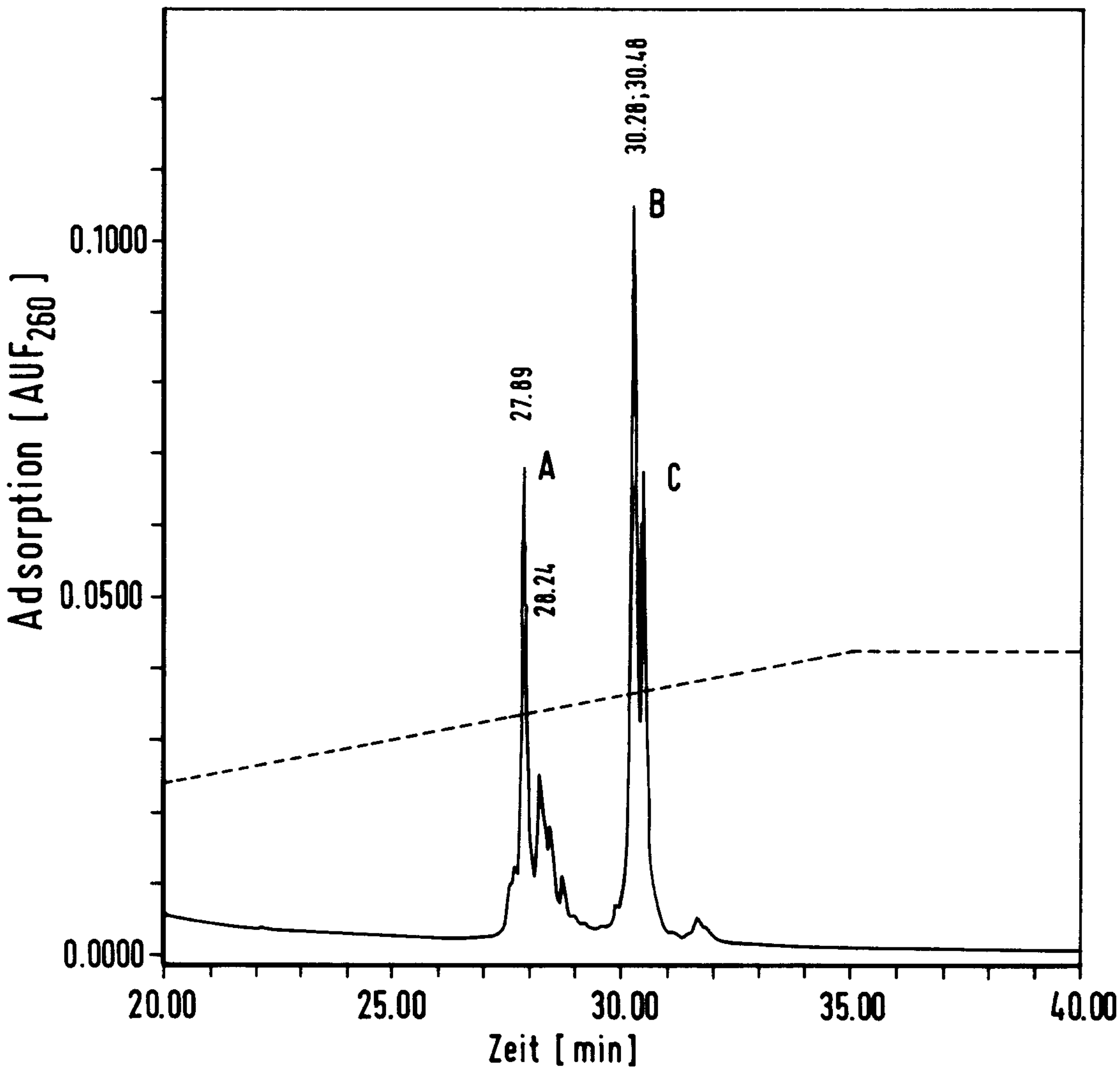
30.28;30.48
B
27.89
A
C
28.24
0.1000
0.0500
0.0000
Adsorption [AUF260]
20.00
25.00
30.00
35.00
40.00
Zeit [min]

Fig. 5a:

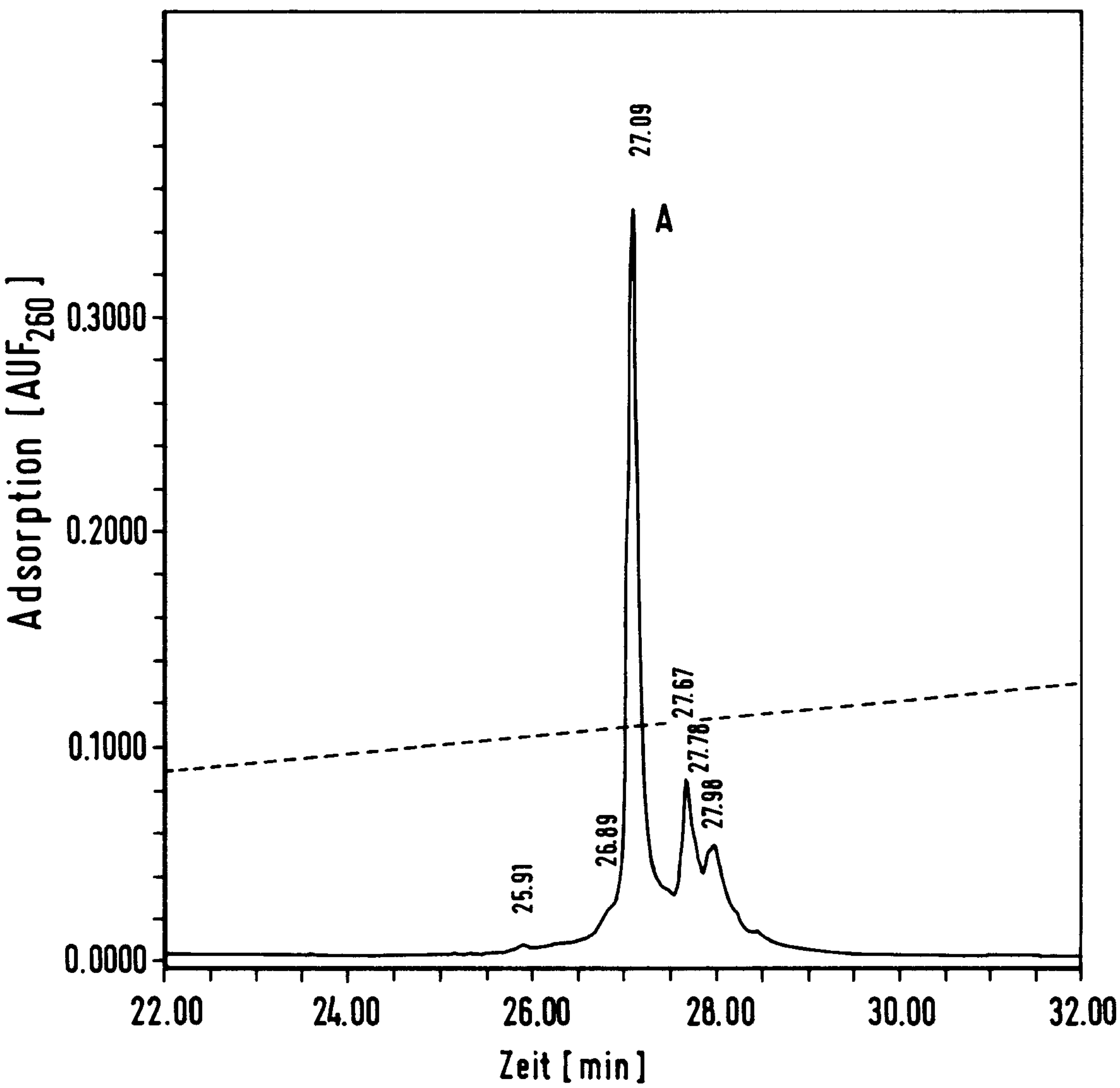
27.09
A
27.67
27.78
27.98
26.89
25.91
Adsorption [AUF260]
0.3000
0.2000
0.1000
0.0000
22.00
24.00
26.00
28.00
30.00
32.00
Zeit [min]

Fig. 5b:

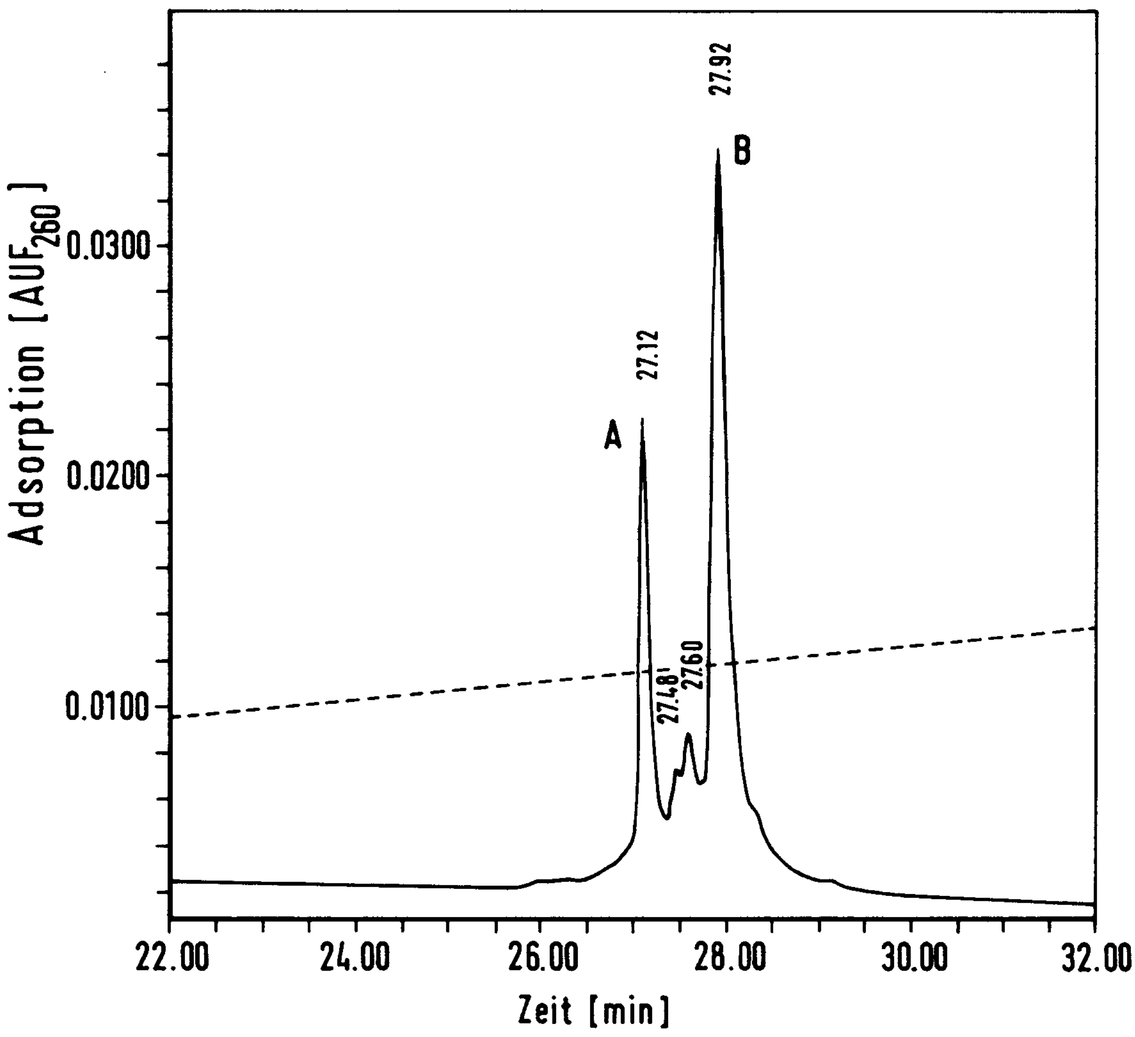
Adsorption [AUF260]
0.0300
0.0200
0.0100
27.92
B
27.12
A
27.48
27.60
22.00
24.00
26.00
28.00
30.00
32.00
Zeit [min]

Europäisches Patentamt

# EUROPÄISCHER RECHERCHENBERICHT

Nummer der Anmeldung
EP 93 11 9783

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | WO-A-89 02933 (ML TECHNOLOGY VENTURES)<br>* Seite 1, Zeile 1 - Zeile 9 *<br>* Seite 16, Zeile 16 - Seite 18, Zeile 33 * | 1,2,4,6, 8,10-14 | C07H21/00<br>C07D219/16<br>C07D405/14<br>C07D473/22<br>G01N33/533<br>C12Q1/68 |
| D,X | WO-A-89 02896 (ML TECHNOLOGY VENTURES)<br>* das ganze Dokument * | 1,2,4,6, 8,10-14 | |
| X | WO-A-90 03383 (THE UNITED STATES OF AMERICA)<br>* Zusammenfassung * | 1 | |
| X | EP-A-0 312 248 (ML TECHNOLOGY VENTURES)<br>* das ganze Dokument * | 1,2,4,6, 8,10-14 | |
| D,X | EP-A-0 212 951 (INTEGRATED GENETICS INC.)<br>* das ganze Dokument * | 1,2,4,6, 8,10-14 | RECHERCHIERTE SACHGEBIETE (Int.Cl.5) |
| D,X | EP-A-0 407 816 (ABBOTT LABORATORIES)<br>* das ganze Dokument * | 1,3,4,8, 10-14 | C07H<br>C07D<br>G01N<br>C12Q |
| D,X | EP-A-0 310 312 (ML TECHNOLOGY VENTURES L.P.)<br>* Seite 9; Beispiel 5 * | 1,2,4,6, 10-14 | |
| P,X | EP-A-0 552 766 (HOECHST AKTIENGESELLSCHAFT)<br>* das ganze Dokument * | 1,10-14 | |
| P,X | EP-A-0 552 767 (HOECHST AKTIENGESELLSCHAFT)<br>* das ganze Dokument * | 1,10-14 | |

Der vorliegende Recherchenbericht wurde für alle Patentansprüche erstellt

| Recherchenort | Abschlußdatum der Recherche | Prüfer |
|---|---|---|
| DEN HAAG | 23. März 1994 | Scott, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)